(19) Europäisches Patentamt
European Patent Office
Office européen des brevets

(11) Publication number: **0 051 990**
**B1**

(12) # EUROPEAN PATENT SPECIFICATION

(45) Date of publication of patent specification: **02.01.86**

(21) Application number: **81305272.7**

(22) Date of filing: **06.11.81**

(51) Int. Cl.[4]: **C 07 D 335/04,**
C 07 D 337/06,
C 07 D 311/00,
C 07 D 221/22,
C 07 D 313/06,
C 07 D 313/20,
C 07 D 335/10,
C 07 D 495/18,
C 07 D 495/08, A 01 N 43/18,
A 01 N 43/32

(54) **Biologically active heterobicyclic hydroximidates and thiolhydroximidates and carbamate ester derivatives thereof.**

(30) Priority: **10.11.80 US 205436**

(43) Date of publication of application:
**19.05.82 Bulletin 82/20**

(45) Publication of the grant of the patent:
**02.01.86 Bulletin 86/01**

(84) Designated Contracting States:
**AT BE CH DE FR GB IT LI LU NL SE**

(58) References cited:
**BE-A- 766 109**
**US-A-3 231 599**
**US-A-3 317 562**
**US-A-3 576 834**

**Pesticide Biochemistry and Physiology, vol. 6 (1976), page 571 ff**

**The file contains technical information submitted after the application was filed and not included in this specification**

(73) Proprietor: **SDS BIOTECH CORPORATION**
**P.O. Box 348 7528 Auburn Road**
**Painesville Ohio 44077 (US)**

(72) Inventor: **Magee, Thomas Alexander**
**7301 Case Avenue**
**Mentor Ohio 44060 (US)**
Inventor: **Battershell, Robert Dean**
**82 Bellaire Drive**
**Painesville Ohio 44077 (US)**
Inventor: **Limpel, Lawrence Eugene**
**431D Clearview Drive**
**Euclid Ohio 44123 (US)**
Inventor: **Ho, Andrew Wai-Wah**
**1911 Corte Cruz**
**Pinole California 94564 (US)**
Inventor: **Friedman, Arthur Jay**
**26218 N. Woodland Road**
**Beachwood Ohio 44122 (US)**
Inventor: **Corkins, Harry Glenn**
**4 Rockleigh Drive**
**Ewing New Jersey 08628 (US)**
Inventor: **Brand, William Wayne**
**6223 Indian Point Road**
**Painesville Ohio 44077 (US)**
Inventor: **Buchman, Russell**
**178 Willobend Drive**
**Madison Ohio 44057 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: **Storace, Louis**
**Apt. 1081 5782 Andrews Road**
**Mentor-on-the-Lake Ohio 44060 (US)**
Inventor: **Osgood, Edmond Randolph**
**7656 Mary Lane**
**Mentor Ohio 44060 (US)**

(74) Representative: **Oliver, Roy Edward et al**
**POLLAK MERCER & TENCH High Holborn House**
**52-54 High Holborn**
**London WC1V 6RY (GB)**

**Description**

BACKGROUND OF THE INVENTION

The present invention relates generally to biologically active hydroximidates, thiolhydroximidates, amidoximes and, more particularly, to novel pesticidally active carbamate and sulfenylated carbamate derivatives thereof.

Carbamate derivatives of hydroximidates and thiolhydroximidates have heretofore been previously described. For example, U.S. Patent No. 3,576,834 discloses acyclic compounds of the structure

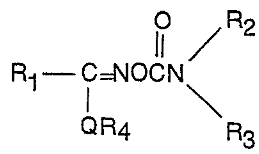

wherein Q is sulfur or oxygen and the insecticidal and acaricidal activity thereof.

Similarly, carbamates of monocyclic thiolhydroximidates of the structure

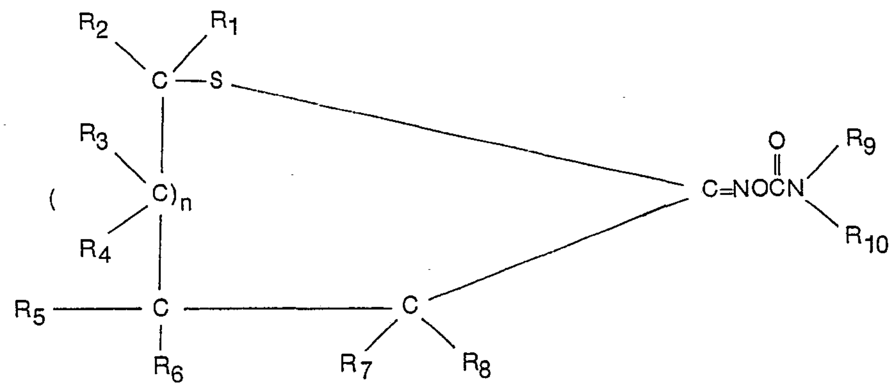

are disclosed in U.S. Patent No. 3,317,562 as insecticides.

Carbamate derivatives of bicyclic oximes have been previously reported. U.S. Patent No. 3,231,599 discloses compounds of the structure

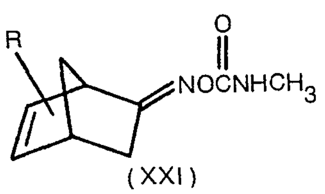

(XXI)

and

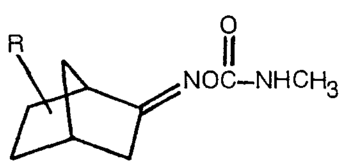

(XXII)

as insecticidally active compounds.

Further, Belgian Patent No. 766109 reports quinuclidine oxime carbamates of the structure

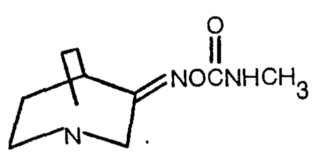

(XXIII)

also with reported insecticidal activity.

U.S. Patent Nos. 3,317,562 and 3,574,233 further disclose thiabicycloalkanes and -alkenes of the formulas

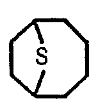

and

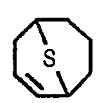

The patentees indicate that compounds of the above formulas possess insecticidal, fungicidal, nematocidal, antibacterial and herbicidal activity. It will be appreciated that the foregoing thiabicyclo-alkanes and alkenes are not carbamates.

U.S. Patent No. 4,219,658 discloses lactones and thiolactones of the formulas

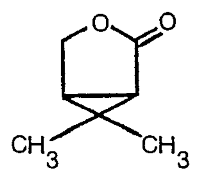

and

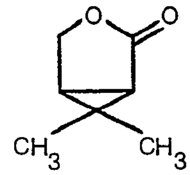

as intermediates in the preparation of hydrocarbylthiomethyl-2,2-dimethylcyclopropane carboxylic acids useful as pesticides.

It has now been surprisingly found, in accordance with the present invention, that substituted heterobicyclic compounds of the formula (I)

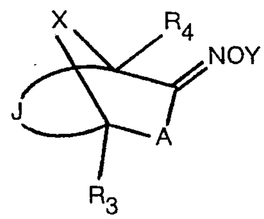

(I)

as defined hereinafter possess a broad range of useful biological properties, as well as a high degree of activity as arthropodicides, i.e., insecticides, acaricides, aphicides, as well as nematocides.

As detailed hereinbelow, the compounds of the above formula (I) are chemically and biologically distinguishable from the acyclic and monocyclic hydroximidates and thiolhydroximidates, bicyclic oximes and thiabicyclo-alkanes and -alkenes described above in reference to heretofore suggested compounds by reason of the mode of action, type of action and level of action demonstrated for the compounds of the present invention, as well as the fact that the compounds of the present invention are not obtainable by previously suggested synthesis methods employed to prepare the prior art compounds which appear to be structurally similar.

Certain of the compounds of the present invention are derived from Diels-Alder adducts of thiocarbonyl compounds with cyclic dienes. The ability of carbon-sulfur double bonds to serve as dienophiles in the Diels-Alder reaction was first reported in 1965 by Middleton [*J. Org. Chem. 30,* 1390 (1965)] who described the reactions of perfluorinated thioketones, thiophosgene and thiocarbonyl fluoride with several dienes, including cyclopentadiene, e.g.,

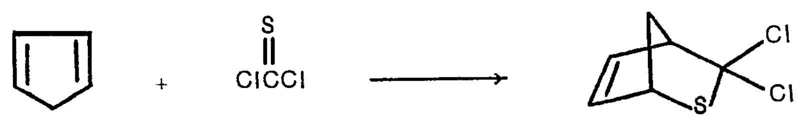

Since that time, many other examples of such reactions have been reported, including in addition to the above dienophiles such thiocarbonyl compounds as $NCC(=S)SCH_2$ [*Can J. Chem. 49,* 3755 (1971)], $NCC(=S)NR_1R_2$ [*Tetrahedron, Letters,* 2139 (1977)] $RSO_2C(=SR'$ [*Tetrahydron 30,* 2735 (1974)], and

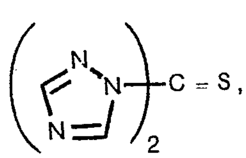

,

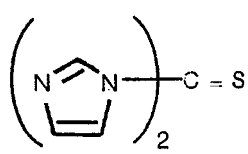

[ *J. Org. Chem. 45,* 3713 (1980) ]

A limited number of reports have appeared on further reactions of the initial Diels-Alder adducts. Johnson et al. [*J. Org. Chem. 34,* 860 (1969)] describe oxygenation of the sulfur, followed by epoxidation of the double bond as depicted below:

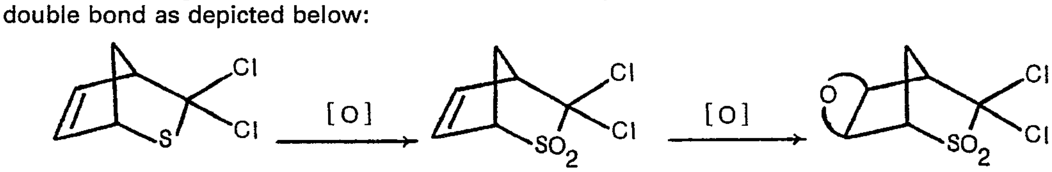


The authors indicate that one chlorine can be reduced from the dichloro sulfone by treatment with divalent chromium:

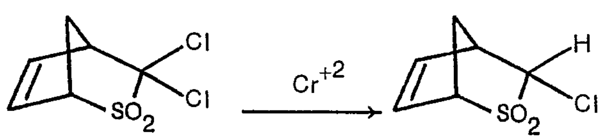

Reduction with lithium aluminium hydride yields the bicyclic thioalkene:

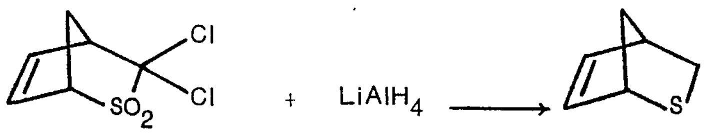


In 1973, Reich et al. [*J. Org. Chem. 38,* 2637 (1973)] reported the reaction of thiophosgene with cyclohexadienes, to yield 3,3-dichloro-2-thiabicyclo[2.2.2]oct-5-enes, as well as the hydrolysis of these Diels-Alder adducts to the corresponding thiolactones.

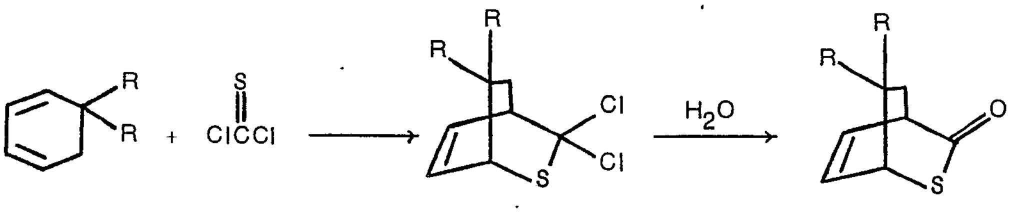


(X)

Benassi et al. [*Synthesis 735 (1974)*] describe a similar hydrolysis in the [2.2.1] system during reduction using an aqueous workup procedure:

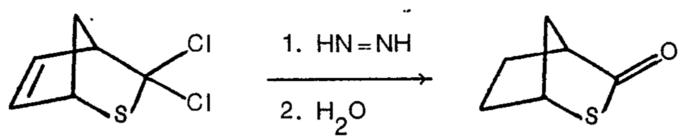


Raasch [*J. Org. Chem. 40,* 161 (1975)] reports on the addition of halogen or sulfenyl halides to the carbon-carbon double bond of the 2-thiabicyclo[2.2.1]hept-5-enes with rearrangement to yield the 6,7-disubstituted thiabicyclo[2,2,1] heptanes, e.g.,

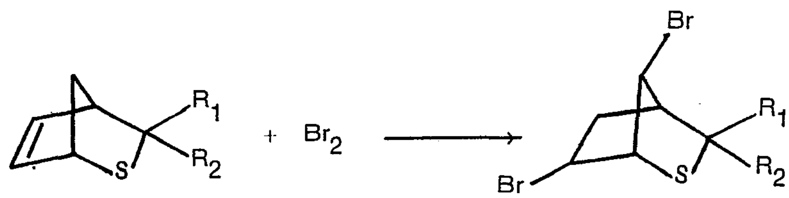


In 1976, Allgeier et al. [*Tetrahedron Letters,* 215 (1976)] also described the reaction of thiophosgene with anthracene to give the Diels-Alder adduct which is subsequently hydrolyzed to the thiolactone. Hong [*Dissertation Abstracts International 40,* 5672-B (1980)] states that solvolysis with methanol of the Diels-Alder adduct from thiophosgene and cyclopentadiene results in ring opening to yield (XIV) in almost quantitative yield.

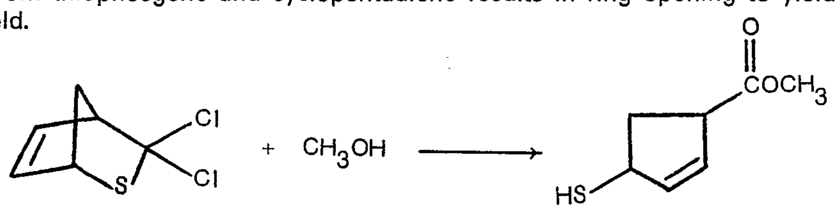


(XIV)

For purposes of indexing, *Chemical Abstracts* classifies compoun ds of type X above as 2-thiabicyclo[2.2.2]oct-5-en-3-ones. In referring to these compounds, it is important to distinguish nomenclature used for indexing convenience from nomenclature signifying chemical type. Thus, *Chemical Abstracts* refers to both compounds XV and XVI (shown below) as thiabicycloalkanones. Chemically, however, compound XV is a thiolactone, i.e., a cyclic thiolester; whereas compound XVI [*J. Org. Chem. 43,* 4013 (1978)] is a true ketone and specifically a β-thioketone.

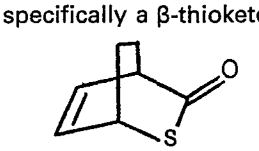
(XV)

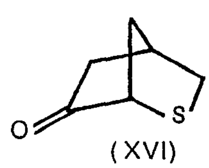
(XVI)

Esters and ketones are distinct chemical classes with many distinctly different properties. One of these distinctions is their reactivity with hydroxylamine. Ketones are known to react readily with hydroxylamine to form oximes:

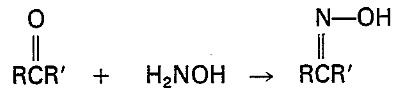

Esters or thiolesters (and their cyclic counterparts, lactones and thiolactones) do not react with hydroxylamine to yield the analogous hydroximidates or thiolhydroximidates.

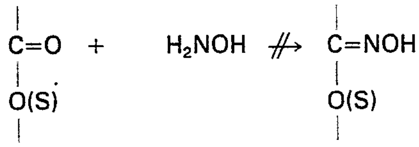

Instead they react with cleavage of the carbon-oxygen or carbon-sulfur single bond to yield as displacement products, the hydroxamic acid or, in the case of the lactones and thiolactones, a lactam (*Advanced Organic Chemistry,* Second Edition, Jerry March, McGraw-Hill Book Company, New York, 1977, p. 386, 388).

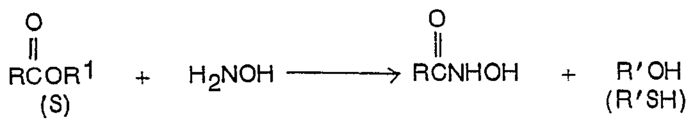

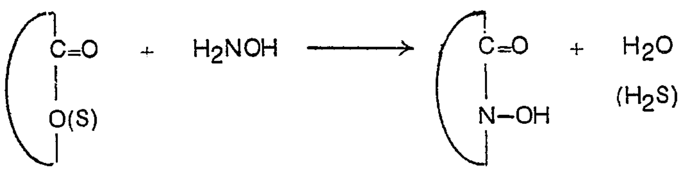

An example of the failure of thiolactones to react with hydroxylamine to yield thiolhydroximidates is reported by Bruice and Fedor [*J. Am. Chem. Soc. 86,* 4886 (1964)]. The authors confirm that thiolactones, e.g., thiolbutyrolactone, undergo hydroxylaminolysis in a manner analogous to their thiolester acyclic counterparts.

The hydroximidates and thiolhydroximidates, i.e.,

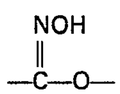 and 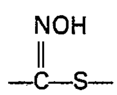

have been reported in the literature. At least four methods of preparation have been described. U.S. Patent No. 3,576,834 (and the references therein) describes two of these: (1) the reaction of an iminoether hydrochloride with hydroxylamine and (2) chlorination of an aldoximine to form a hydroxamoyl chloride followed by reaction of the latter with a salt of a mercaptan:

(1) 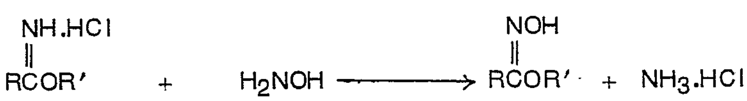

(2) 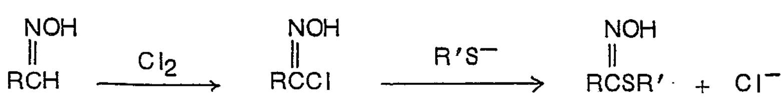


U.S. Patent No. 3,787,470 reports the formation of thiolhydroxamate esters from nitroalkanes and alkyl mercaptans:

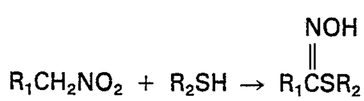

Faust et al. [*Journal fuer Praktische Chemie,* Leipzig, *311*(1), 61, (1969)] describe the conversion of a thiopyranthione to a cyclic thiolhydroximidate.

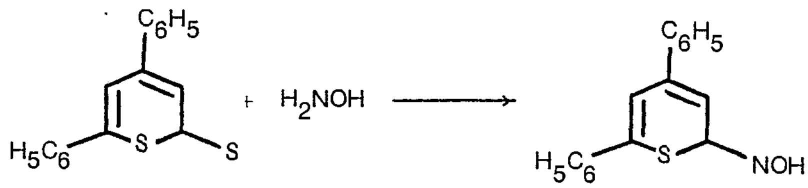


It will be seen from the foregoing that applicants have discovered that the Diels-Alder adducts of certain thiocarbonyl compounds with cyclic dienes are converted easily and in high yield to bicyclic thiolhydroximidates which not only possess significant biological activity but which are valuable as intermediates in the synthesis of carbamate and sulfenylated carbamate final products. Moreover, the outstanding arthropodicidal and nematocidal activity exhibited by these compounds is entirely unexpected in light of the prior art set forth hereinabove which relates to the ease of hydrolysis of the thiophosgene adducts, along with the reported inability of the thiolactones thus formed to react with hydroxylamine to yield compounds of the subject invention.

In addition to the above observations relative to the absence of prior art synthesis methods which would allow one to predictably obtain the compounds of the present invention by analogy, it is likewise important to note the material differences in biological activity observed with the compounds of the present invention compared to that of the seemingly related prior art compounds described hereinabove. In making such comparisons, it is again important to distinguish between nomenclature used for indexing convenience and nomenclature signifying actual chemical type. For example, for indexing purposes, *Chemical Abstracts* would describe the compounds of the present invention, as well as those of formulas (XXI)—(XXIII) as carbamates of bicycloalkanone oximes. However, in reality, the compounds of formulas (XXI)—(XXIII) are derivatives of true oximes; whereas, the compounds of the present invention are derivatives of hydroximidates or thiolhydroxyimidates. The significance of the foregoing distinction is readily apparent when one considers the work of Huhtanen and Dorough [*Pesticide Biochemistry and Physiology 6,* 571 (1976)] who have shown that the chemical difference between the ketoxime derivative XXIV and the thiolhydroximidate derivate XXV

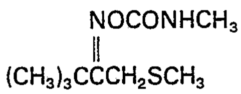
(XXIV)

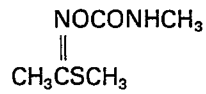
(XXV)

results in surprisingly marked differences in their biological activity. Although both (XXIV) and (XXV) are insecticides, their spectrum of activity, persistence, use pattern and method of application are totally different. It is evident that these documented differences result from the difference in chemical structure between a true ketoxime and a thiolhydroxyimidate. It will also be appreciated that a similar distinction between the compounds of the present invention and compounds of formulas (XXI)—(XXIII) prevails.

In accordance with a first aspect thereof and by way of enlargement of what is stated previously, the present invention provides substituted heterobicyclic compounds of the formula

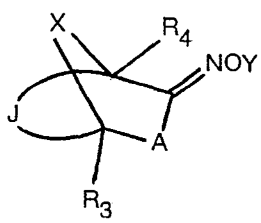
(I)

characterized in that:

A represents S, O, $S(O)_m$, where m is 1 or 2, or $NR_5$, where $R_5$ is hydrogen, alkyl, aryl or cyano;

J represents the group

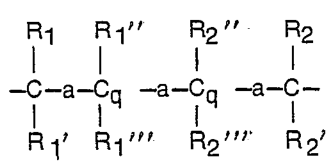

where q, independently, is 0 or 1, a, independently, is a single or double bond and $R_1$, $R_1'$, $R_1''$, $R_1'''$, $R_2$, $R_2'$, $R_2''$ and $R_2'''$ are as defined below;

X represents O, S, $NR_6$,

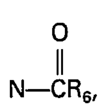

where $R_6$ is hydrogen or alkyl, or a bridge member selected from

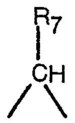 , 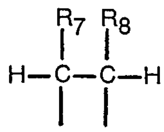 , 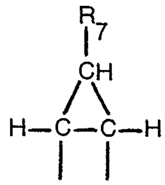 , 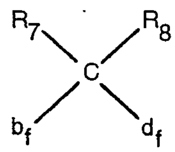 and 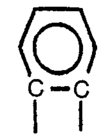 ,

where $R_7$ and $R_8$ independently represent hydrogen, halogen, cyano, alkyl, alkoxy, alkoxycarbonyl or alkylthio, and b and d independently represent carbon or oxygen and f is 0 or 1;

$R_1$—$R_1'''$, inclusive, $R_2$—$R_2'''$, inclusive, $R_3$ and $R_4$ independently represent hydrogen, halogen, hydroxy, cyano, alkyl, alkoxy, haloalkyl, alkylcarboxy, arylcarboxy, alkylaminocarboxy, carbamoyl, alkylcarbamoyl, dialkylcarbamoyl, alkylthio, alkylsulphinyl, alkylsulphonyl, alkylamino, dialkylamino, alkoxycarbonyl, trifluoromethyl, pyrrolidyl, phenyl, nitro, thiocyano, thiocarbamyl, alkylthiocarbamyl, dialkylthiocarbamyl, arylthiocarbamyl or the group

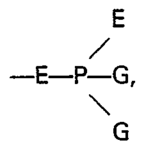

where E is O or S and G represents alkyl, alkoxy, alkylthio, amino, alkylamino or dialkylamino; or, when a is a single bond and at least two of $R_1'$, $R_1'''$, $R_2'$ or $R_2'''$ are hydrogen on adjacent C atoms, $R_1$, $R_1''$, $R_2$ or $R_2''$ on the same adjacent C atoms together represent

—O—, 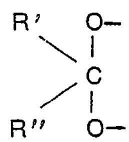 or 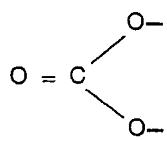 ,

where R′ and R″ represent hydrogen or alkyl; or any of $R_1$ and $R_1'$, $R_1''$ and $R_1'''$, $R_2''$ and $R_2'''$ or $R_2$ and $R_2'$ represent =O; or when q, independently, is 0 or 1 and a, independently, is a double bond, $R_1'$, $R_1'''$, $R_2'''$ or $R_2'$ are absent; and

Y represents hydrogen or

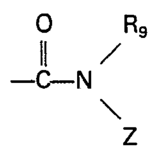

where $R_9$ represents hydrogen, alkyl, hydroxyalkyl, alkenyl, alkynyl, aralkyl, alkoxyalkyl or polyoxyalkylene; and Z represents

a) 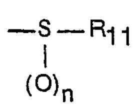 ,

where n is 0, 1 or 2 and $R_{11}$ is pyridyl, pyrimidyl, phenyl or phenyl substituted with at least one member selected from hydroxy, alkyl, alkoxy, halogen, nitro, trifluoromethyl and cyano;

b) 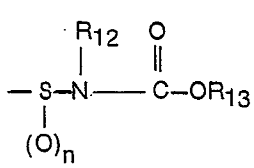 ,

where n is 0, 1 or 2, and $R_{12}$ is alkyl, alkoxyalkyl or

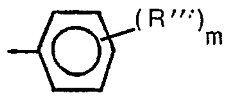,

where m is 0, 1, 2 or 3 and R''' is hydrogen, halogen, cyano, nitro, alkyl, alkoxy, alkylthio, alkylsulphonyl or phenyloxy, and $R_{13}$ is alkyl, alkoxyalkyl, naphthyl, alkylthioalkyl,

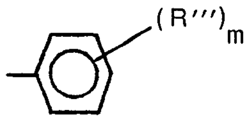 , 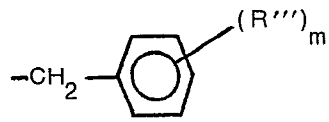 ,

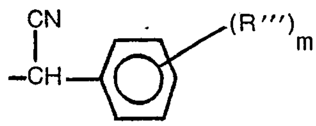 or 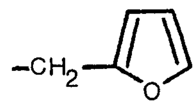

where $(R''')_m$ has the meaning defined above,

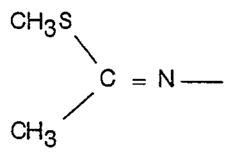 , 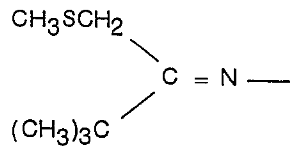

or Q, where Q—OY represents formula (I) as defined above;

c) 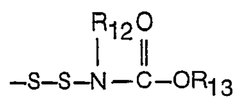,

where $R_{12}$ and $R_{13}$ have the meanings defined above;

d) 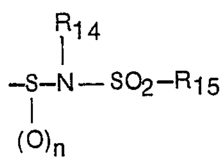 ,

where n is 0, 1 or 2, $R_{14}$ is phenyl, alkyl, alkoxyalkyl, alkoxycarbonylalkyl, alkylthioalkyl or carboxyalkyl and $R_{15}$ is alkyl,

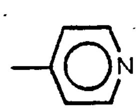 , 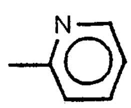 , 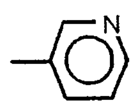 or 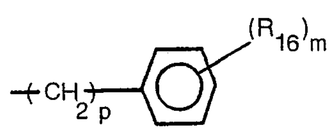

where m is 0—5, p is 0—5, and $R_{16}$ is halogen, alkyl, trifluoromethyl, nitro or alkoxy;

e) $-S-NR_{17}R_{18}$,

where $R_{17}$ and $R_{18}$ are alkyl or aryl or together with the nitrogen atom represent

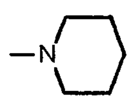 , 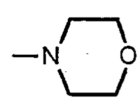 , 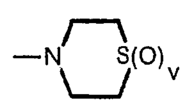 ,

where v is 0, 1 or 2, or

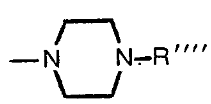 ,

where R'''' is alkyl;

f) 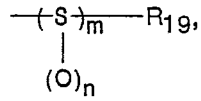

where n is 0, 1 or 2, m is 1 or 2 and $R_{19}$ is alkyl, cycloalkyl, haloalkyl, cyanoalkyl, alkoxycarbonyl, (alkylthio)carbonyl, alkoxy(thiocarbonyl), alkylthio(thiocarbonyl), aryl or substituted aryl with at least one substituent selected from halogen, cyano, nitro, alkyl, alkoxy, alkylthio, alkylsulphonyl and phenyloxy, with the proviso that when $R_{19}$ is aryl or substituted aryl, m is 2;

g) 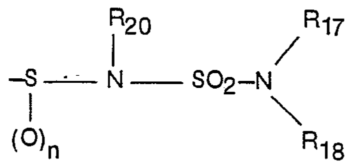

where n is 0, 1 or 2, $R_{20}$ is alkyl and $R_{17}$ and $R_{18}$ have the meanings defined above;

h) 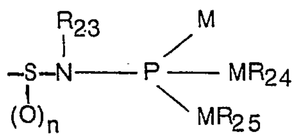 ,

where n is 0, 1 or 2, each M, independently, is S or O and $R_{23}$, $R_{24}$ and $R_{25}$ independently represent alkyl or $R_{24}$ and $R_{25}$ together represent

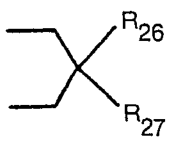 or 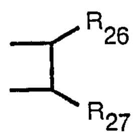 ,

where $R_{26}$ and $R_{27}$ independently represent hydrogen or alkyl;

i) 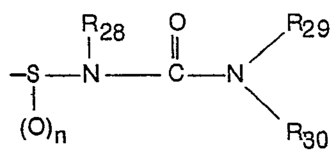 ,

where n is 0, 1 or 2, $R_{28}$ is alkyl or aryl and $R_{29}$ and $R_{30}$ independently represent hydrogen, alkyl, aryl or alkoxy;

j) 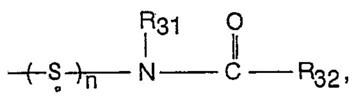

where n is 1 or 2, $R_{31}$ is alkyl and $R_{32}$ is fluoro, alkyl, aryl or aralkyl;

k) 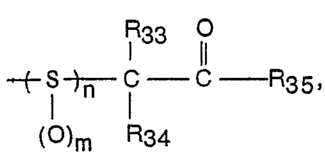

where m is 0, 1 or 2, n is 1 or 2 and $R_{33}$, $R_{34}$ and $R_{35}$ independently represent hydrogen, alkyl or aryl; or

l) 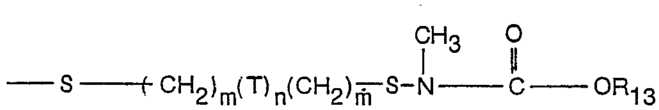 ,

where T is O, S or $-CH_2-$, m is 1 or 2, n is 0 or 1 and $R_{13}$ has the meaning defined above; where, in the meanings defined above, unless otherwise specified, "alkyl" and "alkoxy" signify groups which contain 1

to 22 carbon atoms and include all straight and branched structures, "alkenyl" and "alkynyl" signify groups which contain up to 22 carbon atoms and include all straight and branched structures, "cycloalkyl" signifies groups which contain 3 to 8 carbon atoms, "aryl" signifies groups which contain 6 to 12 carbon atoms and "aralkyl" signifies groups which contain an aryl group, as above defined, attached to an alkyl group, as above defined.

The present invention also provides the compound of general formula I in which J represents —CH=CH—, X represents —$CH_2CH_2$—, A represents S, $R_3$ represents H, $R_4$ represents CN and Y represents $C(=O)N(CH_3)SN(C_{12}H_{25}\text{-}n)SO_2NC_4H_8$.

According to a preferred embodiment of this aspect of the invention, a heterobicyclic compound is provided, of the formula

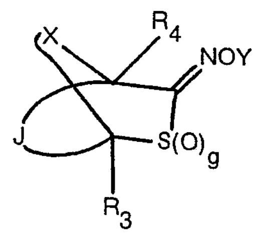

wherein

g is 0, 1 or 2, J, X and Y have the meanings defined above, and

$R_1$—$R_1'''$, inclusive, $R_2$—$R_2'''$, inclusive, $R_3$ and $R_4$ independently represent hydrogen, halogen, hydroxy, cyano, alkyl, alkoxy, haloalkyl, alkylcarboxy, arylcarboxy, alkylaminocarboxy, carbamoyl, alkylcarbamoyl, dialkylcarbamoyl, alkylthio, alkylsulphinyl, alkylsulphonyl, alkylamino, dialkylamino, alkoxycarbonyl, trifluoromethyl, pyrrolidyl, phenyl, nitro, thiocyano, thiocarbamyl, alkylthiocarbamyl, dialkylthiocarbamyl, arylthiocarbamyl or the group

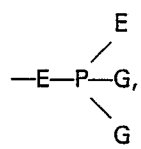

where E is O or S and G represents alkyl, alkoxy, alkylthio, amino, alkylamino or dialkylamino.

Within the general context of the valuable pesticidal activity of the compounds of the invention, the invention also includes an arthropodicidal composition comprising a carrier and a heterobicyclic compound according to formula (I) as defined above. Another embodiment of the invention consists in a nematocidal composition comprising a carrier and a heterobicyclic compound according to the formula defined in the preceding paragraph, in which g is 0.

A further aspect of the invention consists in a method of combating arthropods or nematodes, which comprises applying to the arthropods or nematodes or a habitat thereof an effective amount of at least one compound according to the last-mentioned embodiment or at least one composition containing a carrier and the said compound.

According to another aspect of this invention, a process is provided for the preparation of a compound of the formula:

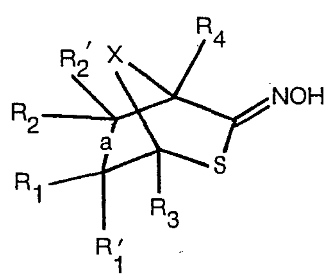

wherein

a is a single or double bond;

X represents O, S, $NR_6$,

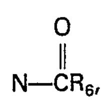

where $R_6$ is alkyl, or a bridge member selected from

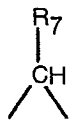, 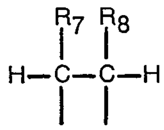, 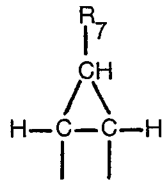, 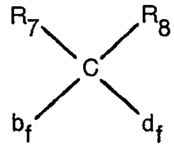 and 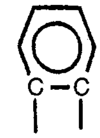,

where $R_7$ and $R_8$ independently represent hydrogen, halogen, cyano, alkyl, alkoxy, alkoxycarbonyl or alkylthio and b and d independently represent carbon or oxygen and f is 0 or 1;

$R_1'$—$R_4$, inclusive, independently represent hydrogen, halogen, hydroxy, cyano, alkyl, alkoxy, haloalkyl, alkylcarboxy, arylcarboxy, alkylaminocarboxy, carbamoyl, alkylcarbamoyl, dialkylcarbamoyl, alkylthio, alkylsulphinyl, alkylsulphonyl, alkylamino, dialkylamino, alkoxycarbonyl, trifluoromethyl, pyrrolidyl, phenyl, nitro, thiocyano, thiocarbamyl, alkylthiocarbamyl, dialkylthiocarbamyl, arylthiocarbamyl or the group

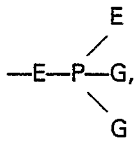

where E is O or S and G represents alkyl, alkoxy, alkylthio, amino, alkylamino or dialkylamino; or, when a is a single bond and $R_1'$ and $R_2'$ are hydrogen, $R_1$ and $R_2$ together represent

—O—, 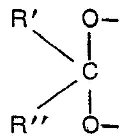 or 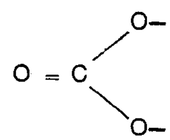,

where R′ and R″ represent hydrogen or alkyl; or

$R_1'$ and $R_1$ or $R_2$ and $R_2'$ represent =O; or,

when a is a double bond, $R_1'$ and $R_2'$ are absent;

wherein a cyclic diene of the formula

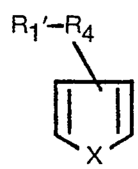,

wherein $R_1'$—$R_4$ and X have the meanings defined above herein, is reacted with a dienophile selected from

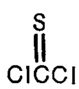, 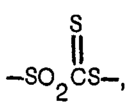, 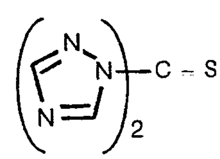 and 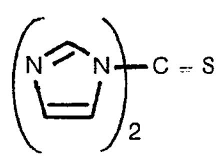

under cyclization conditions,

characterized in that the resulting adduct is reacted with hydroxylamine to form a thiolhydroximidate.

As indicated above, the expressions "alkyl" and "alkoxy" used throughout the present specification and claims, unless otherwise defined, denote straight and branched carbon-carbon linkages containing from 1 to 22 carbon atoms, e.g., methyl, ethyl, n-propyl, isopropyl, n-butyl, isobutyl, sec-butyl, tert-butyl, hexyl, heptyl, octyl, decyl or dodecyl. The expressions "alkenyl" and "alkynyl", unless otherwise defined, denote the respective unsaturated radicals containing up to 22 carbon atoms and include all straight and branched structures. The expression "cycloalkyl" denotes cyclic alkanes containing from 3 to 8 carbon atoms, whereas "aryl" denotes unsaturated ring systems containing from 6 to 10 carbon atoms. The term "halogen" includes chlorine, fluorine and bromine. The expression "polyoxyalkylene", unless otherwise defined, includes alkylene moieties of 1—4 carbon atoms and wherein the poloyoxyalkylene moiety may contain up to 50 oxyalkylene repeating units.

It will be appreciated by those skilled in the art that the compounds of the present invention may be prepared in various salt forms and, as used herein, the expression "pesticidally acceptable salts" is

intended to include those salts capable of being formed with the compounds and substituted derivatives thereof in accordance with the invention, without materially altering the chemical structure or pesticidal activity of the parent compounds. For example, alkali metal salts of carboxylic acid derivatives of the invention may be obtained by reaction with suitable bases, e.g., sodium hydroxide or potassium hydroxide. Likewise, alkaline earth metal salts may be similarly obtained.

Similarly, quaternary salts of the amino derivatives of the invention may also be obtained, as well as acid addition salts, where necessary, for instance, to alter solubility properties.

It will be understood that in the structural formulae depicting the compounds of the present invention all unsubstituted bonds are satisfied by hydrogen. It will further be apparent to those skilled in the art that the compounds of the present invention may exist in two geometric forms, the E and Z isomers (i.e., syn- and anti-isomers) around the carbon-nitrogen double bond. Both isomeric forms and their mixtures are encompassed within the scope of the present invention.

The hydroximino derivatives of the present invention, i.e., compounds of formula (I) where Y = hydrogen, are prepared according to the following methods.

Hydroxyimidate (oxabicyclo) compounds of the invention can be obtained, for example, by reaction of chlorosulphonyl isocyanate with appropriately substituted cyclic dienes to form N-chlorosulphonyl-β-lactams which readily rearrange to the (N-chlorosulphonyl)imino-oxabicyclic intermediate (via cyclization through oxygen). See [*J. Org. Chem.* *41,* 3583 (1976)], and [*J. Chem. Soc.* Perkin I, 874 (1977)] and the references cited therein. The thus obtained iminooxabicyclic intermediate is then reacted with hydroxylamine to yield the desired hydroximidate. The foregoing general reaction scheme is set forth below with substituent representations corresponding to those in formula (I). It will be recognized that, while the following scheme illustrates the formation of a compound of the present invention having a [2.2.1] bicyclic ring system, the above generally described process is followed to obtain compounds of the present invention having, for example, a [3.2.1] or [4.2.1], bicyclic ring system. The specific bicyclic ring system obtained will, of course, be determined by the selection of the appropriate reactants (e.g., employing a cyclohexadiene reactant in place of the cyclopentadiene reactant below).

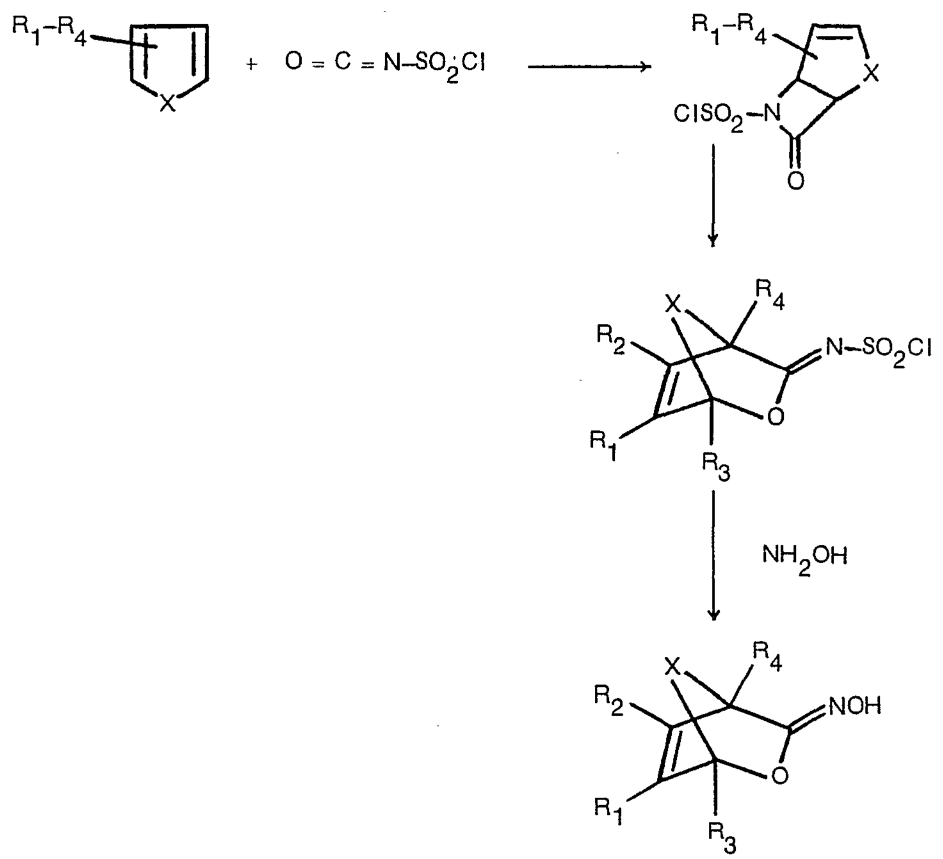


As alluded to previously, the bicyclic thiolhydroximidates of the present invention are obtained by a Diels-Alder reaction of certain thiocarbonyl compounds with appropriately substituted cyclic dienes under suitable cyclization conditions to obtain the initial adducts which are then reacted with hydroxylamine to afford the desired intermediates. The foregoing general reaction scheme is depicted below with substituent representations corresponding to those set forth previously in formula (I):

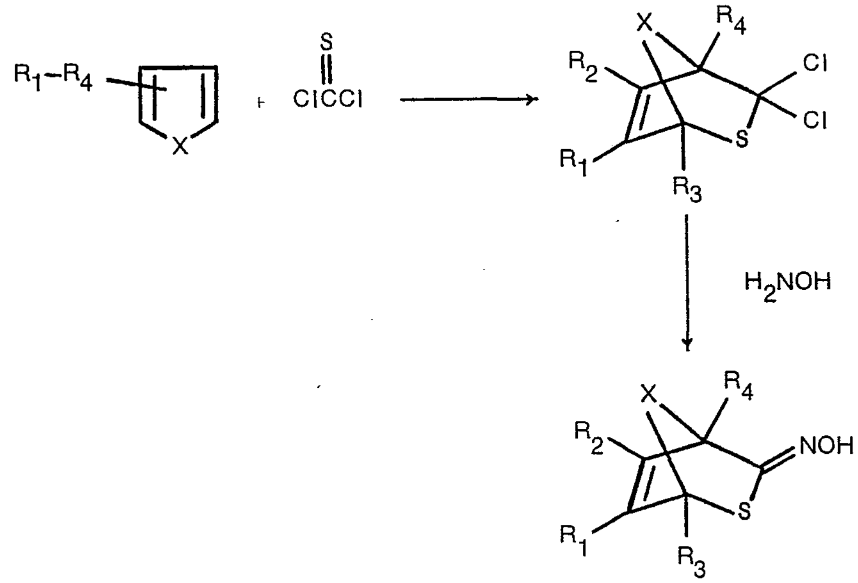


The bicyclic azahydroximidates of the present invention are prepared in a similar manner to the preparation of the oxa- and thiolhydroximidates previously described. More specifically, the appropriate 3-tosyl-2-azabicyclo intermediate [prepared by the procedure of *J. Organic Chemistry 39,* 564 (1974)] is treated with hydroxylamine to give the desired bicyclic azahydroximidate. Alternatively, conversion of the appropriate lactam to the corresponding thionolactam followed by treatment with hydroxylamine will also afford the desired azahydroximidate. The specific reactants, procedures and conditions are further illustrated in the examples.

The hydroximino intermediates can then be converted to the various carbamate derivatives embraced by formula (I) by any of several conventional methods. One preferred method involves the reaction of an isocyanate substituted with groups corresponding to $R_9$ or $R_{10}$ of formula (I) with the particular hydroximino intermediate. The hydroximino intermediate and isocyanate are reacted in an inert organic solvent at from about 0°C to about 150°C, preferably from 20°C to 80°C, and at a pressure from 1 to 10 atmospheres, usually 1 to 3 atmospheres. Reaction pressures will be determined by reaction temperature, concentration and vapour pressure of the isocyanate.

Any inert organic solvent used in the reaction should not contain hydroxy, amino or other groups which will react with the isocyanate function. Useful inert solvents include aliphatic and aromatic hydrocarbons, e.g. hexane, heptane, octane, benzene, xylene; ethers such as diethyl ether or ethylpropyl ether, esters such as ethyl acetate, ethyl propionate; ketones such as acetone, methyl ethyl ketone and various chlorinated hydrocarbons such as methylene chloride and perchloroethylene.

The reaction may be carried out in the presence of 0.1 to 1.0 percent by weight, based on the weight of reactants, of a tertiary amine catalyst such as triethylamine or N,N-dimethylaniline.

The molar ratio of isocyanate to hydroximino reactant can vary from 0.1:1 to 10:1. An equimolar amount or slight excess of isocyanate is preferred to ensure complete reaction. Reaction times may also vary from a few minutes to several days, the usual reaction time being from 1/2 to 6 hours.

Another method for preparing such carbamate derivatives involves reaction of the hydroximino compound with phosgene to obtain the chloroformate which is reacted with an amine. A solution of the hydroximino derivative is generally dissolved in an inert solvent such as diethyl ether which is added slowly to a solution of phosgene dissolved in inert solvent in the presence of an HCl acceptor such as a tertiary amine. Reaction temperatures vary from −30°C to 100°C, the usual temperatures being from 0°C to 50°C. The resulting reaction mixture, a solution of the chloroformate in an inert organic solvent, can be filtered to remove amine hydrochloride before reaction with an amine in step 2 of the reaction. The amine is added to the chloroformate solution in the presence of a suitable amine solvent such as water, at a temperature from −40°C to 80°C. A larger than molar excess of amine can be used so that the amine acts both as a reactant and as an HCl acceptor and complete conversion of chloroformate is obtained. Alternatively, a separate HCl acceptor, such as tertiary amine can be used.

It will also be appreciated by those skilled in the art that the carbamate derivatives of the present invention can be prepared by reaction of N-protected hydroxylamine with an appropriately substituted carbamylating agent, e.g.,

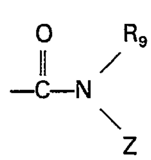

as defined before, deprotection of the resulting intermediate and reaction of the deprotected intermediate with, for example, the initial Diels-Alder adduct to obtain the products of formula (I).

The sulfenylated carbamates of the invention may be prepared from the carbamates and carbamate precursors obtained in accordance with the foregoing description by methods described, for example, in *Angewandte Chemie,* International Edition, *16,* 735 (1977) and the references referred to therein, *J. Org. Chem. 43,* 3953 (1978), *J. Agric. Food Chem. 26,* 550 (1978), U.S. Patent Nos. 4,201,733, 4,148,910, 4,138,423 and 4,108,991.

Essentially, two methods have previously been employed for the preparation of such derivatives. In the first of these, an N-alkyl carbamate is allowed to react with a sulfenyl halide to yield the sulfenylated carbamate in accordance with the following reaction scheme:

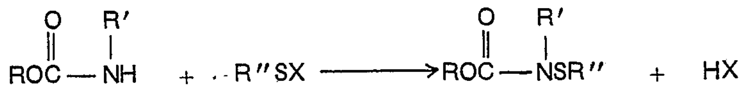

In the second method, the sulfenyl halide is allowed to react with an N-alkyl carbamyl halide to yield an intermediate N-alkyl-N-(substituted thio)-carbamyl halide which is then treated with the desired hydroxylic moiety to provide the carbamate.

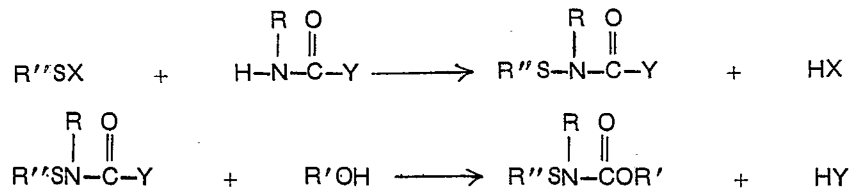

In each of the foregoing reactions, where HX or HY are generated, an acid acceptor is utilized to facilitate the reaction.

The foregoing sulfenylation reactions are normally conducted in an aprotic organic solvent. Illustrative of aprotic organic solvents which are suitable as reaction solvents in accordance with the present invention are those previously mentioned for use in connection with the preparation of the carbamate derivatives of the invention.

The acid acceptor utilized in carrying out the sulfenylation reaction may be either an organic or inorganic base. Organic bases useful as acid acceptors are tertiary amines, alkali metal alkoxides and the like. Bases such as sodium hydroxide, potassium hydroxide and sodium carbonate are illustrative of inorganic bases suitable for use in the conduit of this reaction. Preferred acid acceptors are aromatic and aliphatic tertiary amines, such as triethylamine, pyridine, trimethylamine, or 1,4-diazobicyclo-[2.2.2]octane.

When an organic base is used as the acid acceptor, phase transfer agents may be used to facilitate the transfer of the acid acceptor across the organic/inorganic phase interface. As useful phase transfer agents, there may be mentioned crown ether compounds, quaternary ammonium halide compounds and the like.

In these reactions, the reaction temperature may be varied between −30°C and 130°C, preferably between 0°C and 75°C.

The sulfenylation reactions can be conducted at either subatmospheric, atmospheric or superatmospheric pressures, but conventionally are conducted at atmospheric or autogenous pressure.

Reactants, intermediates or precursor compounds necessary in carrying out the reactions set forth herein are readily obtainable following conventional synthetic methods. For instance, N-methylcarbamyl fluoride may be prepared in accordance with the procedure detailed in *J. Org. Chem. 43,* 3953 (1978) as may N-(N,N-dialkylaminosulfenyl)-N-methylcarbamyl fluoride. N-[(N-alkyl-N-arylsulfonyl)aminosulfenyl[-N-methylcarbamyl fluoride is prepared according to the methods set forth in U.S. Patent No. 4,148,910. The preparations of bis[(N-fluorocarbonyl-N-methyl)amino]sulfide, bis[(N-fluorocarbonyl-N-methyl)-amino]disulfide and N-arenesulfenyl-N-methylcarbamyl fluoride are described in U.S. Patent No. 3,639,471, N-(substituted-cyanoalkanesulfenyl and thiosulfenyl-N-alkylcarbamyl halides are also described in U.S. Patent No. 4,058,549.

The active compounds are well tolerated by plants, have a favorable level of toxicity to warm-blooded animals and can be used for combating arthropod pests, especially insects and arachnids, and nematode pests. They are active against normally sensitive and resistant species and against all or some stages of development, i.e., eggs, larvae, nymphs, cysts and adults. The above-mentioned pests include:

from the class of the Isopoda, for example, *Oniscus asellus, Armadillium vulgare* and *Cylisticus convexus;* from the class of the Diplopoda, for example, *Blaniulus guttulatus;* from the class of the Chilopoda, for example, *Geophiluscarpophagus* and *Scutigera* spp.; from the class of the Symphla, for example, *Scutigerella immaculata*; from the order of the Thysanura, for example, *Lepisma saccharina*; from the order of the Collembola, for example, *Onychirus armatus*; from the order of the Orthoptera, for example, *Blatta orientalis, Periplaneta americana, Blattella germanica, Acheta domesticus,* Gryllotalpa spp., *Locusta migratoria migratorioides, Melanoplus differentialis* and *Schistocerca gregaria*; from the

order of the Isoptera, for example, *Reticuliternes spp.*; from the order of the Thysanoptera, for example *Hercinothrips femoralis* and *Thrips tabaci*; from the order of the Hemiptera, for example, *Lygus* spp., *Dysdercus* spp., *Nezara viridula*; from the order of the Homoptera, for example, *Bemesia tabaci, Trialeurodes* spp., *Aphis* spp., *Macrosiphum* spp., Myzus spp., *Empoasca* spp., *Nephotettix cincticeps, Psylla* spp.; from the order of the Lepidoptera, for example, *Pectinophora gossypiella, Plutella maculipennis, Malacosoma neustria, Porthetria dispar, Bucculatrix thurberiella,* Agrotis spp.; Euxoa spp., *Earias insulana,* Heliothis spp., *Prodenia litura,* Spodoptera spp., *Trichoplusia ni, Carpocapsa pomonella,* Pieris spp., chilo spp., *Pyrausta nubilalis, Ephestia kuehniella, Galleria mellonella, Choristoneura fumiferana,* and *Tortrix viridana*; from the order of the Coleoptera, for example, *Leptinotarsa decemlineata,* Diabrotica spp., *Epilachna varivestis, Oryzaephilus surinamensis,* Anthonomus spp., Sitophilus spp., *Hypera postica,* Dermestes spp., Trogoderma spp., Anthrenus spp., Attagenus spp., Lyctus spp., *Conotrachelus nenuphar, Popillia japonica,* Ptinus spp., Tribolium spp., *Tenebrio molitor,* Agriotes spp., Conoderus sp., *Melolonthas melolontha,* and *Costelytra zealandica*; from the order of the Hymenoptera, for example, Diprion spp., Hoplocampa spp., Lasius spp., *Monomorium pharaonis,* Vespa spp., and *Caliroa cerasi*; from the order of the Diptera, for example, Aëdes spp., Anopheles spp., Culex spp., *Drosophila melanogaster,* Musca spp., Fannia spp., *Calliphora erythrocephala,* Lucilia spp., Chrysomya spp., Cuterebra spp., Gastrophilus spp., Hyppobosca spp., Stomoxys spp., Oestrus spp., Hypoderma spp., Tabanus spp., Tannia spp., *Oscinella frit, Pegomyia hyoscyami, Ceratitis capitata, Dacus oleae,* and *Tipula paludosa;* from the class of the Arachnida, for example, *Scorpio maurus* and *Latrodectus mactans*; from the order of the Acarina, for example, *Acarus* spp., Argas spp., Ornithodoros spp., *Dermanyssus gallinae, Eriophyes ribis,* Boophilus spp., Rhipicephalus spp., Amblyomma spp., Hyalomma spp., Ixodes spp., Psoroptes spp., Chorioptes spp., Sarcoptes spp., Tarsonemus spp., *Bryobia praetiosa,* Panonythus spp., and Tetranychus spp.; from the order of the Phylum nematoda, for example, species of the following genera *Meloidogyne, Heterodera, Trichodorus, Xiphinema, Ditylenchus, Pratylenchus, Tylenchus, Radopholus, Longidorus,* and *Tylenchorhynchus.*

Exemplary of preferred compounds for use in the arthropodicidal compositions and methods of the present invention are compounds of formula (I) wherein A and J have the meanings defined above, X is a bridge member selected from

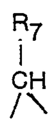 , 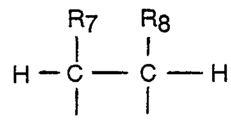 , 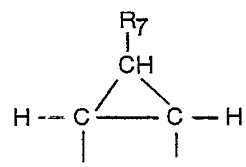 ,

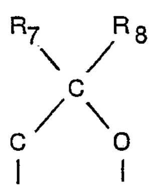 or 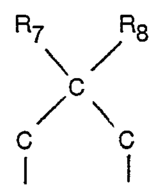 , where $R_7$ and $R_8$

independently represent hydrogen or cyano,

$R_1$—$R_1'''$, inclusive, and $R_2$—$R_2'''$, inclusive, are hydrogen or, when a is a double bond, $R_1$ and $R_2$ and, in addition, if q, independently in each case, is 1, $R_1''$ or $R_2''$ also are independently hydrogen or halogen and $R_1'$, $R_2'$, $R_1'''$ or $R_2'''$ are absent;

$R_3$ and $R_4$ independently represent hydrogen, halogen, cyano or alkoxycarbonyl;

Y represents

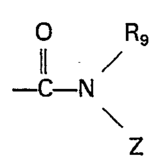

where $R_9$ represents hydrogen, alkyl, hydroxyalkyl, alkenyl or alkynyl; and Z represents

a) 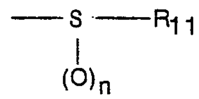 ,

where n is 0, 1 or 2 and $R_{11}$ is phenyl or phenyl substituted with at least one member selected from halogen, alkyl, alkoxy, trifluoromethyl, nitro and cyano;

b) 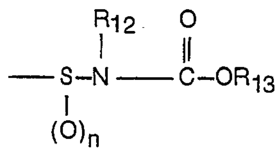 ,

where n is 0, 1 or 2, $R_{12}$ is alkyl and $R_{13}$ is alkyl, alkoxyalkyl or Q, where Q—OY represents formula (I) as defined above herein;

c) 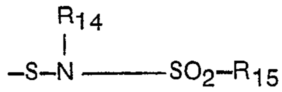 ,

where $R_{14}$ is alkyl and $R_{15}$ is alkyl or

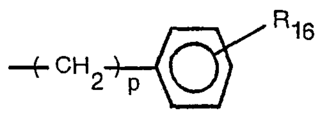

where p is 0—5 and $R_{16}$ is halogen, alkyl, trifluoromethyl, nitro, methoxy, cyano or dialkylamino;

d) $-S-NR_{17}R_{18}$,

where $R_{17}$ and $R_{18}$ are alkyl or aryl or together with the nitrogen atom represent

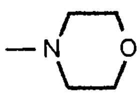 or 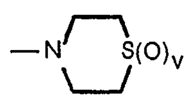 ,

where v is 0, 1 or 2,

e) $-(S)_n-R_{19}$ ,

where n is 1 or 2, and $R_{19}$ is alkyl, cyanoalkyl or alkoxycarbonyl;

f) 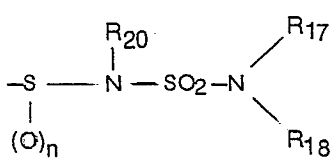 ,

where n is 0, 1 or 2, $R_{20}$ is alkyl and $R_{17}$ and $R_{18}$ have the meanings defined above herein;

g) 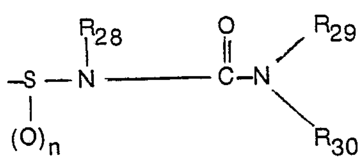 ,

where n is 0, 1 or 2, $R_{28}$ is alkyl or aryl and $R_{29}$ and $R_{30}$ independently represent hydrogen, alkyl, aryl or alkoxy; or

h) 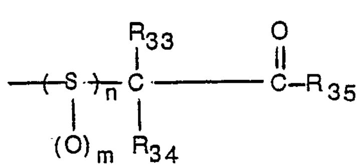 ,

where m is 0, 1 or 2, n is 1 or 2 and $R_{33}$, $R_{34}$ and $R_{35}$ independently represent hydrogen, alkyl or aryl.

As preferred compounds for use in the nematocidal compositions and methods of the present invention, there may be mentioned those compounds of Formula (I), wherein

A represents S or O;

J has the meaning defined above

X is a bridge member selected from

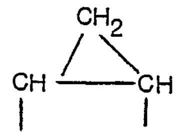 ;

$R_1$—$R_1'''$, inclusive, $R_2$—$R_2'''$, inclusive, $R_3$ and $R_4$ have the meanings defined above; and Y represents

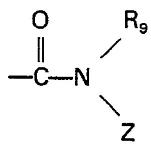

where $R_9$ represents hydrogen, alkyl, alkenyl or alkynyl; and Z represents

a) 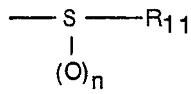 ,

where n is 0, 1 or 2, $R_{11}$ is alkyl, phenyl or phenyl substituted with at least one member selected from halogen, alkyl, alkoxy, trifluoromethyl, nitro and cyano;

b) 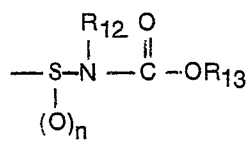 ,

where n is 0, 1 or 2, $R_{12}$ is alkyl and $R_{13}$ is alkyl, alkoxyalkyl or Q, where Q—OY represents formula (I) as defined above herein;

c) 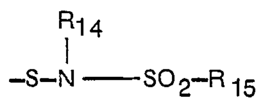 ,

where $R_{14}$ and $R_{15}$ are alkyl, aryl or substituted aryl with at least one substituent selected from halogen, alkyl, trifluoromethyl, nitro, methoxy, cyano and dialkylamino;

d) $-S-NR_{17}R_{18}$,

where $R_{17}$ and $R_{19}$ are alkyl or aryl or together with the nitrogen atom represent

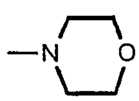 , 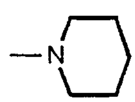 or 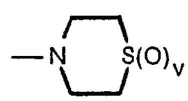 ,

where v is 0, 1 or 2;

e) 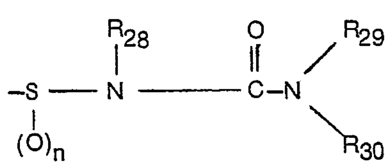 ,

where n is 0, 1 or 2, $R_{28}$ is alkyl or aryl and $R_{29}$ and $R_{30}$ independently represent hydrogen, alkyl, aryl or alkoxy; and

f) 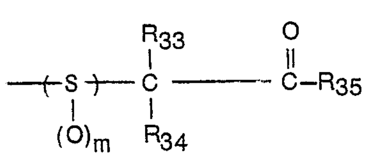 ,

where m is 0, 1 or 2, n is 1 or 2 and $R_{33}$, $R_{34}$ and $R_{35}$ independently represent hydrogen, alkyl or aryl.

In general, in the foregoing arthropodicidal and nematocidal compositions of the invention as well as the methods of selectively killing, combating or controlling such pests, the compounds of the invention, either alone or in admixture, will be applied to the pests or their habitat, including growing crops, in an arthropodicidally or nematocidally effective amount of the compound(s) within the range from 0.112 to 22.4 kg/ha (0.1 to 20 lb/A) and, preferably, from 0.28 to 1.68 kg/ha (0.25 to 1.5 lb/A), for arthropods and from 0.14 to 22.4 kg/ha (0.125 to 20 lb/A) and, preferably, from 0.28 to 4.48 kg/ha (0.25 to 4 lb/A) for nematodes. The $LD_{50}$ of the carbamate ester derivatives of the invention in rats is generally in the range from 1.5 to 3 mg/kg, whereas the sulphenylated derivatives exhibit considerably reduced toxicities, i.e., up to a 15 fold decrease or greater.

The pesticidally active compounds in accordance with the present invention may be utilized in the form of formulations or compositions with appropriate dispersible pesticide carrier vehicles. As employed typically in the methods of the present invention, such compositions will contain between 0.1 and 98 percent by weight, and usually between 1 percent and 90 percent by weight of active compound. The heterobicyclic compounds of the invention are practically insoluble in water and only sparingly soluble in organic solvents. Accordingly, the compounds of the present invention are formulated in accordance with conventional practices in the form of, for example, wettable powders, dust or emulsifiable concentrates, water-based flowables, and dispersible granules. In such compositions, the active compound of the invention will be combined with suitable dispersing agents (e.g., lignin, sulphite waste liquors or methyl cellulose,), surfactants, such as nonionic and anionic emulsifying agents (e.g., polyethylene oxide esters of fatty acids, polyethylene oxide ethers of fatty alcohols, alkyl sulphonates, aryl sulphonates, alkyl aryl-polyglycol ethers or magnesium stearate), and appropriate solid pesticidally acceptable carriers or diluents (e.g., kaolins, alumina, silica, calcium carbonate or talc). In general, in preparing the active compound for incorporation in such formulations, the compound will be subjected to conventional comminuting treatment, such as air milling, hammer milling, ball milling or wet milling, to obtain an average particle size of 3 to 5 μ. Depending upon the ultimate intended use and particular storage conditions, other optional adjuvants such as anticaking agents, antifoam agents and freeze-thaw depressants may be incorporated in the compositions. Likewise, the compounds of the present invention may be employed in combination with other pesticidal agents, including, for example, insecticides, miticides, bactericides, fungicides, nematocides, herbicides and plant growth regulants. Moreover, the compounds of the present invention have surprisingly demonstrated good activity for destroying parasitic worms. Thus their use as anthelmintic agents is also contemplated herein.

The following nonlimiting examples are afforded in order that those skilled in the art may more readily understand the present invention and specific preferred embodiments thereof with respect to the preparation of starting materials, intermediates and product compounds in accordance with the foregoing description. The assigned structures for the title compounds exemplified below as well as in compound Table 1 thereafter are consistent with nuclear magnetic resonance and infrared spectra and, where applicable, X-ray crystallography.

## Example 1

2-Thiabicyclo[2.2.1]hept-5-en-3-one oxime

A stirred solution of 33 g (0.5 m) of cyclopentadiene in 150 ml of petroleum ether was maintained at 0° to −10°C as 23 g (0.2 m) of thiophosgene was added over 25 minutes. The colorless solution was stripped of volatiles. A solution of the residue in 200 ml of 1,2-dimethoxyethane was added over one hour at 0°C to 2.0 m of hydroxylamine in 500 ml of water. The reaction mixture was stirred overnight and allowed to come to room temperature. A dried methylene chloride extract was stripped to yield a semi-solid residue which was purified by dry column chromatography (silica gel, ethyl ether:hexane:1:1). Two bands were observed. Extraction of each individually yielded 2.4 g of the less polar material, m.87—88°C and 5.9 g of the more polar, m.137—137.5°C. The NMR and IR spectra indicate these to be the E and Z isomers of the title compound. The more polar material was identified as the Z isomer by X-ray crystallography.

## Example 2A

2-Thiabicyclo[2.2.2]oct-5-en-3-one oxime

A solution of 735 ml (7.3 m) of 1,3-cyclohexadiene in 650 ml of chloroform was stirred at 30—50°C while 600 ml (7.86 m) of thiophosgene was added over one hour. The mixture was stirred at 60±5°C for two hours, cooled, and added over 90 minutes at 25—40°C to a stirred mixture of hydroxylamine (from 2300 g (28 m) of hydroxylamine sulfate, 2700 ml of water, and 1130 g (27.8 m) of sodium hydroxide) and 800 ml of chloroform. The resulting mixture was stirred at 40±5°C for four hours, filtered, and the filter cake washed with chloroform. The chloroform layer from the combined filtrate and washings was separated, dried, and reduced in volume. After the addition of three liters of carbon tetrachloride, the solvent was distilled until a pot temperature of 75°C was reached. The carbon tetrachloride solution was decanted from some gummy insolubles and allowed to cool to yield the title compound as a pale yellow solid, m.118.5—122°C.

Calculated for $C_7N_9NOS$: C, 54.2; H, 5.9; N, 9.0; S, 20.7
Found: C,53.7; H, 6.0; N, 9.0; S, 20.4

*Method B*

A solution of 0.22 g (0.00085 m) of 3,3-*bis*-(1,2,4-triazol-1-yl)-2-thiabicyclo[2.2.2]oct-5-ene [*J. Org. Chem.* 45, 3713 (1981)] in 30 ml of *tert*-butyl alcohol was treated with 0.75 g (0.011 m) of hydroxylamine hydrochloride. The mixture was heated under reflux, cooled, and partitioned between water and dichloromethane. The organic layer was dried over $MgSO_4$. The residue after removal of solvent was purified by thick layer chromatography to yield 0.036 g of product which was spectrometrically identical with that obtained in Method A of this example.

Example 2B

Reaction of 2-thiabicyclo[2.2.2]oct-5-en-3-one with hydroxylamine

A solution of 1.4 g (0.01 m) of 2-thiabicyclo[2.2.2]oct-5-en-3-one [*J. Org. Chem. 38,* 2637 (1973)] in 30 ml of dimethoxyethane was added to a mixture of 3.01 g (0.04 m) of hydroxylamine hydrochloride and 2.27 g (0.02 m) of sodium carbonate in 30 ml of water and 30 ml of dimethoxyethane at 0°C. The mixture was stirred at 0—10°C for 3 hours. TLC in ethyl acetate/hexane/ethanol ([1:1.0:1) showed two intense spots (Rf 0.02 and 0.25). the mixture was extracted with methylene chloride. A white solid precipitated out in the aqueous layer. This was filtered and washed with water and methylene chloride to give 0.34 g of a compound identified by spectral analysis as 4,4'-(N-hydroxycarbamyl)cyclohex-2-enyl disulfide (*2*), m. 156—158°C, Rf=0.02, H'—NMR (DMSO—$d_7$): δ 1.5—2.28 (4H, m, $CH_2$), 2.65—3.1 (1H, m, CHCO), 3.4—3.7 (1H, m, SCH), 5.78 (2H, s, CH), 8.73 (1H, s, NH), 10.2 (1H, s, OH); IR (KBr): 3150 (NHOH), 1610 $cm^{-1}$ (C=0).

The methylene chloride extract was dried with magnesium sulfate and filtered. After the solvent was removed at reduced pressure, the mixture was chromatographed on dry column silica gel using ethyl acetate/hexane/ethanol (1:1:0.1) as eluent to give a solid identified by NMR and IR spectroscopy as N-hydroxy-(4-mercaptocyclo-hex-2-enyl)carboxamide (*1*), m. 100—101°C. Rf=0.25, H'—NMR (DMSO—$d_6$): δ 1.95—2.2 (4H, m, $CH_2$), 2.6—3.05 (1H, m, CHCO), 3.3—3.8 (1H, m SCH), 5.3—6.0 (2H, m, CH), 8.7 (1H, s, NH), 10.2 (1H, s, OH); IR (KBr); 3200 (OH), 3035 (NH), 2550 (SH), 1612 $cm^{-1}$ (C=O). Compound *1* gradually converted to compound *2*.

None of the 2-thiabicyclo[2.2.2]oct-5-en-3-one oxime was observed.

Example 3

2-Thiabicyclo[2.2.2]oct-5-en-3-one oxime

Thiophosgene (296 g of 85%, 2.19 m) was added dropwise to a stirred solution of 167 g (2.08 m) of 1,3-cyclohexadiene in 500 ml of chloroform. The exothermic reaction caused the temperature to rise from 25°C to 68°C with refluxing of the solvent. After 45 minutes, the reaction mixture was cooled and added in portions over four hours to a stirred solution of hydroxylamine (prepared from 480 g (6.9 m) of hydroxylamine hydrochloride in 175 ml water by dropwise addition at 0°C of a solution of 434 g of 89% potassium hydroxide (6.9 m) in 250 ml of water) at −15°C. After being stirred overnight, the reaction mixture was filtered and the filter cake washed with methylene chloride. The combined filtrate and washings were separated and the organic layer dried ($MgSO_4$) and reduced in volume at about 20 Torr. After addition of carbon tetrachloride to the residue, distillation of solvent was continued at atmospheric pressure until a pot temperature of 75°C was attained. The hot mixture was filtered. Cooling of the filtrate gave a solid which was recrystallized from carbon tetrachloride to yield 271 g of the title compound.

Example 4

Z-2-Thiabicyclo[2.2.1])hept-5-en-3-one oxime

*Method A*

A stirred solution of 14.5 g (0.22 m) of cyclopentadiene in 50 ml of petroleum ether was maintained at 0±5°C as 23 g (0.2 m) of thiophosgene was added over 40 minutes. The colorless solution was stripped of volatiles on a rotary evaporator. A solution of the residue in 100 of 1,2-dimethoxyethane was added at −5°C to a stirred slurry of 55.8 g (0.8 m) of hydroxylamine hydrochloride, 115 g (0.4 m) of sodium carbonate decahydrate, 90 ml of water and 100 ml of 1,2-dimethoxyethane. The mixture was stirred overnight as the temperature was allowed to reach 25°C.

The upper liquid layer was separated, combined with a methylene chloride extract of the lower aqueous layer, washed and dried ($MgSO_4$). A small portion was removed and stripped to dryness. The presence of E and Z isomers of the title compound was demonstrated by nuclear magnetic resonance and thin layer chromatography. The methylene chloride solution was treated with 2.5 ml of trifluoroacetic and allowed to stand at room temperature for four days. The solvent was stripped to yield 23.3 g of pale tan residue. This was confirmed as the Z isomer of the title compound by thin layer chromatography and nuclear magnetic resonance versus an authentic sample.

*Method B*

Thiocarbonyldiimidazole [9.0 g, (10.0 g of 90 percent technical material), 0.05 ml was dissolved in 250 ml of dichloromethane. The solution was treated with 10 ml of freshly cracked cyclopentadiene, and the mixture was refluxed for 4 hours. Evaporation of the solvent gave 16.8 g of a dark brown oil, the NMR of which was consistent with the structure, 3,3-bis(1-imidazolyl)-2-thiabicyclo[2.2.1]hept-5-one.

A portion (11.1 g, 0.033 m) of the oil was dissolved in 200 ml of absolute ethanol, and the solution was treated with 9.5 g of hydroxylamine hydrochloride. the mixture was refluxed for 2 hours. The solvent was evaporated, and the residue was partitioned between water and dichloromethane. The organic layer and 4 dichloromethane extracts of the aqueous layer were dried ($MgSO_4$), filtered with carbon and the solvent was evaporated. The residual brown oil was taken up in dichloromethane and hexane. Upon standing an oil was deposited. The solution was decanted, the solvent was evaporated, and residue was triturated with dry ether. The ether soluble material was chromatographed on silica (preparative layer, 50 percent ether in hexanes). The purified material was eluted with acetone; there was obtained 1.51 g of material which was spectrometrically identical with the compound prepared by method 4A.

Example 5

6,7-Dibromo-2-thiabicyclo[2.2.1]heptan-3-one oxime

A solution of 0.036 m of bromine in 167 ml of carbon tetrachloride was added over 30 minutes at 23—30°C to a stirred slurry of 5.0 g (0.035 m) of 2-thiabicyclo[2.2.1]hept-5-en-3-one oxime in 50 ml of carbon tetrachloride. After a further 30 minutes, the solvent was removed to yield 10.6 g of white solid residue, m. 168°C. Recrystallization from acetone gave 6.9 g of the title compound as a white solid m. 168°C (dec.).

| | | | |
|---|---|---|---|
| Calc'd for $C_6H_7Br_2NOS$: | C, 23.9; | H, 2.4; | N, 4.7. |
| Found: | C, 24.0; | H, 2.4; | N, 4.7. |

Example 6

2-Oxabicyclo]2.2.2]oct-5-en-3-one-oxime

To a 0°C solution of hydroxylamine [from 4.2 g (0.06 m) of hydroxylamine hydrochloride, 8.6 g (0.03 m) of sodium carbonate decahydrate and 30 ml of water] in 50 ml of 1,2-dimethoxyethane was added dropwise, with stirring under nitrogen, 4.4 g (0.02 m) (N-chlorosulfonyl)imino-2-oxabicyclo[2.2.2]oct-5-ene [*J. Chem. Soc.,* Perkin I, 874 (1977)] in 200 ml of 1,2-dimethoxyethane. After being stirred for 18 hours, the mixture was filtered to give 0,5 g of solid. Ether extraction of the filtrate yielded an addition 1.3 g of product. The combined solids were recrystallized from ethanol to provide the title compound as a white solid, m.p. 153—4°C.

| | | | |
|---|---|---|---|
| Calc'd for $C_9H_{12}N_2O_3$: | C, 60.4; | H, 6.5; | N, 10.1. |
| Found: | C, 60.7; | H, 6.8; | N, 10.2. |

Example 7

2-Thiabicyclo[2.2.2]oct-5-en-3-one O-(2-tetrahydropyranyl)oxime (Intermediate)

A solution of 10 g (0.06 m) of 2-thiabicyclo[2.2.2]oct-5-en-3-one oxime, 9 g (0.1 m) of 98% dihydropyran, and a catalytic amount of p-toluenesulfonic acid in 30 ml of methylene chloride was heated under reflux overnight. Sodium bicarbonate was added to neutrality. The residue after removal of solvent was purified by dry column chromatography (silica gel-ethyl acetate:hexane::1:1) to give 14.4 g of yellow solid. Recrystallization from ether-hexane yielded 10.1 g of the title compound as a white solid, m. 84—5°C.

| | | | |
|---|---|---|---|
| Calc'd for $C_{12}H_{17}NO_2S$: | C, 60.2; | H, 7.2; | N, 5.9. |
| Found: | C, 60.0; | H, 7.2; | N. 5.9. |

Example 8

5,6-Dihydroxy-2-thiabicyclo[2.2.2]octan-3-one O-(2-tetrahydropyranyl)oxime (Intermediate)

A mixture of 8 g (0.033 m) of the product of Example 7, 5.3 g (0.039 m) of N-methylmorpholine N-oxide, 4 ml of 2.5% osmium tetroxide in t-butanol, and 20 ml of acetone was stirred for four hours then extracted with ethyl-acetate. The organic solution was washed with brine, dried, and stripped to give 12 g of crude product which was purified by dry column chromatography (silica gel, ethylacetate:hexane: 1:1) to yield 5.8 g of the title compound as a white solid, M.124—6°C.

| | | | |
|---|---|---|---|
| Cal'd for $C_{12}H_{19}NO_4S$: | C, 52.7; | H, 7.0; | N, 5.1. |
| Found: | C, 52.6; | H, 7.4; | N, 5.0. |

Example 9

5,6-Dimethoxy-2-thiabicyclo[2.2.2]octan-3-one oxime

To a suspension of 1.4 g (0.03 g) of 60% sodium hydride in mineral oil was added a solution of 4 g (0.015 m) of the product of Example 8 in tetrahydrofuran. The mixture was stirred until gas evolution

ceased, then cooled to 0°C and treated with 2.3 g (0.015 m) of methyl iodide. This mixture was stirred at 40°C. After slow addition of a little methanol, followed by water, the mixture was extracted with methylene chloride. The organic solution was washed with brine, dried and stripped to give 6 g of oil which was purified by high performance liquid chromatography to yield an oil.

A solution of 1.8 g (0.0033 m) of this oil and 3 drops of 5N hydrochloric acid in 80 ml of methanol was heated under reflux overnight. Water was added. A methylene chloride extract of this mixture was dried and stripped to yield 1.25 g of the title compound as a solid.

Example 10

2-Thiabicyclo[3.2.2]non-5-en-3-one oxime

A solution of 29 g (0.3 m) of 1,3-cycloheptadiene, 46 g (0.4 m) of thiophosgene, and 25 ml of cyclohexane was heated under reflux for 24 hours, then stripped of volatiles under reduced pressure. The oily residue was taken up in 150 ml of methylene chloride and stirred with 1.5 m of hydroxylamine in 200 ml of water at room temperature for three days. The separated organic layer was dried over magnesium sulfate and stripped to yield 26.7 g of viscous oil.

Purification by dry column chromatography using silica gel and 1:4::ether:hexane as eluant. There was obtained 14 g of yellow solid. Recrystallization of 2 g portion from 1:1 ether-hexane yielded 1.4 g of the title compound as white crystals, m 130—132-C.

| | | | | |
|---|---|---|---|---|
| Calc'd for $C_8H_{11}NOS$: | C, 56.8; | H, 6.6; | N, 8.3; | S, 18.9. |
| Found: | C, 57.1; | H, 6.7; | N,8.2; | S, 18.5. |

Example 11

2-Thiabicyclo[3.2.2]nonan-3-one oxime

Air was passed through a stirred solution of 3.4 g (0.02 m) of the product of Example 10, 20 ml of 85% hydrazine hydrate, and 0.1 g of cupric acetate in 50 ml of isopropyl alcohol for six hours at room temperature. The mixture was poured into 300 ml of water. A methylene chloride extract was washed with water, dried over magnesium sulfate, and stripped to yield 3.7 g of solid residue. Dry column chromatography on silica gel using 1:4::ether:hexane yielded 3.1 g of the title compound as a white solid, m. 146—8°C.

| | | | | |
|---|---|---|---|---|
| Calc'd for $C_8H_{13}NOS$: | C, 56.1; | H, 7.7; | N, 8.2; | S, 18.7. |
| Found: | C, 56.0; | H, 7.9; | N, 7.9; | S, 18.6. |

Example 12

N'N'-thiobis[(methylimino)carbonyloxy]-2-thiacicyclo[2.2.2]octan-3-imine

A solution of 10.7 g (0.05 m) of 2-thiabicyclo[2.2.2]octan-3-one O-[(methylamino)carbonyl]oxime in 30 ml of pyridine was cooled at 0°C as 3.8 g (0.028 m) of sulfur monochloride was added over five minutes. The mixture was stirred at 0°C overnight, poured onto ice and water, and filtered. The filter cake was washed with cold dilute hydrochloric acid, then with water and dried to yield 12.2 g of solid, m. 158—62°C. Recrystallization from hot 2-butanone gave the title compound as an off-white solid, m. 174—6°C (dec.).

| | | | |
|---|---|---|---|
| Calc'd for $C_{18}H_{26}N_4O_4S_3$: | C, 47.1; | H, 5.7; | N, 12.2. |
| Found: | C, 47.3; | H, 5.9; | N, 12.3. |

Example 13

2-Thiabicyclo[2.2.2]octan-3-one O-[N-methyl-N-([N-methyl-N-butoxy-carbonyl)amino]thio)carbonyl]oxime

To a stirred solution of 10.7 g (0.05 m) 2-thiabicyclo[2.2.2]octan-3-one O[(methylamino)carbonyl]oxime and 4.4 g (0.055 m) of pyridine in 100 ml of methylene chloride at 0-C was added 10.9 g (0.055 m) of butyl N-chlorothio-N-methylcarbamate. After being stirred overnight as it was allowed to come to room temperature, the reaction mixture was diluted with 200 ml of ethyl ether, then washed with three 150 ml portions of water. The organic solution was dried over magnesium sulfate, filtered, and the filtrate stripped of solvent. The residue was purified by dry chromatography (silica gel, hexane:ethyl ether::1:1) to yield 12 g of the title substance as a white powder, m. 77.5—80°C.

| | | | |
|---|---|---|---|
| Calc'd for $C_{15}H_{25}N_3O_4S_2$: | C, 48.0; | H, 6.7; | N, 11.2. |
| Found: | C, 48.0; | H, 6.9; | N, 11.2. |

Example 14

2-Thiabicyclo[2.2.2]octan-3-one O-[N-(N-fluorocarbonyl-N-methyl)aminothio]-n-methylaminocarbonyloxime

To a cooled (−10°C) solution of 18.4 g (0.1 m) of bis[N-fluorocarbonyl-N-methyl)amino]sulfide and 15.7 g (0.1 m) of 2-thiabicyclo[2.2.2]octan-3-one oxime in 200 ml of methylene chloride was added, over 30 minutes, 14.0 ml (0.1 m) of triethylamine. After being stirred at −10°C for 40 minutes, the reaction mixture was poured directly onto a silica gel dry column which was developed with ether:hexane::1:1. This gave

26.4 g of solid. Purification on HPLC and dry column chromatography using ether yielded the title compound as a solid, m. 98—100°C.

| | | | | |
|---|---|---|---|---|
| Calc'd for $C_{11}H_{16}FN_3O_3S_2$: | C, 41.1; | H, 5.0; | N,13.1; | S, 20.0. |
| Found: | C, 41.2; | H, 5.3; | N, 13.3; | S, 20.1. |

Example 15

2-Thiabicyclo[2.2.2]octan-3-one O-(N-methyl)]-N-[N-methyl-N-(1-methylthio-ethylideneamino-oxycarbonyl)aminothio]aminocarbonyloxime

To a stirred solution of 3.2 g (0.01 m) of the product of Example 14 and 1.05 g (0.01 m) of methylthioacetohydroxamate in 50 ml of methylene chloride was added 1.4 ml (0.01 m) of triethylamine. The reaction mixture was stirred overnight, washed with water, dried and stripped to give 4.3 g of a white solid. Purification by dry column chromatography (silica gel, ether) yielded 3.25 g of the title compound as a white solid, m. 176—9°C.

| | | | | |
|---|---|---|---|---|
| Calc'd for $C_{14}H_{22}N_4O_4S_3$: | C, 41.4; | H, 5,5; | N, 13.8; | S, 23.7. |
| Found: | C, 41.2: | H, 5.8; | N, 13.7; | S, 23.4. |

Example 16

2-Thiabicyclo[2.2.1]hept-5-en-3-one O-[N-methyl-N-(2-cyano-2-propylthiosulfenyl)]aminocarbonyloxime

A solution of 6.1 g (0.043 m) of 2-thiabicyclo[2.2.1]hept-5-en-3-one oxime and 9.0 g (0.043 m) of N-methyl-N(2-cyano-2-propylthiosulfenyl)aminocarbonyl chloride in 200 ml of methylene chloride was treated with 4.4 g (0.43 m) of triethylamine. After being stirred overnight, the mixture was washed with three portions of water, dried, and stripped to give 14.6 g of brown gum. Purification by dry column chromatography (silica gel, ether:hexane::9:1) yielded 8 g of the title compound as an off-white solid, m. 76-8°C.

| | | | |
|---|---|---|---|
| Calc'd for $C_{12}H_{15}N_3O_2S_3$: | C, 43.8; | H,4.6; | N, 12.8. |
| Found: | C, 43.5; | H, 4.8; | N, 12.6. |

Example 17

2-Thiabicyclo[2.2.1]hept-5-en-3-one O-(N-methyl-[(N-methyl)-N-(P-toluenesulfonyl)aminosulfenyl]aminocarbonyloxime

A solution of 6.8 g (0.048 m) of 2-thiabicyclo[2.2.1]-hept-5-en-3-one oxime and 14.0 g (0.048 m) of (N-methyl)-[N-methyl-N-(p-toluenesulfonyl)-amino]sulfenylaminocarbonyl fluoride in 100 ml of methylene chloride was treated with 4.8 g (0.048 m) of triethylamine. After being stirred for 72 hours, the solution was washed with water, dried, and stripped to give 13.2 g of dark viscous residue. Purification by dry column chromatography (silica gel, ethyl ether) yielded 6 g of the title compound as a brown solid, m. 120—122°C.

| | | | |
|---|---|---|---|
| Calc'd for $C_{16}H_{19}N_3O_4S_3$: | C, 46.5; | H, 4.7; | N, 10.2. |
| Found: | C, 46.2; | H, 4.7; | N, 10.2. |

Example 18

2-Thiabicyclo[2.2.2]octan-3-one O-(N-methyl)-N-(3-trifluoromethylphenylthio)aminocarbonyloxime

To a stirred suspension of 5.36 g (0.025 m) of 2-thiabicyclo[2.2.2]-octan-3-one O[(methylamino)carbonyl]oxime in 50 ml of carbon tetrachloride was added 3.96 (0.025 m) of 1,5-diazabicyclo[5.4.0]-undec-5-ene. The reaction mixture was cooled at −10°C during the addition of 6.38 g (0.03 m) of 3-trifluorobenzenesulfenyl chloride in 25 ml of carbon tetrachloride. After being stirred for 18 hours, the reaction mixture was filtered. Stripping of the filtrate gave 6 g of an oil which was purified by dry column chromatography (silica gel, ether) to give 3.1 g of solid, recrystallization from ethanol yielded the title compound as a white solid, m. 60—2°C.

| | | | |
|---|---|---|---|
| Calc'd for $C_{16}H_{17}F_3N_2O_2S_2$: | C, 49.2; | H, 4,4; | N, 7.2. |
| Found: | C, 49.1; | H, 4.7; | N, 7.1. |

Example 19

4-Cyano-2-thiabicyclo[2.2.2]oct-5-en-3-one O-(N-methyl)-N(4-butylphenylthio)aminocarbonyloxime

To a stirred mixture of 5.9 g (0.03 m) of 4-cyano-2-thiabicyclo-[2.2.2]oct-5-en-3-one oxime and 7.24 g (0.03 m) of N(p-t-butylphenylthio)-N-methylcarbamyl fluoride in 150 ml of methylene chloride was added 3.04 g of triethylamine in 50 ml of methylene chloride. After being stirred for 72 hours, the reaction mixture was stripped. Purification of the residue by dry column chromatography gave a solid which was recrystallized from ethanol to yield 3.8 g of the title compound as a white solid, m. 148—9°C.

| | | | |
|---|---|---|---|
| Calc'd for $C_{20}H_{23}N_3O_2S_2$: | C, 59.8; | H, 5.8; | N, 10.5. |
| Found: | C, 59.8; | H, 6.0; | N, 10.6. |

Example 20

2-Thiabicyclo[2.2.2]oct-5-ene-3-one O-(N-methyl)-N-(4-tertbutylphenylthio)aminocarbonyl oxime, hydrate

A flame dried 3-neck flask was equipped with a gas inlet tube, mechanical stirrer and injection septum. Under nitrogen atmosphere, 4.37 g (.03 m) 2,2,6,6-tetramethylpiperidine was dissolved in 100 ml THF (distilled from LAH) with stirring, while 18.8 ml of a 1.6 molar (in hexane) solution on n-butyllithium was injected through the septum. The reaction mixture assumed a yellow color. Stirring was continued for 10 minutes, after which the reaction mixture was cooled to −78°. Over a two-minute period, a solution of 6.38 g (.03 m) of 2-thiabicyclo[2.2.2]oct-5-ene-3-one O-(N-methylaminocarbonyl)oxime in 100 ml. THF was added. Stirring was continued for 5 minutes, 6.03 g (.03 m) p-t-butylbenzenesulfenyl chloride in 100 ml tetrahydrofuran was added dropwise during 15 minutes, and the reaction mixture was allowed slowly to come to ambient temperature over an 18-hour period. The reaction mixture was then quenched with saturated aqueous ammonium chloride, extracted with 300 ml ether and water washed five times. The ether extract was dried over $MgSO_4$, filtered and the solvent removed under reduced pressure to give 9 g of a brown oil which was chromatographed on a silica gel dry column (ether) to give 2.51 g (21 percent) of the title compound as a yellow oil. Crystallization was induced by trituration with ether. Recrystallization from ethanol afforded pure product (0.6 g), m. 119—120°C.

| | | | |
|---|---|---|---|
| Calc'd for $C_{19}H_{24}N_2O_2S_2H_2O$: | C, 57.8; | H, 6.6; | N, 7.1. |
| Found: | C, 57.8; | H, 6.3; | N, 7.1. |

Example 21

N,N'-dithiobis[(methylimino)carbonyloxy]-2-thiabicyclo[2.2.1]hept-5-en-3-imine

A solution of 10.1 g (0.1 m) of triethylamine in 25 ml of methylene chloride was added over 30 minutes at 0°C to a stirred solution of 14.1 g (0.1 m) of 2-thiabicyclo[2.2.1]hept-5-en-3-one oxime and 10.8 g (0.05 m) of bis[(N-methyl-N-fluorocarbonyl)amino]disulfide in 200 ml of methylene chloride. The mixture was allowed to give a semisolid residue. After being passed through a short column of silica gel (ethyl acetate as eluent), the material was purified by dry column chromatography (silica gel, methylene chloride:acetone::95:5) to give a brown syrup. Trituration with ether gave 10.2 g of the title compound as a tan solid, m. 148—150°C (dec.).

| | | | | |
|---|---|---|---|---|
| Calc'd for $C_{16}H_{18}N_4O_4S_4$: | C, 41.9; | H, 4.0; | N, 12.2; | S, 28.0. |
| Found: | C, 41.9; | H, 4.2; | N, 12.1; | S, 27.8. |

Example 22

2-Azabicyclo[2.2.1]hept-5-en-3-one oxime

To a solution of hydroxylamine (from 2.1 g (0.03 m) of hydroxylamine hydrochloride, 4.3 g (0.015 m) of sodium carbonate, 10 ml of water and 200 ml of tetrahydrofuran) at 0°C was added 5.16 g (0.021 m) of 3-tosyl-2-azabicyclo[2.2.1]hepta-2,5-diene (prepared by the procedure of *J. Organic Chemistry,* 39, 564 (1974)) in 50 ml of tetrahydrofuran. After being stirred overnight, during which time the temperature was allowed to warm to 25°C, the organic layer was separated, dried, and stripped of solvent. Purification of the residue by chromatography (silica gel: 1:1::dichloromethane:ethyl acetate) gave 0.8 g of material whose spectra were consistent with the title structure.

Example 23

2-Methyl-2-azabicyclo[2.2.2]octan-3-one oxime

A slurry of 33.0 g (0.218 m) of 4-(methylamino)benzoic acid and 4.0 g of 5% rhodium of alumina catalyst in 350 ml of water was shaken under 1500 psig of hydrogen for 24 hours. The insolubles were removed by filtration. The slurry from partial removal of water under reduced pressure was diluted with 300 ml of N,N-dimethylformamide and chilled. The precipitated solid was filtered, washed with several portions of cold acetone, and dried to yield 23 g (75%) of 4-(methylamino)cyclohexanecarboxylic acid.

The crude acid in 200 ml of diphenyl ether was heated at 230—250°C for 20 minutes with removal of water. Removal of the diphenylether under reduced pressure gave 10.6 g (52%) of 2-methyl-2-azabicyclo[2.2.2)octan-3-one.

A solution of this in 100 ml of tolene was added to a mixture of 28.3 g (0.07 g) of 4-methoxyphenylthionophosphine sulfide in 100 ml of toluene. After 3 hours under reflux, the cooled mixture was filtered and the filtrate evaporated to dryness. The oily residue was purified by column chromatography (silica gel: 3:1::ether:petroleum ether) to yield 8.8 g of 2-methyl-2-azabicyclo[2.2.2]octan-3-thione.

A solution of the thione in 50 ml of acetone was added to 80.9 g (0.57 m) of methyl iodide under nitrogen at room temperature. After being stirred overnight, the mixture was chilled and filtered under nitrogen to give 10.6 g of solid. A partial solution of this solid in 75 ml of chloroform was added dropwise to a stirred solution of hydroxylamine (from 5 g (0.072 m) hydroxylamine hydrochloride, 15.5 g (0.054 m) of hydrated sodium carbonate, 10 ml of water, and 150 ml of chloroform) at 0°C. After 6 hours, during which the mixture was allowed to warm to room temperature, the organic phase was separated, dried and stripped to yield 4.4 g of residue whose spectra were consistent with the title structure.

The following compounds were prepared using the foregoing synthesis procedures with appropriate selection of substituted dienes,, dienophiles and carbamylating agents to obtain the derivatives of formula I set forth in Tables I—V.

TABLE I

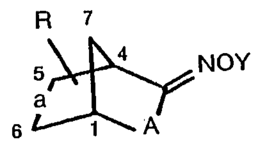

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 34 | S | double | H | $C(=O)N(CH_3)S-N(CH_3)C(=O)Q$ | 156 | 45.1 | 4.2 | 13.1 | 44.8 | 4.4 | 12.9 |
| 42 | S | double | | $C(=O)N(CH_3)S-C_6H_4C(CH_3)_3-4$ | 99–100 | 59.6 | 6.1 | 7.7 | 59.7 | 6.2 | 7.7 |
| 47 | S | double | H | $C(=O)N(CH_3)S-N(CH_3)SO_2N(CH_3)_2$ | 109–110 | 36.0 | 5.0 | 15.3 | 36.0 | 5.1 | 15.3 |
| 50 | S | double | H | $C(=O)N(CH_3)S-C_6H_5$ | oil | 54.9 | 4.6 | 9.1 | 54.5 | 4.8 | 8.7 |
| 54 | S | double | H | $C(=O)N(CH_3)S-NCH_2CH_2OCH_2CH_2$ | 115–116 | 45.7 | 5.4 | 13.3 | 45.5 | 5.3 | 13.2 |
| 63 | S | double | H | $C(=O)N(CH_3)SC_6H_4CH_3-2$ | 115–116 | 56.2 | 5.0 | 8.7 | 56.0 | 5.3 | 8.5 |
| 74 | S | double | H | $C(=O)N(CH_3)SC_6H_4Cl-4$ | 64–68 | 47.3 | 3.8 | 8.2 | 49.1 | 4.0 | 8.1 |
| 79 | S | double | H | $C(=O)N(CH_3)SC_6H_3CH_3-4$ | 73–74 | 56.2 | 5.0 | 8.7 | 56.2 | 5.2 | 8.6 |
| 86 | S | double | H | $C(=O)N(CH_3)SN(CH_2CH_2CH_3)SO_2C_6H_4-CH_3-4$ | 85–87 | 50.0 | 5.3 | 9.5 | 50.0 | 5.6 | 9.8 |
| 87 | S | double | H | $C(=O)N(CH_3)SN(C_4H_9\text{-}n)SO_2C_6H_4CH_3-4$ | oil | 50.1 | 5.5 | 9.2 | 50.3 | 5.9 | 9.0 |
| 91 | S | double | H | $C(=O)N(CH_3)SC_6H_4Br-4$ | 67–69 | 43.6 | 3.4 | 7.3 | 43.4 | 3.4 | 7.1 |
| 93 | S | double | H | $C(=O)N(CH_3)SN(CH(CH_3)_2)SO_2C_6H_4-CH_3-4$ | 155–157 | 49.0 | 5.2 | 9.5 | 48.8 | 5.4 | 9.6 |

TABLE I (Continued)

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 97 | S | single | 5,6–$Cl_2$ | H | 137–138 | 34.0 | 3.3 | 6.6 | 33.7 | 3.2 | 6.5 |
| 99 | S | double | H | $C(=O)N(CH_3)SC_6H_4Cl$–2 | 112–113 | 49.3 | 3.8 | 8.2 | 49.2 | 4.1 | 8.1 |
| 100 | S | double | H | $C(=O)N(CH_3)SN(CH_2CH_2CH_3)SO_2C_6H_5$ | syrup | 47.8 | 4.9 | 9.8 | 48.0 | 5.3 | 9.6 |
| 103 | S | single | H | H | 100–102 | 50.3 | 6.3 | 9.8 | 50.4 | 6.6 | 9.9 |
| 111 | S | double | H | $C(=O)N(CH_3)SN(CH_2CH_2CH_3)SO_2$–$C_6H_4Cl$–4 | 108–111 | 44.2 | 4.4 | 9.1 | 44.5 | 4.5 | 9.3 |
| 113 | S | double | H | $C(=O)N(CH_3)SN(CH_2CH_2CH_3)SO_2CH_3$ | 91–94 | 39.4 | 5.2 | 11.5 | 39.3 | 5.6 | 11.4 |
| 115 | S | double | H | $C(=O)N(CH_3)SN(CH_3)C(=O)OCH_3$ | 93–95 | 41.6 | 4.8 | 13.2 | 41.7 | 4.9 | 13.2 |
| 121 | S | single | H | $C(=O)N(CH_3)SN$–$(CH(CH_3)_2)SO_2C_6H_4CH_3$–4 | 139–140.5 | 48.7 | 5.7 | 9.5 | 48.4 | 5.7 | 9.8 |
| 124 | S | double | H | $C(=O)N(CH_3)SN(CH(CH_3)_2)SO_2N(CH_3)_2$ | 112–114 | 39.6 | 5.6 | 14.2 | 39.5 | 5.9 | 13.9 |
| 138 | S | double | H | $C(=O)N(CH_3)SN(C_{12}H_{25}n)SO_2N(CH_3)_2$ | 82–84 | 50.7 | 7.8 | 10.8 | 51.0 | 8.0 | 10.4 |
| 140 | S | double | H | $C(=O)N(CH_3)SN(CH_3)C_6H_5$ | 91–93 | 53.7 | 5.1 | 12.5 | 53.6 | 5.0 | 12.1 |
| 150 | S | single | H | $C(=O)N(CH_3)SC_6H_4C(CH_3)_3$–4 | 121.5–125 | 59.3 | 6.6 | 7.7 | 59.5 | 6.6 | 7.7 |
| 151 | S | double | H | $C(=O)N(CH_3)SN(C_8H_{17}n)SO_2C_6H_4CH_3$–4 | oil | 54.0 | 6.5 | 8.2 | 54.2 | 6.7 | 8.1 |

TABLE II

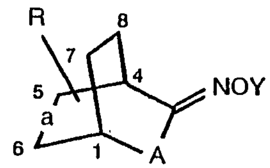

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 24 | S | double | 7(8)–C–(=0)$OCH_3$ | H | oil | 50.7 | 5.2 | 6.6 | 50.1 | 5.5 | 6.2 |
| 25 | S | double | 1(4)C–(=0)$OCH_3$ | H | 178–180 | 50.7 | 5.2 | 6.6 | 50.5 | 5.2 | 6.5 |
| 26 | S | double | 4–CN | H | 196–199 | 53.3 | 4.5 | 15.5 | 53.1 | 4.7 | 15.3 |
| 27 | S | single | H | C(=0)N($CH_3$)S–N($CH_3$)C(=0)$OCH_2CH_3$ | 77–78.5 | 44.9 | 6.1 | 12.1 | 44.8 | 6.3 | 11.8 |
| 28 | S | double | H | C(=0)N($CH_3$)S–N($CH_3$)C(=0)O$(CH_2)_3CH_3$ | 94.5–95 | 48.2 | 6.2 | 11.2 | 48.0 | 6.5 | 11.2 |
| 29 | S | single | H | C(=0)N($CH_3$)S–$C_6H_4$–C$(CH_3)_3$–4 | 140–140.5 | 60.3 | 6.9 | 7.4 | 59.7 | 7.1 | 7.5 |
| 30 | S | single | H | C(=0)N($CH_3$)S–N($CH_3$)C(=0)$OCH_2$–$CH_2OCH_3$ | 104–104.5 | 44.6 | 6.1 | 11.1 | 44.5 | 6.4 | 11.0 |
| 31 | S | double | 1(4)C(=0)–$OCH_2CH_3$ | H | 132–134 | 52.8 | 5.8 | 6.2 | 52.8 | 6.0 | 6.1 |
| 32 | S | single | H | C(=0)N($CH_3$)S–N($CH_3$)C(=0)OCH$(CH_3)_2$ | 93–95 | 46.5 | 6.4 | 11.6 | 46.7 | 6.5 | 11.6 |
| 33 | S | double | H | C(=0)N($CH_3$)S–N($CH_3$)C(=0)Q | 190 | 47.6 | 4.9 | 12.3 | 47.8 | 5.0 | 12.2 |
| 35 | S | single | H | C(=0)N($CH_3$)S–$NCH_2CH_2OCH_2CH_2$ | 95–96 | 46.8 | 6.7 | 12.5 | 47.1 | 6.4 | 12.7 |

TABLE II (Continued)

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 36 | S | double | 5(6)–Br | H | 142–145 | 35.9 | 3.4 | 6.0 | 36.2 | 3.5 | 6.0 |
| 37 | S | double | 5(6)–Br | H | 126–128 | 35.9 | 3.4 | 6.0 | 36.0 | 3.6 | 5.9 |
| 38 | S | single | H | $C(=0)N(CH_3)S-N(CH_3)C(=0)OCH_3$ | 96–99 | 43.2 | 5.7 | 12.6 | 43.1 | 6.0 | 12.4 |
| 39 | S | single | H | $C(=0)N(CH_3)SNC_5H_{10}$ | oil | 51.0 | 7.0 | 12.7 | 51.2 | 7.2 | 12.4 |
| 40 | S | single | H | $C(=0)N(CH_3)S(=0)-C_6H_4C-(CH_3)_3-4$ | 156–158 | 57.8 | 6.6 | 7.1 | 57.8 | 6.9 | 6.9 |
| 41 | S | double | H | $C(=0)N(CH_3)S-C_6H_4C(CH_3)_3-4.H_2O$ | 119–120 | 57.8 | 6.6 | 7.1 | 57.9 | 6.3 | 7.1 |
| 43 | S | double | H | $C(=0)N(CH_3)S-C_6H_4-C(CH_3)_3-4$ | 122–123 | 60.6 | 6.4 | 7.4 | 60.3 | 6.7 | 7.3 |
| 44 | S | double | 5(6)Cl | H | 116–119 | 44.3 | 4.2 | 7.4 | 44.1 | 4.2 | 7.3 |
| 45 | S | double | 5(6)Cl | H | 110–113 | 44.3 | 4.2 | 7.4 | 44.0 | 4.5 | 7.1 |
| 46 | S | single | H | $C(=0)N(CH_3)S-N(CH_3)SO_2N(CH_3)_2$ | oil | 37.7 | 5.8 | 14.6 | 37.1 | 6.1 | 14.8 |
| 48 | S | single | H | $C(=0)N(CH_3)S-C_6H_5$ | 97–98 | 55.9 | 5.6 | 8.7 | 55.8 | 6.0 | 8.6 |
| 49 | S | double | H | $C(=0)N(CH_3)SC_6H_5$ | 112–113 | 56.2 | 5.0 | 8.7 | 56.4 | 4.8 | 8.7 |
| 51 | S | double | 4–CN | $C(=0)N(CH_3)SC_6H_5$ | 139–141 | 55.6 | 4.4 | 12.2 | 55.5 | 4.3 | 12.2 |
| 52 | S | double | H | $C(=0)N(CH_3)S-NCH_2CH_2OCH_2CH_2$ | 112–113 | 47.4 | 5.8 | 12.7 | 47.4 | 6.0 | 12.7 |
| 53 | S | double | 4–CN | $C(=0)N(CH_3)SN(CH_3)C(=0)Q$ | 198–199 | 47.6 | 4.0 | 16.7 | 47.5 | 4.0 | 16.2 |
| 54 | S | double | H | $C(=0)N(CH_3)S-N(CH_3)SO_2N(CH_3)_2$ | 125–126 | 37.9 | 5.3 | 14.7 | 37.7 | 5.5 | 14.7 |

TABLE II (Continued)

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 55 | S | single | H | $C(=O)N(CH_3)S-(2-CH_3-4-(CH_3)_3C)C_6H_3$ | 125–126 | 61.2 | 7.2 | 7.1 | 60.9 | 7.4 | 7.2 |
| 56 | S | double | 4–CN | $C(=O)N(CH_3)S-(2-CH_3-4-(CH_3)_3C)C_6H_3$ | 189–190 | 60.7 | 6.1 | 10.1 | 60.6 | 6.3 | 10.0 |
| 58 | S | single | 4–CN | H | 222–225 | 52.7 | 5.5 | 15.4 | 52.7 | 5.6 | 15.3 |
| 59 | S | double | 4–Cl | H | 185–188 | 44.3 | 4.2 | 7.4 | 44.4 | 4.4 | 7.1 |
| 60 | S | single | H | $C(=O)N(CH_3)S-C_6H_4-CH_3-2$ | 118–119 | 57.1 | 6.0 | 8.3 | 57.3 | 6.0 | 8.4 |
| 61 | S | double | H | $C(=O)N(CH_3)S-C_6H_4-CH_3-2$ | 120–121 | 57.5 | 5.4 | 8.4 | 57.2 | 5.7 | 8.3 |
| 62 | S | single | H | $C(=O)N(CH_3)S-C_6H_4-(OCH_3)-4$ | 119–120 | 54.5 | 5.7 | 7.9 | 54.5 | 6.0 | 8.0 |
| 64 | S | single | H | $C(=O)N(CH_3)SN(CH_3)SO_2C_6H_4CH_3-4$ | 154–156 | 47.5 | 5.4 | 9.8 | 47.2 | 5.6 | 9.5 |
| 65 | S | double | 4–CN | $C(=O)N(CH_3)S-C_6H_4CH_3-3$ | 158–159 | 56.8 | 4.8 | 11.7 | 56.4 | 4.7 | 11.6 |
| 66 | S | double | H | $C(=O)N(CH_3)S-C_6H_4CF_3-3$ | 93–94 | 49.5 | 3.9 | 7.2 | 50.0 | 4.2 | 7.2 |
| 67 | S | double | 4–CN | $C(=O)N(CH_3)S-C_6H_4CF_3-3$ | 148–149 | 49.4 | 3.4 | 10.2 | 49.6 | 3.6 | 10.2 |
| 68 | S | double | H | $C(=O)N(CH_3)SC_6H_4CH_3-3$ | 77–78 | 57.5 | 5.4 | 8.4 | 57.1 | 5.5 | 8.2 |
| 69 | S | double | H | $C(=O)N(CH_3)SC_6H_4(OCH_3)-4$ | 77–78 | 54.4 | 5.4 | 7.9 | 54.8 | 5.2 | 8.0 |
| 70 | S | double | 4–CN | $C(=O)N(CH_3)SN(CH_3)SO_2C_6H_4CH_3-4$ | 180–182 | 47.8 | 4.4 | 12.4 | 47.9 | 4.6 | 12.5 |
| 71 | S | double | H | $C(=O)N(CH_3)SSC(CH_3)_2CN$ | 125.5–127 | 45.5 | 5.0 | 12.2 | 45.5 | 5.2 | 12.1 |
| 72 | S | single | H | $C(=O)N(CH_3)SC_6H_4Cl-4$ | 110–111 | 50.5 | 4.8 | 7.8 | 50.3 | 5.0 | 7.9 |

TABLE II (Continued)

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 73 | S | double | H | $C(=0)N(CH_3)SC_6H_4Cl-4$ | 108–109 | 50.8 | 4.3 | 7.9 | 50.8 | 4.4 | 7.8 |
| 74 | S | double | 4–CN | $C(=0)N(CH_3)SC_6H_4Cl-4$ | 156–158 | 50.6 | 3.7 | 11.1 | 50.4 | 3.8 | 11.1 |
| 75 | S | double | 4–CN | $C(=0)N(CH_3)S-N(CH_2CH_2CH_3)SO_2C_6H_4CH_3-4$ | 142–149 | 50.0 | 5.0 | 11.7 | 49.6 | 5.1 | 11.6 |
| 76 | S | double | H | $C(=0)N(CH_3)S-N(CH_2CH_2CH_3)SO_2C_6H_4CH_3-4$ | 110–115 | 50.1 | 5.5 | 9.2 | 49.6 | 5.7 | 9.2 |
| 77 | S | double | 4–CN | $C(=0)N(CH_3)S-C_6H_4CH_3-4$ | 122–124 | 56.8 | 4.8 | 11.7 | 56.7 | 5.0 | 11.9 |
| 79 | S | single | H | $C(=0)N(CH_3)SC_6H_4CH_3-4$ | 74–75 | 57.1 | 5.8 | 8.3 | 57.0 | 6.3 | 8.3 |
| 80 | S | single | H | $C(=0)N(CH_3)SC_6H_4-NO_2-2$ | 167–168 | 49.0 | 4.7 | 11.4 | 49.1 | 5.0 | 11.3 |
| 81 | S | double | H | $C(=0)N(CH_3)SN(C_4H_9\text{-n})SO_2C_6H_4CH_3-4$ | 107–108.5 | 51.1 | 5.8 | 9.0 | 50.9 | 6.0 | 9.0 |
| 82 | S | double | 4–CN | $C(=0)N(CH_3)SN(C_4H_9\text{-n})SO_2C_6H_4CH_3-4$ | 149.5–150.5 | 51.0 | 5.3 | 11.3 | 50.6 | 5.5 | 11.2 |
| 84 | S | double | H | $C(=0)N(CH_3)SSN(CH_3)C(=0)Q$ | 194–195 | 44.4 | 4.6 | 11.5 | 44.4 | 4.6 | 11.2 |
| 85 | S | single | H | $C(=0)N(CH_3)SSN(CH_3)C(=0)Q$ | 184 | 44.1 | 5.3 | 11.4 | 43.8 | 5.4 | 11.3 |
| 88 | S | single | H | $C(=0)N(CH_3)SC_6H_4Br-4$ | 137–138 | 44.9 | 4.3 | 7.0 | 44.8 | 4.3 | 6.9 |
| 89 | S | double | H | $C(=0)N(CH_3)SC_6H_4Br-4$ | 128–129 | 45.1 | 3.8 | 7.0 | 45.0 | 3.8 | 7.0 |
| 90 | S | single | H | H | 150–152.5 | 53.5 | 7.1 | 8.9 | 53.4 | 7.2 | 9.1 |
| 92 | S | double | 4–CN | $C(=0)N(CH_3)SC_6H_5-Cl-2$ | 166–167 | 50.6 | 3.7 | 11.1 | 50.6 | 3.7 | 11.2 |
| 94 | S | double | H | $C(=0)N(CH_3)SN(CH(CH_3)_2)SO_2C_6H_4CH_3-4$ | 181–181.5 | 50.1 | 5.5 | 9.2 | 50.0 | 5.8 | 9.1 |
| 95 | S | double | 4–CN | $C(=0)N(CH_3)SN(CH(CH_3)_2)SO_2C_6H_4CH_3-4$ | 202–203 | 50.0 | 5.0 | 11.7 | 49.9 | 5.3 | 11.8 |

TABLE II (Continued)

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 96 | S | single | H | $C(=O)N(CH_3)SC_6H_4Cl-2$ | 116–118 | 50.5 | 4.8 | 7.8 | 50.6 | 4.9 | 7.8 |
| 98 | S | double | H | $C(=O)N(CH_3)SN(CH_3)C(=O)OCH_3$ | 120–122 | 43.5 | 5.2 | 12.7 | 43.5 | 5.4 | 12.4 |
| 101 | S | double | 4–CN | $C(=O)N(CH_3)SN(CH_2CH_2CH_3)SO_2C_6H_5$ | 122–123 | 48.9 | 4.7 | 12.0 | 48.6 | 4.9 | 12.2 |
| 102 | S | double | H | $C(=O)N(CH_3)SN(CH_2CH_2CH_3)SO_2C_6H_5$ | 92–96 | 49.0 | 5.2 | 9.5 | 49.3 | 5.5 | 9.6 |
| 104 | S | single | H | $C(=O)N(CH_3)SC_6H_3(NO_2)_2-2,4$ | 206–208 | 43.7 | 3.9 | 13.6 | 43.5 | 3.9 | 13.6 |
| 105 | S | single | H | $C(=O)N(CH_3)SN(C_4H_9\text{-n})SO_2C_6H_4CH_3-4$ | 123.5–126.5 | 50.9 | 6.2 | 8.9 | 50.9 | 6.4 | 9.0 |
| 106 | S | single | H | $C(=O)N(CH_3)SC_6H_4F-4$ | 66–67 | 52.9 | 5.0 | 8.2 | 52.8 | 5.3 | 8.3 |
| 107 | S | single | H | $C(=O)N(CH_3)SN(CH_2CH_2CH_3)SO_2C_6H_5$ | 128–131 | 48.7 | 5.7 | 9.5 | 48.7 | 5.7 | 9.7 |
| 108 | S | double | H | $C(=O)N(CH_3)SN(CH_2CH_2CH_3)SO_2CH_3$ | 115–117.5 | 41.1 | 5.6 | 11.1 | 40.8 | 5.9 | 11.0 |
| 109 | S | double | 4–CN | $C(=O)N(CH_3)SN(CH_2CH_2CH_3)SO_2C_6H_4Cl-4$ | 155–156.5 | 45.5 | 4.2 | 11.2 | 45.5 | 4.2 | 11.2 |
| 110 | S | double | H | $C(=O)N(CH_3)S-N(CH_2CH_2CH_3)SO_2C_6H_4Cl-4$ | 142–143.5 | 45.4 | 4.7 | 8.8 | 45.3 | 4.7 | 8.9 |
| 112 | S | single | H | $C(=O)N(CH_3)SN(CH_2CH_2CH_3)SO_2CH_3$ | 119.5–121.5 | 40.9 | 6.1 | 11.0 | 41.2 | 6.4 | 11.0 |
| 114 | S | double | 4–CN | $C(=O)N(CH_3)SN(CH_2CH_2CH_3)SO_2CH_3$ | 155–156.5 | 41.6 | 5.0 | 13.8 | 41.4 | 5.3 | 13.8 |
| 116 | S | double | H | $C(=O)N(CH_3)SN(CH_2CH_2CH_3)C(=O)OCH_3$ | 79–80 | 46.8 | 5.9 | 11.7 | 46.2 | 6.1 | 11.6 |
| 118 | S | double | 4–CN | $C(=O)N(CH_3)SC_6H_4F-4$ | 112–115 | 52.9 | 3.9 | 11.6 | 53.1 | 4.0 | 11.3 |
| 119 | S | single | H | $C(=O)N(CH_3)SN(CH(CH_3)_2)SO_2N(CH_3)_2$ | 90–92 | 40.9 | 6.4 | 13.6 | 41.0 | 6.7 | 13.5 |

TABLE II (Continued)

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 120 | S | single | H | $C(=0)N(CH_3)SC_6H_4CN-2$ | 137–138 | 55.3 | 4.9 | 12.1 | 55.5 | 5.1 | 12.2 |
| 122 | S | double | H | $C(=0)N(CH_3)S-N(C_6H_5)C(=0)OCH_3$ | 120–121 | 51.9 | 4.9 | 10.7 | 51.6 | 5.1 | 10.5 |
| 123 | S | single | H | $C(=0)N(CH_3)SN(CH_3)C(=0)ON=(-2-CH_3-2,5-(-C(CH_3)_2-)C_6H_7)$ | 160–161.5 | 53.8 | 6.9 | 12.0 | 53.6 | 7.4 | 11.7 |
| 125 | S | double | H | $C(=0)N(CH_3)S-N(CH(CH_3)_2SO_2N(CH_3)_2$ | 126–128 | 41.1 | 5.9 | 13.7 | 41.3 | 6.2 | 13.8 |
| 126 | S | double | 4–CN | $C(=0)N(CH_3)SN(CH_3)C(=0)OCH_3$ | 130–131 | 43.8 | 4.5 | 15.7 | 43.8 | 5.1 | 15.4 |
| 127 | S | double | 4–CN | $C(=0)N(CH_3)S-C_6H_4(OCH_3)-2$ | 163–164 | 54.4 | 4.6 | 11.2 | 54.2 | 4.5 | 11.3 |
| 128 | S | single | H | $C(=0)N(CH_3)SN(CH_3)C(=0)OCH(CN)-C_6H_4(OC_6H_5)-3$ | foam | 57.0 | 5.0 | 10.6 | 57.0 | 5.0 | 10.3 |
| 130 | S | single | H | $C(=0)N(CH_3)SSCH_2CH_2CH_2CH_3$ | semi solid | 46.7 | 6.6 | 8.4 | 46.4 | 6.7 | 8.2 |
| 131 | S | single | H | $C(=0)N(CH_3)SN(CH_3)C(=0)OCH_2C_6H_4-(OC_6H_5)-3$ | glass | 56.6 | 5.4 | 8.2 | 56.0 | 5.5 | 8.2 |
| 132 | S | single | H | $C(=0)N(CH_3)SC_6H_4(OCH_3)-2$ | 134–135 | 54.5 | 5.7 | 7.9 | 54.2 | 6.0 | 8.0 |
| 133 | S | single | H | $C(=0)N(CH_3)SNCH_2CH_2SCH_2CH_2$ | 110–111 | 44.9 | 6.1 | 12.1 | 44.9 | 6.4 | 12.2 |
| 134 | S | single | H | $C(=0)N(CH_3)SN(CH_3)C_6H_5$ | 115–116 | 54.6 | 6.0 | 12.0 | 54.8 | 6.1 | 11.9 |
| 135 | S | single | H | $C(=0)N(CH_3)SN(CH_3)C(=0)NHC_6H_5$ | 67.5 | 51.2 | 5.6 | 14.0 | 51.1 | 5.7 | 14.0 |

TABLE II (Continued)

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 136 | O | double | H | $C(=0)N(CH_3)SC_6H_4C(CH_3)_3-4$ | 129–130 | 63.3 | 6.7 | 7.8 | 63.1 | 6.7 | 8.1 |
| 137 | S | single | H | $C(=0)N(CH_3)SN(C_{12}H_{25}n)SO_2N(CH_3)_2$ | 92–93 | 51.4 | 8.3 | 10.4 | 51.2 | 8.4 | 10.6 |
| 139 | S | double | H | $C(=0)N(CH_3)SN(C_{12}H_{25}n)SO_2N(CH_3)_2$ | 76–78 | 51.6 | 7.9 | 10.5 | 51.7 | 8.1 | 10.3 |
| 141 | S | double | 4–CN | $C(=0)N(CH_3)SN(C_{12}H_{25}n)SO_2N(CH_3)_2$ | 116–117 | 51.5 | 7.4 | 12.5 | 51.2 | 7.6 | 12.5 |
| 142 | S | double | 4–CN | $C(=0)N(CH_3)SN(CH_3)C_6H_5$ | 133–134 | 54.5 | 4.8 | 15.0 | 54.7 | 4.7 | 15.0 |
| 143 | S | double | 4–CN | $C(=0)N(CH_3)SN(CH_3)C(=0)ON=C(CH_2-SCH_3)C(CH_3)_3$ | 57–64 | 48.3 | 5.5 | 14.1 | 47.6 | 5.8 | 14.6 |
| 144 | S | double | H | $C(=0)N(CH_3)SN(C_8H_{17}n)SO_2C_6H_4CH_3-4$ | oil | 54.8 | 6.7 | 8.0 | 54.3 | 6.8 | 7.5 |
| 145 | S | double | 4–CN | $C(=0)N(CH_3)SSCH_2CH_2CH_2CH_3$ | 101–102.5 | 47.0 | 5.4 | 11.8 | 46.9 | 5.3 | 12.1 |
| 146 | O | single | H | H | 165–166 | 59.6 | 7.8 | 9.9 | 59.2 | 7.8 | 10.0 |
| 147 | O | double | H | H | 153–154 | 60.4 | 6.5 | 10.1 | 60.8 | 6.5 | 9.9 |
| 149 | S | double | H | $C(=0)N(CH_3)SSCH_2CH_2CH_2CH_3$ | 62–64 | 47.0 | 6.1 | 8.4 | 47.3 | 6.1 | 8.7 |
| 152 | S | double | 4–CN | $C(=0)N(CH_3)SN(CH(CH_3)_2)SO_2C_6H_5$ | 177–179.5 | 48.9 | 4.8 | 12.0 | 49.2 | 4.8 | 12.0 |
| 153 | S | double | 4–CN | $C(=0)N(CH_3)SN(C_8H_{17}\text{-}n)SO_2C_6H_4CH_3-4$ | 133.5–135 | 54.5 | 6.2 | 10.2 | 54.5 | 6.3 | 10.0 |
| 155 | S | double | 4–CN | $C(=0)N(CH_3)SN(CH_3)C(=0)O(CH_2)_{11}CH_3$ | 93–95 | 56.4 | 7.5 | 11.0 | 56.7 | 7.7 | 11.0 |
| 156 | O | double | H | $C(=0)N(CH_3)S-SN(CH_3)C(=0)OQ$ | 195 | 47.6 | 4.9 | 12.3 | 47.0 | 5.0 | 12.2 |

TABLE II (Continued)

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 157 | S | double | 4–CN | $C(=0)N(CH_3)SC_6H_4CH_3-2$ | 171–172 | 56.8 | 4.8 | 11.7 | 56.6 | 4.7 | 11.7 |
| 158 | S | single | H | $C(=0)N(CH_3)SSC_6H_5$ | oil | 50.8 | 5.1 | 7.9 | 51.2 | 5.2 | 7.4 |
| 159 | S | double | 4–CN | $C(=0)N(CH_3)SN(C_4H_9\text{-n})_2$ | oil | 54.5 | 7.1 | 14.1 | 54.6 | 7.3 | 13.7 |
| 160 | S | double | 4–CN | $C(=0)N(CH_3)SC(CH_3)_2C(=0)CH_3$ | 128–129.5 | 51.0 | 5.4 | 11.9 | 50.7 | 5.4 | 12.0 |
| 161 | S | double | 4–CN | $C(=0)N(CH_3)SN(C_{12}H_{25}\text{-n})C(=0)N(CH_3)_2$ | 61.5–65 | 57.3 | 7.9 | 13.4 | 57.2 | 7.8 | 13.3 |
| 162 | S | double | 4–CN | $C(=0)N(CH_3)SN(CH_2C(=0)OCH_2{\cdot}CH_3)-SO_2C_6H_4CH_3-4$ | 139.5–141.5 | 48.1 | 4.6 | 10.7 | 48.1 | 4.6 | 10.7 |
| 163 | S | double | 4–CN | $C(=0)N(CH_3)SN(CH_2CH_2CH_3)SO_2C_6H_4-(OCH_3)-4$ | 122–125 | 48.3 | 4.9 | 11.3 | 48.3 | 4.8 | 11.2 |
| 164 | S | double | 4–Cl | $C(=0)N(CH_3)SC_6H_4C(CH_3)_3-4$ | 137–139.5 | 55.5 | 5.6 | 6.8 | 55.0 | 5.8 | 6.8 |
| 165 | S | double | 4–CN | $C(=0)N(CH_3)SN(CH_3)SO_2N(C_4H_9\text{-n})_2$ | 103–105 | 46.6 | 6.4 | 14.3 | 46.3 | 6.7 | 14.3 |
| 166 | S | double | 4–CN | $C(=0)N(CH_3)SNCH_2CH_3SCH_3CH_2$ | 138–139 | 45.4 | 4.9 | 15.1 | 45.5 | 5.1 | 15.2 |
| 167 | S | double | 4–CN | $C(=0)N(CH_3)SC(=0)OC_{12}H_{25}\text{-n}$ | 74–76 | 57.4 | 7.3 | 8.7 | 57.1 | 7.6 | 8.8 |
| 168 | S | double | 4–CN | $C(=0)N(CH_3)SN(CH_2CH_2CH_3)P(S)-(OCH_2CH_3)_2$ | 89.5–91 | 42.7 | 5.7 | 11.7 | 42.6 | 6.1 | 11.9 |
| 169 | S | double | 4–CN | $C(=0)N(CH_3)SNCH_2CH_2SO_2CH_2CH_2$ | 202–204 | 41.8 | 4.5 | 13.9 | 41.8 | 4.5 | 13.9 |
| 170 | S | double | 4–CN | $C(=0)N(CH_3)SN(CH_3)C(=0)O-CH_2(C_4H_3O)-2$ | 136.5–138 | 48.3 | 4.3 | 13.3 | 48.2 | 4.3 | 13.2 |

TABLE II (Continued)

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 171 | S | double | 4-Cl | $C(=O)N(CH_3)SN(C_{12}H_{25}\text{-}n)C(=O)N(CH_3)_2$ | 77–78.5 | 54.1 | 7.8 | 10.5 | 53.9 | 7.8 | 10.5 |
| 172 | S | double | 4-CN | $C(=O)N(CH_3)S\text{–}N(C_6H_5)SO_2N(CH_3)_2$ | 178.5–180 | 46.2 | 4.5 | 15.0 | 46.4 | 4.5 | 15.0 |
| 173 | S | double | 4-CN | $C(=O)N(CH_3)SSC_6H_5$ | oil | 50.9 | 4.0 | 11.1 | 50.7 | 4.2 | 10.9 |
| 174 | S | double | 4-CN | $C(=O)N(CH_3)SSC_6H_4C(CH_3)_3\text{–}4$ | 118–120 | 55.4 | 5.4 | 9.7 | 55.4 | 5.3 | 9.6 |
| 175 | S | double | 4-CN | $C(=O)N(CH_3)SC_6H_4(OCH_3)\text{–}4$ | 122–123 | 54.4 | 4.6 | 11.2 | 54.1 | 4.6 | 11.2 |
| 176 | S | double | 4-CN | $C(=O)N(CH_3)SN(CH_3)C_8H_{17}\text{-}n$ | 89–91 | 55.6 | 7.4 | 13.6 | 55.7 | 7.3 | 13.6 |
| 177 | S | double | 4-CN | $C(=O)N(CH_3)SN(CH_2CH_2CH_3)SO_2CH_2C_6H_5$ | 154–156 | 50.0 | 5.0 | 11.7 | 50.1 | 5.0 | 11.7 |
| 178 | S | double | 4-CN | $C(=O)N(CH_3)SN(CH_3)C(=O)NHCH_3$ | 136–137 | 43.9 | 4.8 | 19.7 | 44.0 | 4.9 | 19.7 |
| 179 | S | double | 4-CN | $C(=O)N(CH_3)SN(CH_2CH_2CH_3)\text{–}$ $P(S)OCH_2C(CH_3)_2CH_2O$ (ring) | 129–130 | 44.1 | 5.6 | 11.4 | 43.9 | 5.7 | 11.5 |
| 180 | S | double | 4-CN | $C(=O)N(CH_3)SN(CH_3)C(=O)OC_6H_4C_9H_{19}\text{–}4$ | 69–75 | 59.5 | 6.7 | 10.3 | 59.5 | 6.8 | 10.2 |
| 181 | S | double | 4-CN | $C(=O)N(CH_3)SN(CH_3)C(=O)OCH_2CH_2OCH_3$ | 110–112 | 45.0 | 5.0 | 14.0 | 45.2 | 5.0 | 14.0 |
| 182 | S | double | 4-CN | $C(=O)N(CH_3)S\text{–}C_6H_{11}Cl\text{–}2$ | 52 | 49.8 | 5.2 | 10.9 | 49.8 | 5.4 | 10.7 |
| 183 | S | double | 4-CN | $C(=O)N(CH_3)SN(C_{12}H_{25}\text{-}n)SO_2NC_4H_8$ | 121–123 | 53.3 | 7.4 | 12.0 | 53.1 | 7.2 | 11.8 |
| 184 | S | double | 4-CN | $C(=O)N(CH_3)SSC_6H_4\text{–}Cl\text{–}2$ | 105–107 | 46.6 | 3.4 | 10.2 | 47.6 | 3.6 | 10.0 |

TABLE II (Continued)

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 185 | S | double | 4–CN | $C(=O)N(CH_3)SN(CH_3)C(=O)N(C_4H_9\text{-}n)_2$ | 85–86 | 53.0 | 6.9 | 15.4 | 52.8 | 7.0 | 15.5 |
| 186 | S | double | 4–CN | $C(=O)N(CH_3)SC_{12}H_{25}$ | oil | 60.4 | 8.1 | 9.6 | 60.1 | 8.3 | 8.9 |
| 187 | S | double | 4–CN | $C(=O)N(CH_3)SNCH_2CH_2OCH_2CH_2$ | 59–63 | 47.4 | 5.1 | 15.8 | 47.1 | 4.9 | 15.5 |
| 188 | S | double | H | $C(=O)N(CH_3)SSC(CH_3)_3$ | 99.5–103 | 47.0 | 6.1 | 8.4 | 46.9 | 6.4 | 8.5 |
| 189 | S | double | 4–CN | $C(=O)N(CH_3)SN(CH_2CH_2CH_3)SO_2(C_5H_4N)\text{–}3$ | 87–90 | 46.2 | 4.5 | 15.0 | 46.4 | 4.6 | 14.6 |
| 190 | S | double | H | $C(=O)N(CH_3)SN(CH_2CH_2CH_3)SO_2(C_5H_4N)\text{–}3$ | 94–98 | 46.1 | 5.0 | 12.7 | 45.9 | 5.1 | 12.6 |
| 191 | S | double | H | $C(=O)N(CH_3)SSC_{12}H_{25}\text{-}n$ | 36–38 | 56.7 | 8.2 | 6.3 | 56.8 | 8.4 | 6.6 |
| 192 | S | double | H | $C(=O)N(CH_3)SSC_6H_4C(CH_3)\text{–}4$ | 83–85 | 55.8 | 5.9 | 6.9 | 56.1 | 6.0 | 7.1 |
| 197 | S | double | 1–$CH_3$–<br>4–CN | H | 200.5–201.5 | 56.2 | 6.2 | 14.4 | 55.8 | 5.3 | 14.4 |

TABLE III

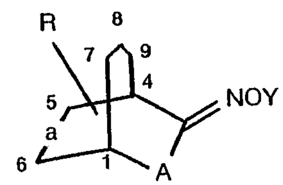

| Ex. No. | A | a | R | Y | Melting Range °C | CALCULATED C | CALCULATED H | CALCULATED N | FOUND C | FOUND H | FOUND N |
|---|---|---|---|---|---|---|---|---|---|---|---|
| 148 | O | double | H | H | 126–127 | 62.7 | 7.2 | 9.1 | 62.7 | 7.4 | 8.9 |

TABLE IV

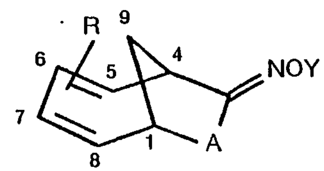

| Ex. No. | A | R | Y | Melting Range °C | CALCULATED C | CALCULATED H | CALCULATED N | FOUND C | FOUND H | FOUND N |
|---|---|---|---|---|---|---|---|---|---|---|
| 154 | O | H | H | 141–151 | 63.6 | 6.0 | 9.3 | 63.5 | 6.1 | 9.4 |

TABLE V

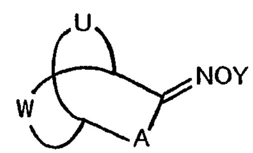

| Ex. No. | A | u | W | Y | Melting Range °C | CALCULATED C | CALCULATED H | CALCULATED N | FOUND C | FOUND H | FOUND N |
|---|---|---|---|---|---|---|---|---|---|---|---|
| 117 | S | 1,2-$C_6H_4$ | 1,2-$C_6H_4$ | H | 216–218 | 71.1 | 4.4 | 5.5 | 70.8 | 4.1 | 5.6 |

*Biological Activity*

The compounds were evaluated for biological activity against the following representative pests: Southern corn rootworm (*Diabrotica undecimpunctata howardi*), Mexican bean beetle (*Epilachna varivestis*), Southern armyworm (*Spodoptera eridania*), housefly (*Musca domestica)*, bean aphid (*Aphis fabae*) and red spider mite (*Tetranychus urticae*). A combination of contact, stomach poison and systemic tests are run on these insects.

The formulation used for insecticide testing contains a final concentration of 0.0128 percent or 128 p.p.m. test chemical, 4 percent acetone and 0.01 percent (100 ppm) Triton X-155 surfactant ("TRITON" is a Regd. Trademark). Accompanying reference insecticides, if any, are formulated by these procedures as are the checks which contain a standard amount of solvent and a surfactant, but no test chemical. The foregoing stock formulations are utilized with appropriate dilution to obtain the desired pound/acre application rates.

The test procedures employed are as follows.

*Bean Aphid Spray and Systemic Test*

This test determines the insecticidal activity of the compound being tested against the bean aphid, *Aphis fabae*. Stock formulations containing 128 p.p.m. of each test chemical are prepared using 12.8 mg of the test chemical, 4.0 ml acetone containing 0.25 percent (V/V) Triton X-155 and 96.0 ml deionized water and are used in both soil drench and spray treatments. The stock formulations are diluted to obtain the appropriate lower concentrations maintaining the concentration level of all adjuvants. The bean aphid is cultured on nasturtium plants (var. Tall Single), no attempt being made to select insects of a given age in these tests. Single nasturtium test plants growing in soil in individual 75 mm (3-inch) fibre pots are then infested with populations of 100 to 200 aphids.

In the spray application, 50 ml of stock or diluted formulation is uniformly sprayed onto the plants. In the systemic application, 11.2 ml of stock or diluted formulation is applied to the soil containing the plant. A dosage of 11.2 ml of formulation containing 128 p.p.m. of test chemical is equivalent to a dosage of the test chemical of 4.48 kg/ha (4 pounds per acre (lb/A)).

The plant test units under fluorescent lights are given bottom watering for the duration of the test. Percentage mortality is determined three days after treatment. Results of this test, as expressed in $LC_{50}$ values, are shown in Table VI as A (aphid contact spray) and AS (aphid systemic soil drench).

*Red Spider Mite Spray and Systemic Test*

This test determines the acaricidal activity of the compound being tested against the red spider mite, *Tetranychus urticae*. Stock formulations containing 128 p.p.m. of each test chemical are prepared by the procedure above and are used in both the soil drench and spray treatments. The stock culture of mites is maintained on Scarlet runner bean foliage. Approximately 18 to 24 hours before testing, mites are transferred to the primary leaves of a Lima bean plant (var. Sieva) grown in 75 mm (3-inch) pots.

The spray and systemic application methods described before are used to apply the test formulations to the infested plants and soil. After three days, one of the two leaves treated is examined and mortality is determined. Results of this test, as expressed in $LC_{50}$ values, are shown in Table VI as M (mite contact spray test) and MS (mite systemic soil drench test).

*Housefly Spray Test*

This test determines the insecticidal activity of the compound being tested against adult houseflies, *Musca domestica*. Stock formulations containing 128 p.p.m. of each test chemical are prepared using the procedure described previously and are diluted to obtain the appropriate lower concentrations.

Ten adult flies are placed in a cylindrical screen cage 37 × 100 mm ($1\frac{1}{2}$ by 4 inches) fabricated from 20-mesh stainless steel screening and are sprayed with 50 ml of the stock or diluted formulation. The flies are supplied food and drink from a sucrose solution by draping a paper wick over the outside of the screen cylinder and are able to feed and drink ad libitum. Percent mortality obtained is determined three days after treatment. Results of this test, as expressed in $LC_{50}$ values, are shown in Table VI as HF (housefly spray test).

*Southern Armyworm Leaf Spray Test*

Paired fully expanded primary leaves excised from Scarlet runner bean plants are maintained in plastic tubes containing water and sprayed with the test formulation prepared as described previously. After the spray deposit on the leaves is dry, the paired leaves are separated. One leaf is placed onto 1.5 percent water agar and infested with 10 newly hatched Southern armyworm larvae. The covered test receptacle is held at 22.2°C (72°F) for four days and then the percent mortality is determined. Results of this test, as expressed in $LC_{50}$ values, are shown in Table VI as AW (Southern armyworm spray test).

*Mexican Bean Beetle Leaf Spring Test*

This test determines the insecticidal activity of the compound being tested against the Mexican bean beetle, *Epilachna varivestis*. The test procedure is the same as that described for the Southern armyworm

with the exception that one-day old larvae of the Mexican bean beetle instead of newly hatched Southern armyworm larvae are used.

These tests are held at 22.2°C (72°F) for four days when mortality and feeding inhibition are determined. The feeding inhibition is an indication of the repellent properties of the test material. Results of this test, as expressed in $LC_{50}$ values, are shown in Table VI as BB (Mexican bean beetle leaf spray test).

TABLE VI

| | $^{LC}50$ in ppm[1] | | | | | $^{LD}50$ in Kg/hectare | |
|---|---|---|---|---|---|---|---|
| EXAMPLE NO. | BB[(2)] | AW[(3)] | HF[(4)] | M[(5)] | A[(6)] | MS[(7)] | AS[(8)] |
| 12 | 2.5 | 33 | >128 | >128 | 3.9 | >16 | >16 |
| 13 | 3.0 | 13.5 | 29 | 5.9 | 2.0 | >16 | >16 |
| 14 | 4.7 | 28 | 71 | 6.6 | 3.3 | >16 | >16 |
| 15 | 7.8 | 11 | 50 | >128 | >128 | >16 | 2.9 |
| 16 | 12 | 23 | 32 | 80 | 28 | >16 | >16 |
| 18 | 22 | 11 | 11 | 54 | 2.4 | >16 | >16 |
| 19 | 1.5 | 17 | 105 | 18 | 71 | >16 | >16 |
| 21 | 3.2 | 17 | 5.5 | 100 | 70 | >16 | >16 |
| 27 | 4.1 | 19 | 15 | 2.9 | 0.78 | >16 | >16 |
| 28 | 5.0 | 18 | 45 | 13 | 2.6 | >16 | >16 |
| 30 | 11 | 19 | 36 | 8.0 | 3.2 | >16 | >16 |
| 32 | 11 | 22 | 40 | 6.9 | 1.5 | >16 | >16 |
| 35 | 5.2 | 34 | 45 | 12 | 3.5 | >16 | >16 |
| 38 | 7.2 | 22 | 18 | 6.6 | 1.9 | >16 | >16 |
| 39 | 6.9 | 18 | 32 | 10 | 2.1 | >16 | >16 |
| 41 | 5.0 | 16 | 14 | 7.0 | 10 | >16 | >16 |
| 42 | 5.8 | 11 | 14 | 59 | 34 | >16 | >16 |
| 43 | 1.6 | 16 | 21 | 35 | 19 | >16 | >16 |
| 46 | 1.8 | 21 | 30 | 15 | 7.5 | >16 | >16 |
| 47 | 3.1 | 20 | 17 | 34 | 55 | >16 | >16 |
| 49 | 6.5 | 18 | 25 | 11 | 5.2 | >16 | <16 |
| 51 | 0.39 | 8.2 | 35 | 2.1 | 4.2 | >16 | 13 |
| 52 | 0.83 | 30 | 17 | 5.5 | 3.9 | >6 | >16 |
| 53 | 0.98 | 6.9 | 97 | 3.6 | 55 | >16 | 13 |
| 54 | 3.7 | 13 | 8.8 | 34 | 30 | >16 | >16 |
| 55 | 5.2 | 25 | 46 | 15 | 15 | >16 | >16 |

TABLE VI (Cont'd)

| EXAMPLE NO. | LC50 in ppm[1] BB[(2)] | AW[(3)] | HF[(4)] | M[(5)] | A[(6)] | LD50 in Kg/hectare MS[(7)] | AS[(8)] |
|---|---|---|---|---|---|---|---|
| 56 | 2.2 | 12 | 23 | >128 | 105 | >16 | >16 |
| 60 | 4.7 | 13 | 8.3 | 4.9 | 1.1 | >16 | >16 |
| 61 | 7.4 | 14 | 3.1 | 4.0 | 2.3 | >16 | >16 |
| 62 | 7.7 | 14 | 8.6 | 2.8 | 3.2 | >16 | >16 |
| 63 | 3.3 | 16 | 3.0 | >128 | 22 | >16 | >16 |
| 66 | 5.5 | 45 | 34 | 4.1 | 5.5 | >16 | >16 |
| 67 | 1.7 | 30 | >128 | 4.0 | 9.4 | >16 | >16 |
| 69 | 4.8 | 30 | 27 | 12 | 7.2 | >16 | >16 |
| 70 | 2.3 | 24 | >128 | >128 | >128 | >16 | >16 |
| 71 | 6.2 | 32 | 60 | 14 | 6.9 | >16 | >16 |
| 73 | 7.6 | 25 | 19 | 15 | 7.9 | >16 | >16 |
| 74 | 4.5 | 18 | >128 | 8.0 | >128 | >16 | >16 |
| 75 | 1.9 | 23 | 114 | 4.0 | 87 | >16 | >16 |
| 77 | 2.6 | 11 | >128 | 2.1 | 16 | >16 | >16 |
| 79 | 4.1 | 9.3 | 99 | 4.7 | 2.2 | >16 | >16 |
| 80 | 6.5 | 19 | 131 | 23 | 8.9 | >16 | >16 |
| 81 | 5.3 | 27 | 32 | 18 | 26 | >16 | >16 |
| 82 | 2.6 | 31 | >128 | 4.6 | >128 | >16 | >16 |
| 86 | 8.4 | 24 | 21 | 25 | 22 | >16 | >16 |
| 88 | 4.6 | 19 | 108 | >128 | 11 | >16 | >16 |
| 89 | 2.4 | 14 | 63 | 1.5 | 21 | >16 | >16 |
| 92 | 1.1 | 13 | 88 | 3.6 | 4.2 | 9 | >16 |
| 96 | 5.2 | 22 | 31 | 19 | 3.0 | >16 | >16 |
| 98 | 1.4 | 18 | 15 | 14 | 2.5 | >16 | 13 |
| 99 | 3.3 | 15 | 16 | >128 | 25 | >16 | >16 |
| 100 | 5.3 | 23 | 21 | 18 | 45 | >16 | >16 |
| 101 | 1.2 | 20 | >128 | 2.0 | 139 | <16 | >16 |
| 102 | 2.4 | 24 | 66 | 5.2 | 11 | >16 | >16 |
| 106 | 5.4 | 32 | 47 | 6.0 | 1.6 | >16 | >16 |

TABLE VI (Cont'd)

| EXAMPLE NO. | LC50 in ppm[1] BB[(2)] | AW[(3)] | HF[(4)] | M[(5)] | A[(6)] | LD50 in Kg/hectare MS[(7)] | AS[(8)] |
|---|---|---|---|---|---|---|---|
| 107 | 5.0 | 16 | 104 | 15 | 12 | >16 | >16 |
| 108 | 7.1 | 17 | 33 | 29 | 11 | >16 | >16 |
| 110 | 7.0 | 25 | 15 | 40 | 49 | >16 | >16 |
| 112 | 3.9 | 22 | 53 | 8.6 | 7.0 | >16 | >16 |
| 114 | 2.1 | 11 | 26 | 4.2 | 59 | >16 | >16 |
| 115 | 16 | 22 | 7.3 | 17 | 8.4 | >16 | 14 |
| 116 | 1.8 | 18 | 23 | 5.7 | 6.1 | >16 | >16 |
| 118 | 1.6 | 17 | 13 | 4.0 | 5.5 | 9 | >16 |
| 119 | 4.2 | 26 | 85 | 13 | 8.9 | >16 | >16 |
| 120 | 14 | 55 | >128 | 16 | 1.4 | >16 | >16 |
| 121 | 14 | 84 | 26 | 7.1 | 4.5 | >16 | <16 |
| 122 | 4.1 | 17 | 48 | 14 | 16 | >16 | >16 |
| 123 | 6.0 | 41 | >128 | 65 | 26 | >16 | >16 |
| 125 | 8.0 | 32 | 26 | 18 | 10 | >16 | >16 |
| 127 | 2.6 | 12 | 125 | 5.7 | 16 | <16 | <16 |
| 132 | 4.8 | 16 | 87 | 29 | 23 | >16 | >16 |
| 133 | 3.7 | 18 | 4.2 | 6.2 | 1.3 | >16 | >16 |
| 160 | 1.9 | 9.0 | 30 | 3.7 | 73 | 16 | 5.5 |
| 162 | 1.7 | 18 | 33 | 36 | 30 | >16 | <16 |
| 166 | 1.2 | 12 | 49 | 7.6 | 12 | >16 | >16 |
| 167 | 2.0 | 17 | >128 | 6.4 | 11 | >16 | >16 |
| 168 | 2.0 | 45 | >128 | 6.1 | 71 | >16 | >16 |
| 178 | 2.7 | 29 | 61 | 3.2 | 15 | >16 | >16 |
| 179 | 1.8 | 23 | 61 | 11 | >128 | >16 | >16 |
| 182 | 2.3 | 31 | 42 | 5.9 | 43 | >16 | >16 |
| 186 | 2.0 | 7.6 | 103 | 2.8 | 15 | >16 | >16 |
| 190 | 3.2 | 28 | 37 | 31 | 42 | >16 | >16 |

TABLE VI

| EXAMPLE NO. | LC50 in ppm[1] | | | | | LD50 in Kg/hectare | |
|---|---|---|---|---|---|---|---|
| | BB[(2)] | AW[(3)] | HF[(4)] | M[(5)] | A[(6)] | MS[(7)] | AS[(8)] |

1. $LC_{50}$ = Concentration lethal to 50% of test species
2. BB = Mexican bean beetle
3. AW = Southern army worm
4. HF = housefly
5. M = mite contact
6. A = aphid contact
7. MS = mite systemic
8. AS = aphid systemic

*Root-Knot Nematode Test*

This test is an evaluation of the effectiveness of the compound being tested against infection by root-knot nematodes, *Meloidogyne spp.*

Pasteurized greenhouse soil, diluted by two-thirds with clean washed sand, is infested with about 15 g of nematode infested tomato roots and soil. The test formulation contains 0.056 g of test compound, 4.0 ml stock emulsifier solution (0.25 percent Triton X-155 in acetone by volume) and 96.0 ml deionized water, giving a concentration of 560 p.p.m. Lower concentrations are achieved by dilution.

Treatment is accomplished by adding 50 ml of the formulated compound into a plastic bag which contains enough infested soil to fill two 4-inch round pots. This is thoroughly mixed, then returned to the pots, after which 5 cucumber seeds (Ohio MR17 var.) are planted per pot.

Two standards are included with each test, Phenamiphos and Aldicarb are most often used, with Carbofuran and Ethoprop used as substitutes. Standard formulations are treated at 140 p.p.m. with lower concentrations achieved by dilution.

Roots are removed from the soil after three weeks of growth and rated for gall (root-knot nematode infection) formation. A rating of infection from 0 to 10 is recorded: 0 = no galls or complete control and 10 = heavily galled roots comparable to controls. Each of the root systems is rated separately. The average of each treatment is subtracted from the average of the control check, that sum is then divided by the average of the control check and multiplied by 100 to obtain a percent nematode control.

Percent control equals:

$$100 \times \frac{\text{average of control check} - \text{average of treatment}}{\text{average of control check}}$$

The results obtained with selected compounds of the invention are shown in Table VII.

TABLE VII

| Compound Example No. | Percent Control At Dosage Rate Indicated | | | | | | |
|---|---|---|---|---|---|---|---|
| Kg/ha<br>lb/A | 8.96<br>8 | 4.48<br>4 | 2.24<br>2 | 1.12<br>1 | 0.56<br>0.5 | 0.28<br>0.25 | 0.14<br>0.125 |
| PHENAMIPHOS[1] | —[3] | 76 | 57 | 30 | 14 | — | — |
| ALDICARB[2] | — | 78 | 66 | 49 | 14 | — | — |
| 27 | 100 | 100 | 100 | 87 | 67 | 22 | — |
| 28 | 100 | 100 | 100 | 74 | 57 | 5 | — |
| 30 | 100 | — | 68 | 59 | 31 | 0 | — |
| 34 | — | 100 | — | 85 | 30 | 13 | — |
| 40 | — | 100 | — | 90 | — | — | — |
| 41 | — | 99 | — | 72 | — | — | — |
| 42 | — | 85 | — | 48 | — | — | — |
| 43 | — | 98 | — | 74 | — | — | — |
| 46 | — | 66 | — | 25 | — | — | — |
| 48 | — | 100 | — | 78 | — | 20 | — |
| 49 | — | 100 | — | 95 | — | 42 | — |
| 51 | — | 100 | 100 | 95 | 73 | 80 | 69 |
| 52 | — | 100 | — | 90 | — | 51 | — |
| 53 | — | 100 | 83 | 98 | 52 | 64 | 48 |
| 54 | — | 100 | — | 91 | — | — | — |
| 56 | — | 100 | 93 | 93 | 73 | 64 | 49 |
| 61 | — | 98 | — | 82 | — | 42 | — |
| 62 | — | 97 | — | 78 | — | 19 | — |
| 67 | — | 94 | — | 83 | — | 66 | — |
| 74 | — | 100 | — | 90 | — | 81 | — |
| 75 | — | 100 | — | 84 | — | 57 | — |
| 77 | — | 100 | — | 94 | — | 62 | — |
| 87 | — | 55 | — | 50 | — | 29 | — |
| 92 | — | 95 | — | 85 | — | 71 | — |
| 95 | — | 97 | — | 94 | — | 73 | — |
| 96 | — | 74 | — | 54 | — | 14 | — |
| 101 | — | 100 | — | 100 | — | 96 | — |

TABLE VII (Cont'd)

| Compound Example No. | Percent Control At Dosage Rate Indicated | | | | | | |
|---|---|---|---|---|---|---|---|
| Kg/ha | 8.96 | 4.48 | 2.24 | 1.12 | 0.56 | 0.28 | 0.14 |
| lb/A | 8 | 4 | 2 | 1 | 0.5 | 0.25 | 0.125 |
| 102 | — | 100 | — | 78 | — | 5 | — |
| 109 | — | 100 | — | 91 | — | 47 | — |
| 114 | — | 97 | — | 92 | — | 43 | — |
| 118 | — | 99 | — | 89 | — | 63 | — |
| 150 | — | 100 | — | 75 | — | 39 | — |
| 153 | 100 | 100 | 94 | 84 | — | 0 | — |
| 156 | 100 | 100 | 97 | 67 | — | 0 | — |
| 163 | — | 95 | — | 91 | — | 50 | — |
| 164 | — | 100 | — | 99 | — | 22 | — |
| 166 | — | 100 | — | 100 | — | 100 | 95 |
| 178 | — | 100 | — | 99 | — | 96 | 39 |
| 179 | — | 100 | — | 99 | — | 30 | — |
| 181 | — | 100 | — | 100 | — | 69 | — |
| 182 | — | 100 | — | 100 | — | 73 | — |
| 185 | — | 98 | — | 98 | — | 22 | — |
| 186 | — | 94 | — | 64 | — | 34 | — |
| 187 | — | 90 | — | 70 | — | 59 | — |
| 189 | — | 88 | — | 78 | — | 52 | — |

[1,2] = Mean of all replications.
[3] = Not tested.

*Southern Corn Rootworm*
*(Larvae of Spotted Cucumber Beetle)*

The test organisms are six- to ten-day old larvae of a chlorinated hydrocarbon resistant strain of *Diabrotica Undecimpunctata howardi* Barb. On the day before test compounds are to be screened, to one ounce polystyrene cups (one cup per test compound) are added: one level teaspoon (5 $cm^3$) air dried greenhouse soil, five corn seeds (*Zea mays L. var. Popcorn*) treated with thiram and a second teaspoon of dry soil. The following day, 0.45 ml of standard 128 p.p.m. formulation and 2.0 ml of water are added and the cups are capped.

The sample cups are stored under conditions of high humidity in a room at 22.2—25.6°C (72—78°F). After four days, the corn seedlings will have shoots 12—25 mm (0.5 to 1 inch) long, and they will have heaved the covering soil. At this time, 5 to 10 larvae are dropped into each test unit. The cups are then returned to the holding room. Three days after infestation, the test is read. Percentage mortality is determined at the dosage rate. The results are summarized in Table VIII.

TABLE VIII

| Compound Example No. | Rate (PPM) | Corn Rootworm (Percent Mortality) |
|---|---|---|
| 51 | 128 | 100 |
| 74 | 128 | 100 |
| | 64 | 100 |
| | 32 | 20 |
| 113 | 128 | 33 |
| | 64 | 46 |
| 115 | 128 | 55 |
| | | 9 |

In addition to the foregoing evaluations, certain of the thiol-hydroximidate compounds of the invention were subjected to the following special tests to determine the presence of other significant biological properties for these compounds.

*Plant Growth Regulant Activity*

Compounds of the invention were evaluated relative to plant growth regulant activity against three plant species in a petri dish test. The test is divided into a primary test where 500 µg and 100 µg chemical per petri dish (100 × 25 mm, disposable) are tested simultaneously and a secondary test where 50 µg, 10 µg and 1 µg chemical per petri dish are tested simultaneously. Compounds which pass the primary test are further evaluated in the secondary test. A special petri dish test may also be run to evaluate particular test compounds, in which case all five rates, i.e., 500, 100, 50, 10 and 1 µg per petri dish, are tested simultaneously.

According to the test procedure, three plant species are planted in a petri dish which has been treated with the test substance. The three species are as follows: 1) a mixture of approximately 50 percent light-sensitive and 50 percent light-insensitive lettuce (*Lactuca sativa* L. 'Grand Rapids'); 2) soft red winter wheat [*Triticum aestivum* L. (Aestivicum Group) 'Abe']; and 3) pasture-type perennial ryegrass (*Lolium perenne* L. 'Linn'). Wheat and perennial ryegrass seeds are surface-sterilized with 1 percent sodium hypochloride for 10 minutes and 5 minutes, respectively.

Each test compound is formulated in acetone and aliquots of the test formulation are placed on three layers of sterilized filter papers (Whatman No. 1, 8.26 cm diameter) in each petri dish. As soon as the acetone evaporates, 7 ml of deionized water are added into each petri dish with an appropriate automatic dispenser. Then, 5 to 8 wheat seeds, 10 to 15 perennial ryegrass seeds and 10 to 15 lettuce seeds are placed on the filter paper of each petri dish. Dishes are then covered and seeds are germinated for 3 days at 20°C at a relative humidity of 65 in the dark. The dishes are then removed from the dark growth chamber and maintained in lighted environmental growth chambers for 4 days.

At the end of the seventh day of planting, growth and developmental responses/characteristics are evaluated.

The compounds of Examples Nos. 1, 2, 10, 11, 24, 25, 26, 31, 36, 37, 44, 45, 58 and 90 exhibited growth retardant activity. Seed germination, root length and hypocotyl length of lettuce were inhibited (i.e., between 15 percent and 85 percent inhibition compared to untreated controls) at rates ranging from about 100 µg/dish to 2000 µg/dish. Root and coleoptile growth of wheat and ryegrass was inhibited at rates ranging from 10 µg/dish to 2000 µg/dish.

Growth stimulation activity was observed with growth of lettuce treated with these compounds was stimulated at rates ranging from about 50 µg/dish to 500 µg/dish.

*Antifungal Activity*

Preselected thiolhydroximidates of the invention were incorporated into an agar based fungal growth medium. Spore suspensions of five different fungus species were prepared. Drops of each suspension were incubated for 24 or 48 hours. The spores were observed microscopically and rated as indicated in Table IX.

TABLE IX

| Compound Ex. No. (1000 ppm) | Alternaria solani 24°C 24 hr | Helminthosporium oryzae 24°C 24 hr | Botrytis cinerea 20°C 24 hr | Colletotrichum lagenarium 20°C 48 hr | Colletotrichum trifoliorum 20°C 48 hr |
|---|---|---|---|---|---|
| 10 | 2 | 1 | 1 | 0 | 0 |
| 37 | 2 | 4 | 0 | 0 | 0 |
| 44 | 1 | 0 | 0 | 0 | 0 |
| 45 | 0 | 0 | 0 | 0 | 0 |

KEY
0 — no germination, no growth
1 — germination, no growth
2 — some growth
3 — ~50% inhibition
4 — slight inhibition

**Claims**

1. A heterobicyclic compound of the formula:

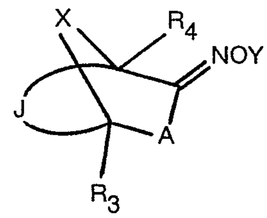

(I)

characterized in that either:

A represents S, O, $S(O)_m$, where m is 1 or 2, or $NR_5$, where $R_5$ is hydrogen, alkyl, aryl or cyano;

J represents the group

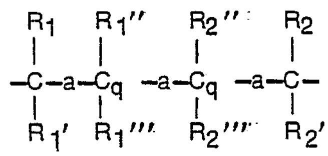

wherein q, independently, is 0 or 1, a, independently, is a single or double bond and $R_1$, $R_1'$, $R_1''$, $R_1'''$, $R_2$, $R_2'$, $R_2''$ and $R_2'''$ are as defined below;

X represents O, S, $NR_6$,

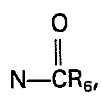

where $R_6$ is hydrogen or alkyl, or a bridge member selected from

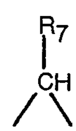 , 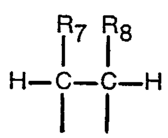 , 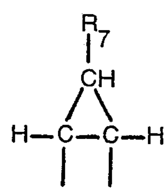 , 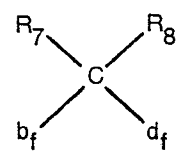 and 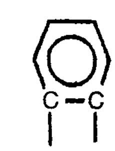 ,

where $R_7$ and $R_8$ independently represent hydrogen, halogen, cyano, alkyl, alkoxy, alkoxycarbonyl or alkylthio, and b and d independently represent carbon or oxygen and f is 0 or 1;

$R_1$—$R_1'''$, inclusive, $R_2$—$R_2'''$, inclusive, $R_3$ and $R_4$ independently represent hydrogen, halogen, hydroxy, cyano, alkyl, alkoxy, haloalkyl, alkylcarboxy, arylcarboxy, alkylaminocarboxy, carbamoyl, alkylcarbamoyl, dialkylcarbamoyl, alkylthio, alkylsulphinyl, alkylsulphonyl, alkylamino, dialkylamino, alkoxycarbonyl, trifluoromethyl, pyrrolidyl, phenyl, nitro, thiocyano, thiocarbamyl, alkylthiocarbamyl, dialkylthiocarbamyl, arylthio carbamyl or the group

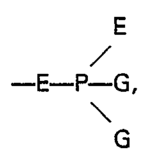

where E is 0 or S and G represents alkyl, alkoxyl, alkylthio, amino, alkylamino or dialkylamino; or, when a is a single bond and at least two of $R_1'$, $R_1'''$, $R_2'$ or $R_2'''$ are hydrogen on adjacent C atoms, $R_1$, $R_1''$, $R_2$ or $R_2''$ on the same adjacent C atoms together represent

—O—, 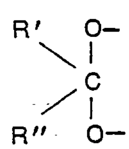 or 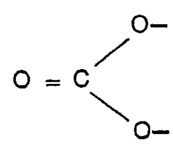 ,

where R′ and R″ represent hydrogen or alkyl; or any of $R_1$ and $R_1'$, $R_1''$ and $R_1'''$, $R_2''$ and $R_2'''$ or $R_2$ and $R_2'$ represent =O; or when q, independently, is 0 or 1 and a, independently, is a double bond, $R_1'$, $R_1'''$, $R_2'''$ or $R_2'$ are absent; and

Y represents hydrogen or

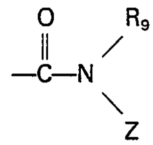

wherein $R_9$ represents hydrogen, alkyl, hydroxyalkyl, alkenyl, alkynyl, aralkyl, alkoxyalkyl or polyoxyalkylene; and

Z represents

a) 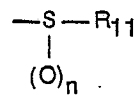 ,

wherein n is 0, 1 or 2 and $R_{11}$ is pyridyl, pyrimidyl, phenyl or phenyl substituted with at least one member selected from hydroxy, alkyl, alkoxy, halogen, nitro, trifluoromethyl and cyano;

b) 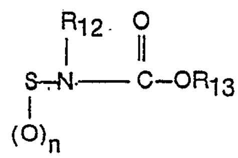 ,

wherein n is 0, 1 or 2, and $R_{12}$ is alkyl, alkoxyalkyl or

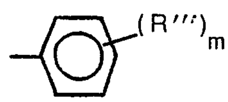 ,

where m is 0, 1, 2 or 3 and R‴ is hydrogen, halogen, cyano, nitro, alkyl, alkoxy, alkylthio, alkylsulphonyl or phenyloxy, and $R_{13}$ is alkyl, alkoxyalkyl, naphthyl, alkylthioalkyl,

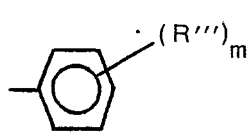 , 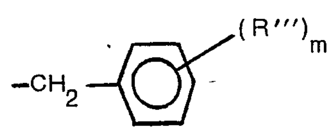 ,

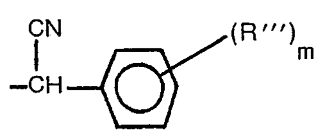 or 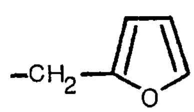

wherein $(R''')_m$ has the meaning defined above,

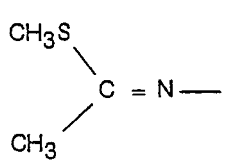 , 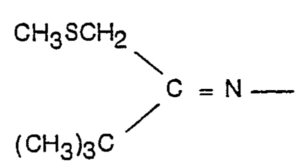

or Q, where Q—OY represents formula (I) as defined above;

c) 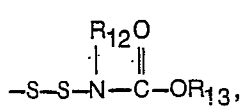

wherein $R_{12}$ and $R_{13}$ have the meanings defined above;

d) 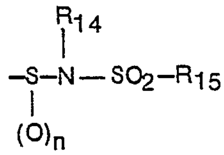 ,

where n is 0, 1 or 2, $R_{14}$ is phenyl, alkyl, alkoxyalkyl, alkoxycarbonylalkyl, alkylthioalkyl or carboxyalkyl and $R_{15}$ is alkyl,

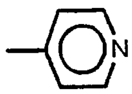 , 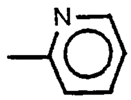 , 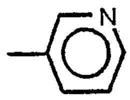 or 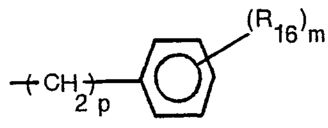

where m is 0—5, p is 0—5, and $R_{16}$ is halogen, alkyl, trifluoromethyl, nitro or alkoxy;

e) $-S-NR_{17}R_{18}$,

where $R_{17}$ and $R_{18}$ are alkyl or aryl or together with the nitrogen atom represent

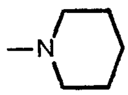 , 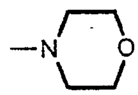 , 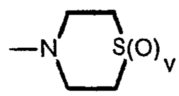 ,

where v is 0, 1 or 2, or

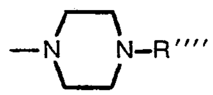 ,

where R'''' is alkyl;

f) 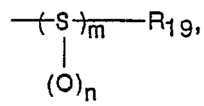

where n is 0, 1 or 2, m is 1 or 2 and $R_{19}$ is alkyl, cycloalkyl, haloalkyl, cyanoalkyl, alkoxycarbonyl, (alkylthio)carbonyl, alkoxy(thiocarbonyl), alkylthio(thiocarbonyl), aryl or substituted aryl with at least one substituent selected from halogen, cyano, nitro, alkyl, alkoxy, alkylthio, alkylsulphonyl and phenyloxy, with the proviso that when $R_{19}$ is aryl or substituted aryl, m is 2;

g) 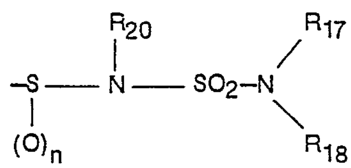

where n is 0, 1 or 2, $R_{20}$ is alkyl and $R_{17}$ and $R_{18}$ have the meanings defined above;

h) 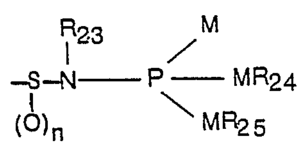 ,

where n is 0, 1 or 2, each M, independently, is S or O and $R_{23}$, $R_{24}$ and $R_{25}$ independently represent alkyl or $R_{24}$ and $R_{25}$ together represent

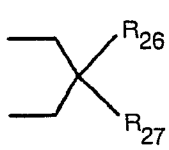 or 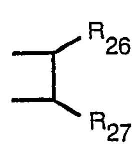 ,

where $R_{26}$ and $R_{27}$ independently represent hydrogen or alkyl;

i) 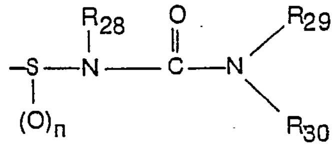 ,

where n is 0, 1 or 2, $R_{28}$ is alkyl or aryl and $R_{29}$ and $R_{30}$ independently represent hydrogen, alkyl, aryl or alkoxy;

j) 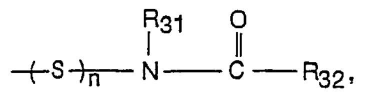

where n is 1 or 2, $R_{31}$ is alkyl and $R_{32}$ is fluoro, alkyl, aryl or aralkyl;

k) 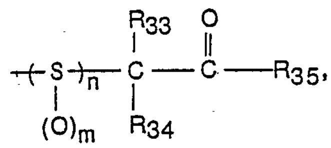

where m is 0, 1 or 2, n is 1 or 2 and $R_{33}$, $R_{34}$ and $R_{35}$ inpendently represent hydrogen, alkyl or aryl; or

l) 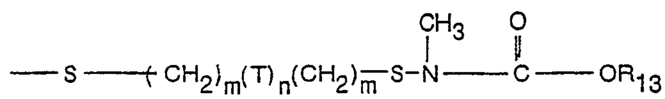 ,

where T is O, S or $—CH_2—$, m is 1 or 2, n is 0 or 1 and $R_{13}$ has the meaning defined above; wherein, in the meanings defined above, unless otherwise specified, "alkyl" and "alkoxy" signify groups which contain 1 to 22 carbon atoms and include all straight and branched structures, "alkenyl" and "alkynyl" signify groups which contain up to 22 carbon atoms and include all straight and branched structures, "cycloalkyl" signifies groups which contain 3 to 8 carbon atoms, "aryl" signifies groups which contain 6 to 12 carbon atoms and "aralkyl" signifies groups which contain an aryl group, as above defined, attached to an alkyl group, as above defined; or A represents S, J represents —CH = CH—, X represents $—CH_2CH_2—$, $R_3$ represents H, $R_4$ represents CN and Y represents $C(=O)N(CH_3)SN(C_{12}H_{25}—N)So_2NC_4H_8$.

2. A heterobicyclic compound of the formula:

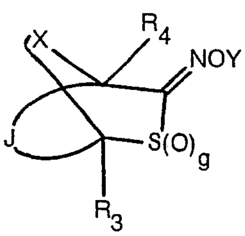

wherein g is 0, 1 or 2, J, X and Y have the meanings defined in claim 1 and

$R_1$—$R_1'''$, inclusive, $R_2$—$R_2'''$, inclusive, $R_3$ and $R_4$ independently represent hydrogen, halogen, hydroxy cayno, alkyl, alkoxy, haloalkyl, alkylcarboxy, arylcarboxy, alkylaminocarboy, carbamoyl, alkyl carbamoyl, dialkylcarbamoyl, alkylthio, alkylsulphinyl, alkylsulphonyl, alkylamino, dialkylamino, alkoxycarbonyl, trifluoromethyl, pyrrolidyl, phenyl, nitro, thiocyano, thiocarbamyl, alkylthiocarbamyl, dialkylthiocarbamyl, arylthiocarbamyl or the group

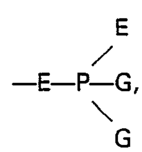

where E is O or S and G represents alkyl, alkoxy, alkylthio, amino, alkylamino or dialkylamino.

3. A compound according to claim 1, wherein A and J have the meanings defined in claim 1, X is a bridge member selected from

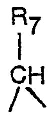 , 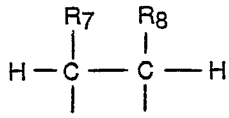 , 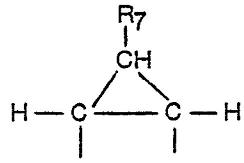 ,

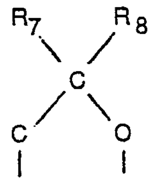 or 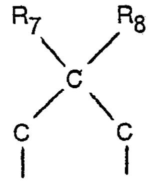 ,

where $R_7$ and $R_8$ independently represent hydrogen or cyano,

$R_1$—$R_1'''$, inclusive, and $R_2$—$R_2'''$, inclusive, are hydrogen or, when a is a double bond $R_1$ and $R_2$ and, in addition, if q, independently in each case, is 1, $R_1''$ or $R_2''$ also are independently hydrogen or halogen and $R_1'$, $R_2'$, $R_1'''$ or $R_2'''$ are absent;

$R_3$ and $R_4$ independently represent hydrogen, halogen, cyano or alkoxycarbonyl;

Y represents

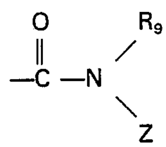

where $R_9$ represents hydrogen, alkyl, hydroxyalkyl, alkenyl or alkynyl; and Z represents

a) 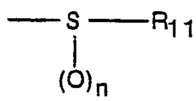 ,

where n is 0, 1 or 2 and $R_{11}$ is phenyl or phenyl substituted with at least one member selected from halogen, alkyl, alkoxy, trifluoromethyl, nitro and cyano;

b) 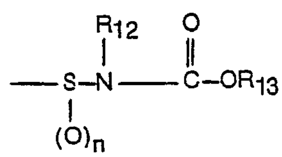 ,

where n is 0, 1 or 2, $R_{12}$ is alkyl and $R_{13}$ is alkyl, alkoxyalkyl or Q, where Q—OY represents formula (I) as defined in claim 1;

c) 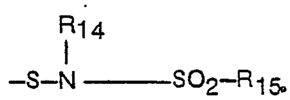 ,

where $R_{14}$ is alkyl and $R_{15}$ is alkyl or

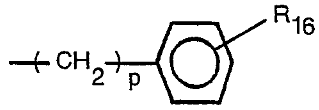

where p is 0—5 and $R_{16}$ is halogen, alkyl, trifluoromethyl, nitro, methoxy, cyano or dialklylamino;

d) $-S-NR_{17}R_{18}$,

where $R_{17}$ and $R_{18}$ are alkyl or aryl or together with the nitrogen atom represent

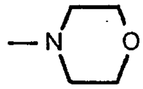 or 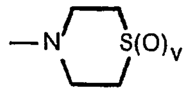 ,

where v is 0, 1 or 2,

e) $-(S)_n-R_{19}$,

where n is 1 or 2 and $R_{19}$ is alkyl, cyanoalkyl, or alkoxycarbonyl;

f) 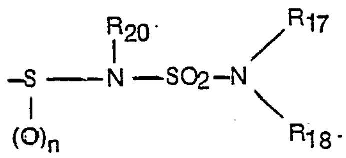

where n is 0, 1 or 2, $R_{20}$ is alkyl and $R_{17}$ and $R_{18}$ have the meanings defined above herein;

g) 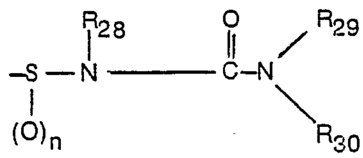,

where n is 0, 1 or 2, $R_{28}$ is alkyl or aryl and $R_{29}$ and $R_{30}$ independently represent hydrogen, alkyl, aryl or alkoxy; or

h) 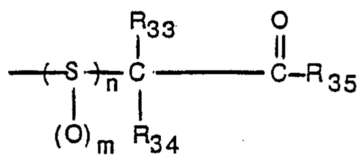,

where m is 0, 1 or 2, n is 1 or 2 and $R_{33}$, $R_{34}$ and $R_{35}$ independently represent hydrogen, alkyl or aryl.

4. A compound according to claim 1, wherein

A represents S or O;

J has the meaning defined in claim 1;

X is a bridge member selected from

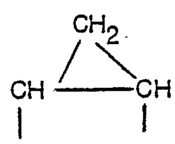;

$R_1$—$R_1'''$, inclusive, $R_2$—$R_2'''$, inclusive, $R_3$ and $R_4$ have the meanings defined in claim 3; and

Y represents

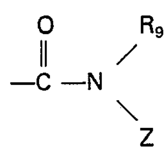

where $R_9$ represents hydrogen, alkyl, alkenyl or alkynyl; and Z represents

a) 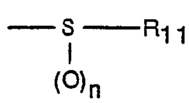,

where n is 0, 1 or 2, $R_{11}$ is alkyl phenyl or phenyl subsituted with at least one member selected from halogen, alkyl, alkoxy, trifluoromethyl, nitro and cyano;

b) 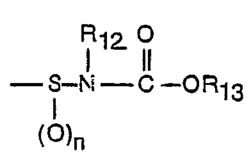,

where n is 0, 1 or 2, $R_{12}$ is alkyl and $R_{13}$ is alkyl, alkoxyalkyl or Q, where Q—OY represents formula (I) as defined in claim 1;

c) 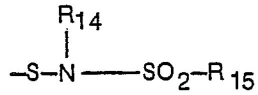 ,

where $R_{14}$ and $R_{15}$ are alkyl, aryl, or substituted aryl with at least one substituent selected from halogen, alkyl, triflurormethyl, nitro, methoxy, cyano and dialkylamino;

d) $-S-NR_{17}R_{18}$,

where $R_{17}$ and $R_{18}$ are alkyl or aryl or together with the nitrogen atom represent

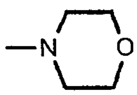 , 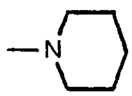 or 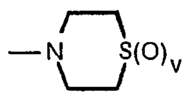 ,

where v is 0, 1 or 2;

e) 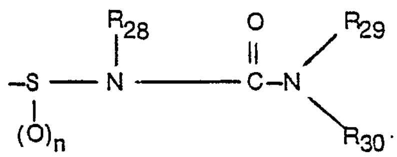 ,

where n is 0, 1 or 2, $R_{28}$ is alkyl or aryl and $R_{29}$ and $R_{30}$ independently represent hydrogen, alkyl, aryl or alkoxy; and

f) 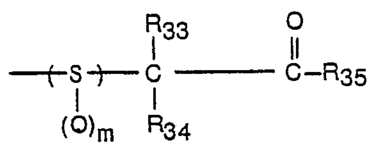 ,

where m is 0, 1 or 2, n is 1 or 2 and $R_{33}$, $R_{34}$ and $R_{35}$ independently represent hydrogen, alkyl or aryl.

5. A compound according to claim 1, containing the structure

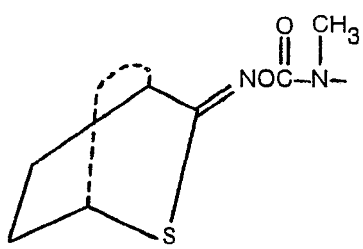

and selected from the group consisting of

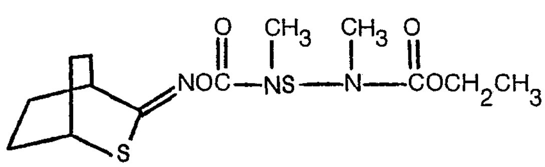 ,

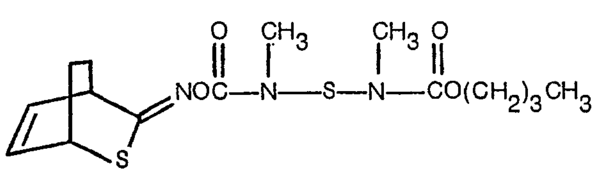 ,

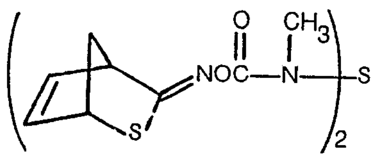 ,

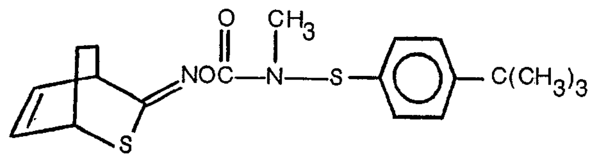 ,

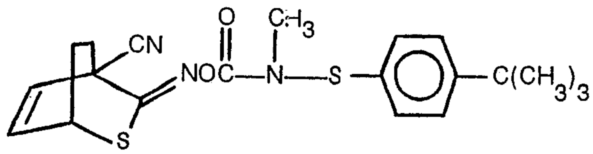 ,

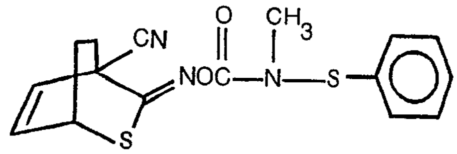 ,

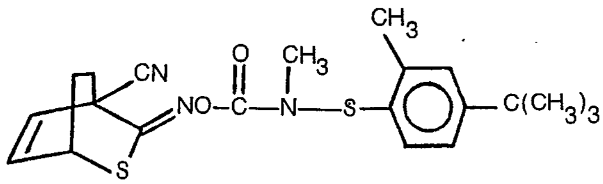

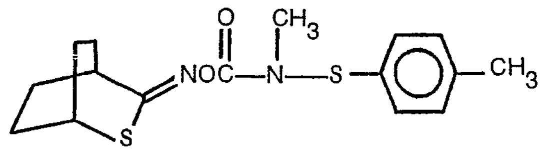 ,

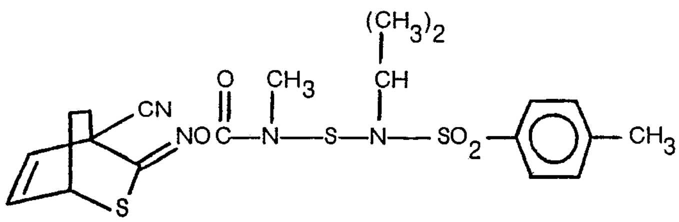

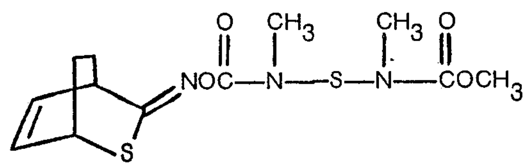 ,

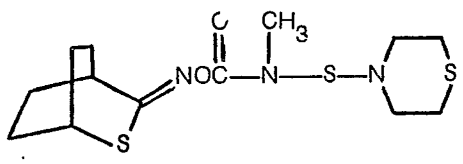 ,

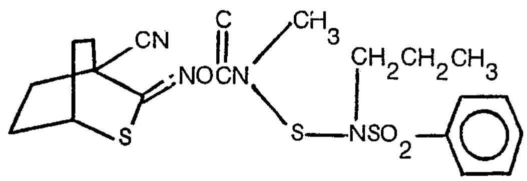 ,

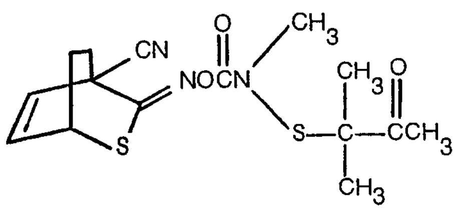 ,

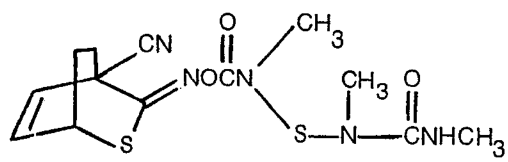 ,

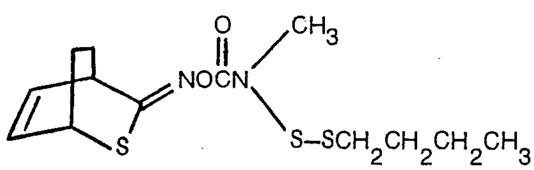 ,

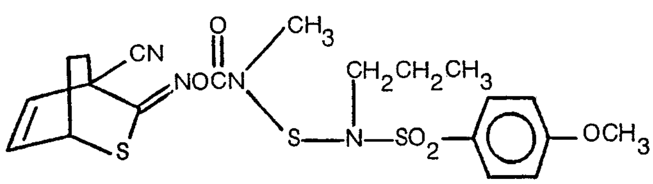 ,

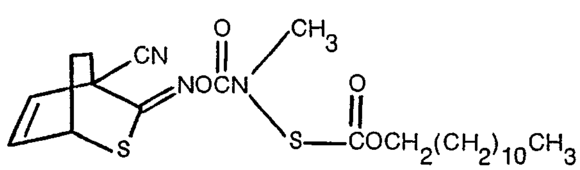 ,

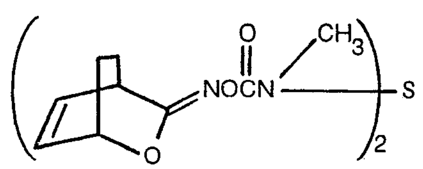 ,

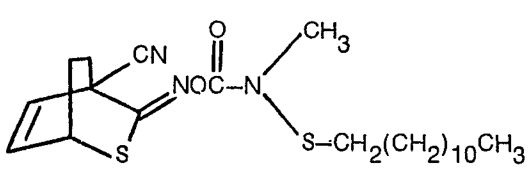 ,

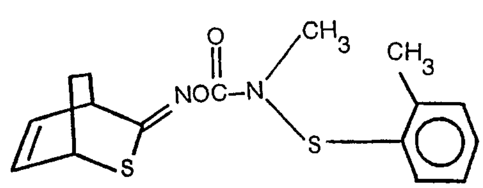 ,

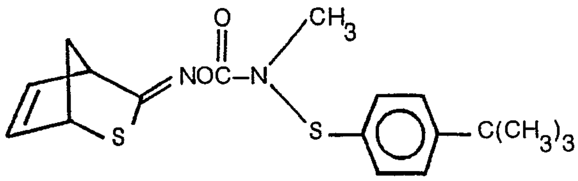

and

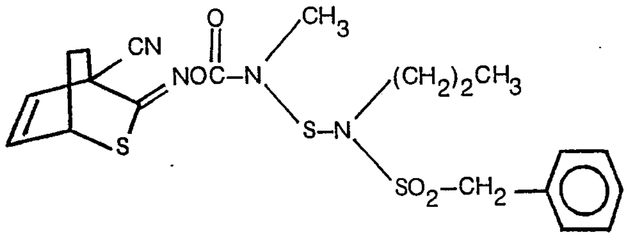

6. An arthropodicidal composition, comprising a carrier and a heterobicyclic compound according to any of claims 1 to 5.

7. A nematocidal composition, comprising a carrier and a heterobicyclic compound according to claim 2, wherein, in the formula, g is 0.

8. A method of combating arthropods or nematodes, which comprises applying to the arthropods or nematodes or a habitat thereof, an effective amount of at least one compound according to claim 2, wherein, in the formula, g is 0 or of at least one composition according to claim 7.

9. A method according to claim 8, wherein the compound or the composition is applied directly to the arthropods or the nematodes so that the compound is provided in an amount in the range from 0.112 to 22.4 kg/ha (0.1 to 20 lb/A).

10. A method according to claim 9, wherein the amount is in the range from 0.28 to 4.48 kg/ha (0.25 to 4 lb/A).

11. A process of preparation of a compound of the formula:

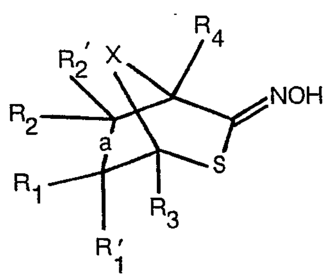

wherein

a is a single or double bond;

X represents O, S, $NR_6$,

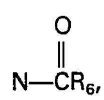

where $R_6$ is alkyl, or a bridge member selected from

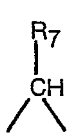 , 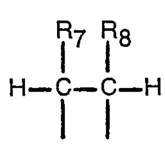 , 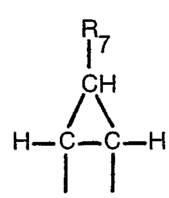 , 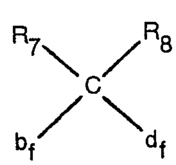 and 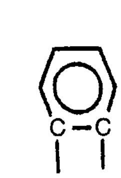 ,

where $R_7$ and $R_8$ independently represent hydrogen, halogen, cyano, alkyl, alkoxy, alkoxycarbonyl or alkylthio and b and d independently represent carbon or oxygen and f is 0 or 1;

$R_1'$—$R_4$, inclusive, independently represent hydrogen, halogen, hydroxy, cyano, alkyl, alkoxy, haloalkyl, alkylcarboxy, arylcarboxy, alkylaminocarboxy, carbamoyl, alkylcarbamoyl, dialkylcarbamoyl, alkylthio, alkylsulphinyl, alkylsulphonyl, alkylamino, dialkylamino, alkoxycarbonyl, trifluoromethyl, pyrrolidyl, phenyl, nitro, thiocyano, thiocarbamyl, alkylthiocarbamyl, dialkylthiocarbamyl, arylthiocarbamyl or the group

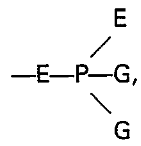

where E is O or S and G represents alkyl, alkoxy, alkylthio, amino, alkylamino or dialkylamino; or, when a is a single bond and $R_1'$ and $R_2'$ are hydrogen, $R_1$ and $R_2$ together represent

—O—, 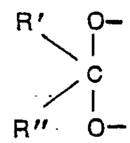 or 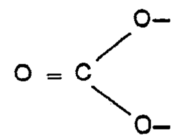 ,

where R′ and R″ represent hydrogen or alkyl; or $R_1'$ and $R_1$ or $R_2$ and $R_1'$ represent =O; or, when a is a double bond, $R_2'$ and $R_2'$ are absent; wherein a cyclic diene of the formula

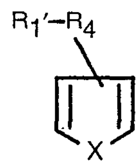 ,

wherein $R_1'$—$R_4$ and X have the meanings defined above herein, is reacted with a dienophile selected from

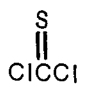 , 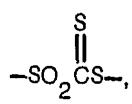 , 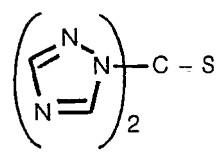 and 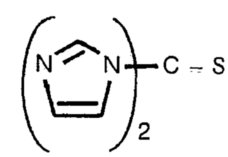

under cyclization conditions, characterized in that the resulting adduct is reacted with hydroxylamine to form a thiolhydroximidate.

12. A process of preparation of a compound according to claim 1, wherein, in the formula, A represents —O— and Y represents H, characterized in that a cyclic diene of the formula

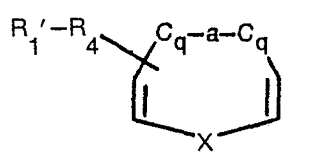 ,

wherein $R_1'$—$R_4$, X, q and a have the meanings defined in claim 1, is reacted with chlorosulphonyl isocyanate under cyclization conditions to form an imino-oxabicyclic compound of the formula

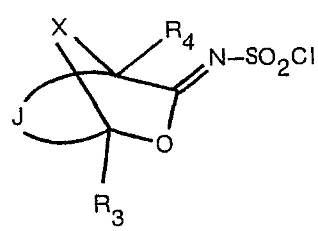

wherein J, X $R_3$ and $R_4$ have the meanings defined in claim 1, and the imino-oxabicyclic compound is reacted with hydroxylamine to form an oxahydroximidate.

**Patentansprüche**

1. Eine heterobicyclische Verbindung der Formel

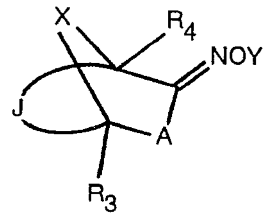

(I)

dadurch gekennzeichnet, daß entweder

A = S, O, $S(O)_m$ ist, wobei m = 1 oder 2 bzw. $NR_5$ ist, wobei $R_5$ ein Wasserstoff, Alkyl, Aryl oder eine Cyanogruppe ist;

J die Gruppe 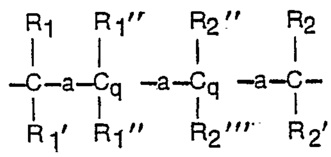

darstellt, wobei q, unabhängig 0 oder 1 ist, a unabhängig eine Einfach- oder Doppelbindung darstellt und $R_1$, $R_1'$, $R_1''$, $R_1'''$, $R_2$, $R_2'$, $R_2''$ und $R_2'''$ die weiter unten definierte Bedeutung hat;

X = O, S, $NR_6$, 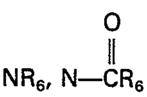

ist, wobei $R_6$ Wasserstoff oder Alkyl bzw. ein Brückenglied ist, welches aus den Gruppen

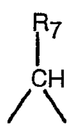 , 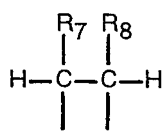 , 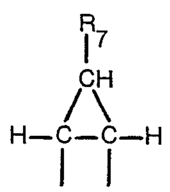 , 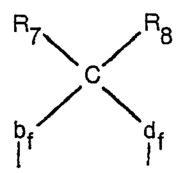 und 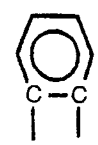 ,

ausgewählt ist, wobei $R_7$ und $R_8$ unabhängig voneinander Wasserstoff, Halogen, Cyano, Alkyl, Alkoxy, Alkoxycarbonyl oder Alkylthiogruppen darstellen und b und d unabhängig für Kohlenstoff oder Sauerstoff stehen und f = 0 oder 1 ist;

$R_1$—$R_1'''$ incl., $R_2$ bis $R_2'''$ incl., $R_3$ und $R_4$ stellen unabhängig Wasserstoff, Halogen, Hydroxy, Cyano, Alkyl, Alkoxy, Halogenalkyl, Alkylcarboxy, Arylcarboxy, Alkylaminocarboxy, Carbamoyl, Alkylcarbamoyl, Dialkylcarbamoyl, Alkylthio, Alkylsulfinyl, Alkylsulfonyl, Alkylamin, Dialkylamin, Alkoxycarbonyl, Trifluormethyl, Pyrrolidyl, Phenyl, Nitro, Thiocyano, Thirocarbamyl, Alkylthiocarbamyl, Dialkylthiocarbamyl, Arylthiocarbamyl oder die Gruppe

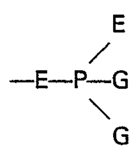

dar, wobei E gleich 0 oder S ist und G eine Alkyl, Alkoxy, Alkylthio, Amino, Alkylamino oder Dialkylaminogruppe darstellt; oder, falls a eine Einfachbindung ist und wenigstens zwei der $R_1'$, $R_1'''$, $R_2'$ oder $R_2'''$ Reste Wasserstoffatome an nebeneinanderliegenden C-Atomen sind, dann stellen $R_1$, $R_1''$, $R_2$ oder $R_2'''$ andenselben aneinanderliegenden C-Atomen zusammen die Gruppen

—O—, 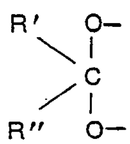 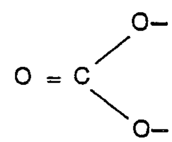 ,

dar,
wobei R′ und R″ Wasserstoff oder Alkylreste sind; oder eine der beiden $R_1$ und $R_1'$, $R_1''$ und $R_1'''$, $R_2''$ und $R_2'''$ oder $R_2$ und $R_2'$ Gruppen gleich 0 sind oder falls q unabhängig 0 oder 1 ist und a unabhängig für eine Doppelbindungf steht, sind $R_1'$, $R_1'''$, $R_2'''$ oder $R_2'$ nicht vorhanden; und

Y stellt Wasserstoff oder

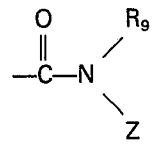

dar, wobei $R_9$ Wiederum für Wasserstoff, Alkyl, Hydroxyalkyl, Alkenyl, Alkynyl, Aralkyl, Alkoxyalkyl oder Polyoxyalkylen steht; und Z eine im folgenden definierten Gruppen bedeutet,

a) 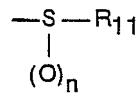 ,

, wobei n = 0, 1 oder 2 ist und $R_{11}$ Pyridyl, Pyrimidyl, Phenyl oder ein substituierter Phenylrest ist, bei dem mindestens ein Substituent eine Hydroxyalkyl, Alkoxyhalogen, Nitrotrifluormethyl oder Cyanogruppe ist;

b) 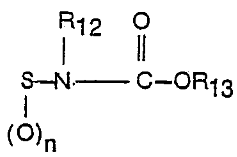 ,

wobei n = 0, 1 oder 2 und $R_{12}$ gleich Alkyl, Alkoxyalkyl oder

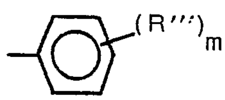 ,

ist, wobei m = 0, 1, 2 oder 3 ist, und R‴ Wasserstoff, Halogen, Cyano, Nitro, Alkyl, Alkoxy, Alkylthio, Alkylsulfonyl oder Phenyloxy ist und $R_{13}$ = Alkyl, Alkoxyalkyl, Naphthyl, Alkylthioalkyl,

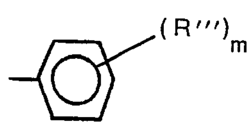 , 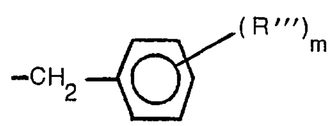 ,

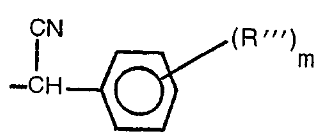 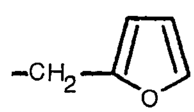

ist,
wobei $(R''')_m$ die oben definierte Bedeutung hat,

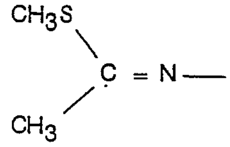 , 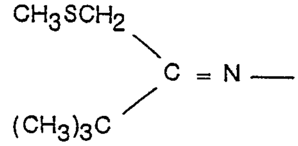

oder Q, wobei Q—OY die obden definierte Formel I darstellt;

c) 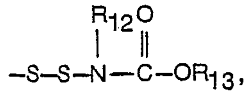

, wobei $R_{12}$ und $R_{13}$ die oben definierte Bedeutung haben;

d) 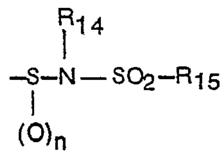 ,

wobei n = 0, 1 oder 2 ist und $R_{14}$ für Phenyl, Alkyl, Alkoxyalkyl, Alkoxycarbonylalkyl, Alkylthioalkyl oder Carboxyalkyl steht und $R_{15}$ = Alkyl,

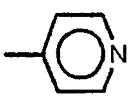 , 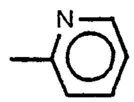 , 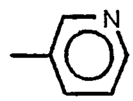 oder 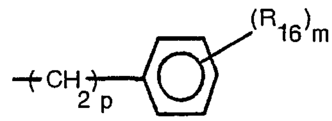

wobei m = 0 bis 5 ist, und p = 0 bis 5 ist, und $R_{16}$ ein Halogen, Alkyltrifluormethyl, Nitro oder Alkoxy darstellt;

e) $-S-NR_{17}R_{18}$,

wobei $R_{17}$ und $R_{18}$ Alkyl oder Arylreste sind oder zusammen mit dem Stickstoffatom eine der folgenden Gruppen darstellen

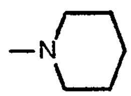 , 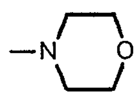 , 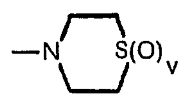 ,

wobei v = 0, 1 oder 2 ist oder

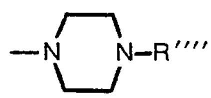 ,

wobei R'''' ein Alkylrest ist;

f) 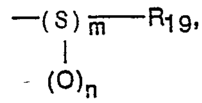

wobei n = 0, 1 oder 2 ist und m = 1 oder 2 ist und $R_{19}$ ein Alkyl, Cycloalkyl, Halogenalkyl, Cyanoalkyl, Alkoxycarbonyl (Alkylthio)carbonyl, Alkoxy(thiocarbonyl), Alkylthio(thiocarbonyl), Aryl oder substituierter Arylrest ist mit wenigstens einem Substituenten aus der Gruppe Halogen, Cyano, Nitro, Alkyl, Alkoxy, Alkylthio, Alkylsulfonyl und Phenyloxy, mit dem Vorbehalt, daß wenn $R_{19}$ eine Aryl oder substituierte Arylgruppe ist m den Wert 2 hat;

g) 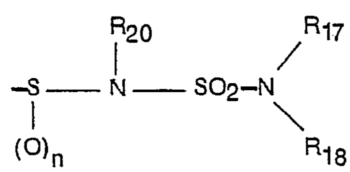

wobei n = 0, 1 oder 2 ist und $R_{20}$ für Alkyl steht und $R_{17}$ und $R_{18}$ die oben definierten Bedeutungen haben;

h) 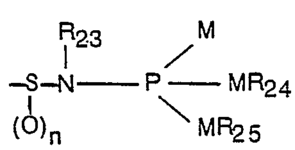 ,

mit n = 0 bis 2 und wobei jedes M unabhängig S oder O sein kann und $R_{23}$, $R_{24}$ und $R_{25}$ unabhängig einen Alkylrest darstellen oder $R_{24}$ und $R_{25}$ zusammen die Gruppen

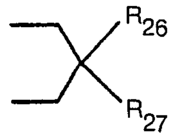

oder

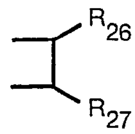

,

darstellen,
wobei $R_{26}$ und $R_{27}$ unabhängig eine Wasserstoff-oder Alkylgruppe ist;

i)

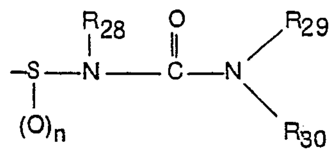

,

wobei n = 0, 1 oder 2 ist und $R_{28}$ = Alkyl oder Aryl und $R_{29}$ und $R_{30}$ unabhängig Wasserstoff, Alkyl, Aryl oder Alkoxy sind;

j)

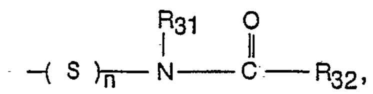

wobei n = 0 oder 2 ist, $R_{31}$ für Alkyl und $R_{32}$ für Fluor, Alkyl, Aryl oder Aralkyl steht;

k)

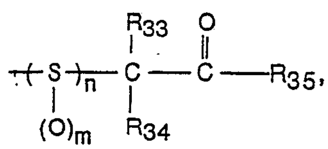

wobei m = 0, 1 oder 2, n = 1 oder 2 ist und $R_{33}$, $R_{34}$ und $R_{35}$ unabhängig Wasserstoff, Alkyl oder Aryl darstellen; oder

l)

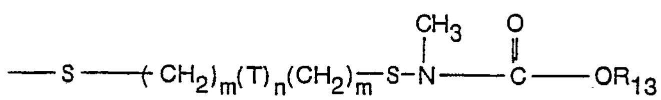

,

wobei R = O, S oder —$CH_2$— ist, m 1 oder 2 ist, n 0 oder 1 ist und wobei $R_{13}$ die zuvor definierte Bedeutung hat; und wobei, sofern nicht anders angegeben "Alkyl" und "Alkoxy" gemäß der oben definierten Bedeutung solche Gruppen darstellen, die 1 bis 22 Kohlenstoffatome enthalten und alle geradkettigen und verzweigten Strukturen einschließen, "Alkenyl" und "Alkynyl" bedeuten Gruppen, die bis zu 22 Kohlenstoffatome enthalten und schließ ebenfalls alle geradkettigen und verzweigten Strukturen ein, "Cycloalkyl" bedeutet Gruppen, die 3 bis 8 Kohlenstoffatome enthalten, "Aryl" bedeutet Gruppen, die 6 bis 12 Kohlenstoffatome enthalten und "Aralkyl" bedeutet Gruppen, die eine der oben definierten Arylgruppen enthält, die mit einer der oben definierten Alkylgruppen verknüpft ist;
oder
A stellt S, J stellt —CH=CH—, X stellt —$CH_2CH_2$—, $R_3$ stellt H, $R_4$ stellt CN und Y stellt $C(=O)N(CH_3)SN(C_{12}H_{25}\text{-n})SO_2NC_4H_9$ dar.

2. Eine heterobicyclische Verbindung der Formel

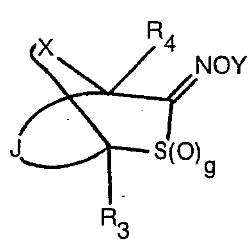

wobei g = 0, 1 oder 2 ist, J, X und Y die in Anspruch 1 definierten Bedeutungen haben und $R_1$—$R_1'''$ incl., $R_2$—$R_2'''$ incl., $R_3$ und $R_4$ stehen unabhängig für Wasserstoff, Halogen, Hydroxy, Cyano, Alkyl, Alkoxy, Halogenalkyl, Alkylcarboxy, Arylcarboxy, Alkylaminocarboxy, Carbamoyl, Alkylcarbamoyl, Dialkylcarbamoyl, Alkylthio, Alkylsulfinyl, Alkylsulfonyl, Alkylamino, Dialkylamino, Alkoxycarbonyl, Trifluormethyl, Pyrrolidyl Phenyl, Nitro, Thiocyano, Thiocarbamyl, Alkylthiocarbamyl, Dialkylthiocarbamyl, Arylthiocarbamyl oder die Gruppe

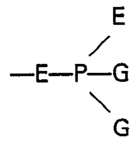

wobei E = O oder S und G = Alkyl, Alkoxy, Alkylthio, Amino, Alkylamino oder Dialkylamino ist.

3. Eine Verbindung nach Anspruch 1, wobei A und J die Bedeutungen wie in Anspruch 1 definiert, aufweisen, und X eine Brückenglied ist, das aus den Gruppen

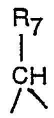 , 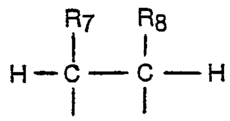 , 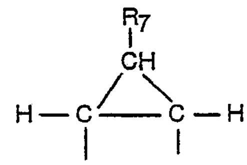 ,

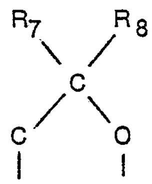 oder 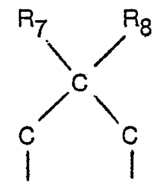 ,

ausgewählt wird, wobei $R_7$ und $R_8$ unabhängig Wasserstoff oder Cyano darstellen; $R_1$ bis $R_1'''$ incl., und $R_2$ bis $R_2'''$ incl., sind Wasserstoff oder wenn a eine Doppelbindung ist, $R_1$ und $R_2$ falls q zusätzlich und in jedem Falle unabhängig = 1 ist, $R_1''$ oder $R_2''$ ebenfalls unabhängig für Wasserstoff oder Halogen stehen und $R_1'$, $R_2'$, $R_1'''$ oder $R_2'''$ sind nicht vorhanden; $R_3$ und $R_4$ stehen unabhängig für Wasserstoff, Halogen, Cyano oder Alkoxycarbonyl; Y steht für

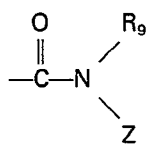

wobei $R_9$ Wasserstoff, Alkyl, Hydroxyalkyl, Alkenyl oder Alkynyl darstellen und Z

a) 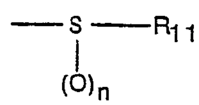 ,

darstellt, wobei n = 0, 1 oder 2 ist und $R_{11}$ Phenyl oder substituiertes Phenyl mit wenigstens einem Substituenten, der aus der Gruppe Halogen, Alkyl, Alkoxy, Trifluormethyl, Nitro und Cyano ausgewählt ist; oder

b) 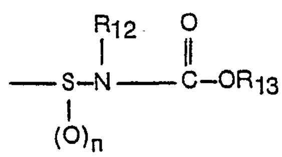 ,

darstellt, wobei n = 0, 1 oder 2 ist, $R_{12}$ Alkyl und $R_{13}$ Alkyl, Alkoxyalkyl oder Q darstellt, wobei Q—OY die im Anspruch 1 definierte Formel I bedeutet;

c) 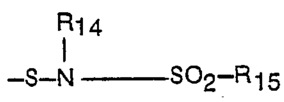 ,

wobei $R_{14}$ = Alkyl und $R_{15}$ = Alkyl oder

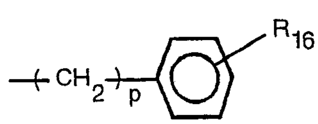

ist, wobei p 0 bis 5 ist und $R_{16}$ für Halogen, Alkyl, Trifluormethyl, Nitro, Methoxy, Cyano oder Dialkylamino steht; oder

d) $-S-NR_{17}R_{18}$,

darstellt, wobei $R_{17}$ und $R_{16}$ Alkyl- oder Arylgruppen sind oder zusammen mit dem Stickstoffatom

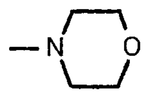 oder 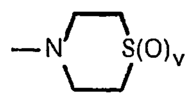 ,

darstellt, wobei v = 0, 1 oder 2 ist; oder

e) $-(S)_n-R_{19}$ ,

darstellt, wobei n 1 oder 2 ist und $R_{19}$ für Alkyl, Cyanoalkyl oder Alkoxycarbonyl steht; oder

f) 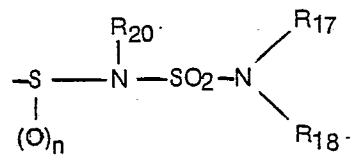

darstellt, wobei n = 0, 1 oder 2 ist und $R_{20}$ eine Alkylgruppe darstellt und $R_{17}$ und $R_{18}$ die oben definierten Bedeutungen aufweisen; oder

g) 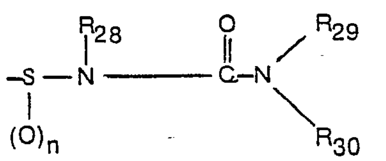 ,

darstellt,
wobei n = 0, 1 oder 2 ist, $R_{28}$ für Alkyl oder Aryl steht und $R_{29}$ und $R_{30}$ unabhängig für Wasserstoff, Alkyl, Aryl oder Alkoxy stehen oder

h) 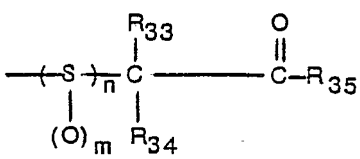 ,

darstellt,
wobei m = 0, 1 oder 2 und n = 1 oder 2 ist und $R_{33}$, $R_{34}$ und $R_{35}$ unabhängig für Wasserstoff, Alkyl oder Aryl stehen.

4. Verbindung nach Anspruch 1, wobei
A S oder O darstellt;
J die in Anspruch 1 definierte Bedeutung hat;
X ein Brückenglied darstellt, das aus der Gruppe

$-CH_2-$, $-CH_2-CH_2-$ und

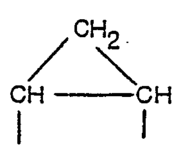 ;

ausgewählt ist;
$R_1$ bis $R_1'''$ incl., $R_2$ bis $R_2'''$ incl., $R_3$ und $R_4$ die in Anspruch 3 definierten Bedeutungen haben; und Y die Gruppe

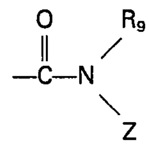

darstellt, wobei $R_9$ für Wasserstoff, Alkyl, Alkenyl oder Alkynyl steht, und Z

a) 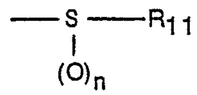 ,

darstellt, wobei n = 0, 1 oder 2 ist, $R_{11}$ für Alkyl, Phenyl oder substituiertes Phenyl steht, mit wenigstens einem Substituenten, der aus der Gruppe Halogen, Alkyl, Alkoxy, Trifluormethyl, Nitro und Cyano ausgewählt ist; oder

b) 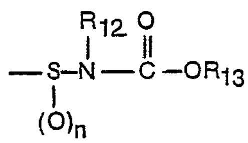 ,

darstellt, wobei n = 0, 1 oder 2 ist, $R_{12}$ für Alkyl und $R_{13}$ für Alkyl, Alkoxyalkyl oder Q steht, wobei Q—OY die Formel (I) des Anspruchs 1 darstellt; oder

c) 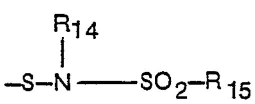 ,

darstellt, wobei $R_{14}$ und $R_{15}$ Alkyl, Aryl oder einen substituierten Arylrest darstellen, wobei mindestens ein Substituent der aus der Gruppe Halogen, Alkyl, Trifluormethyl, Nitro, Methoxy, Cyan oder Dialkylamino ausgewählt wird; oder

d) $-S-NR_{17}R_{18}$,

darstellt, wobei $R_{17}$ und $R_{18}$ Alkyl oder Arylreste sind oder zusammen mit dem Stickstoffatom

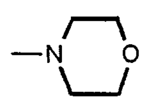 , 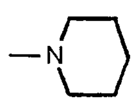 oder 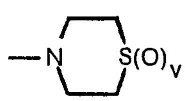 ,

darstellen, wobei v = 0, 1 oder 2 ist; oder

e) 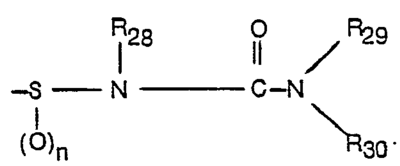 ,

darstellt, wobei n = 0, 1 oder 2 ist, $R_{28}$ für Alkyl oder Aryl steht und $R_{29}$ und $R_{30}$ unabhängig Wasserstoff, Alkyl, Aryl oder Alkoxy darstellen und

f) 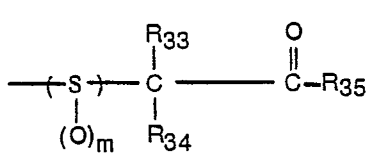 ,

darstellt, wobei m = 0, 1 oder 2 und n = 1 oder 2 ist und $R_{33}$, $R_{34}$ und $R_{35}$ unabhängig Wasserstoff, Alkyl oder Aryl darstellen.

5. Eine Verbindung nach Anspruch 1, die die Struktur

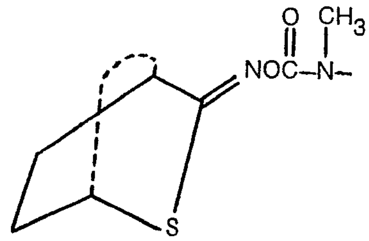

aufweist und aus der Gruppe, bestehend aus

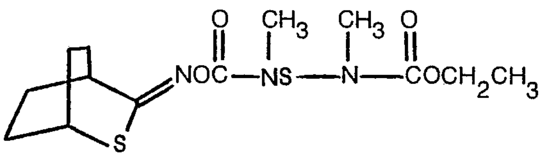 , 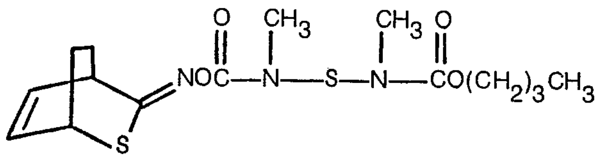 ,

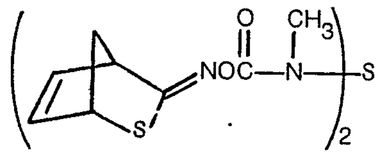 , 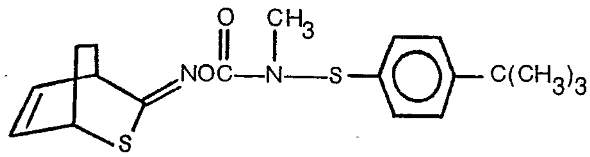 ,

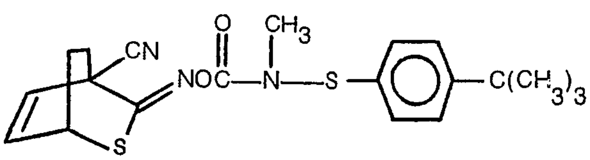 , 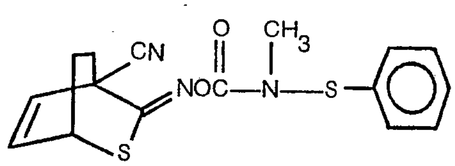 ,

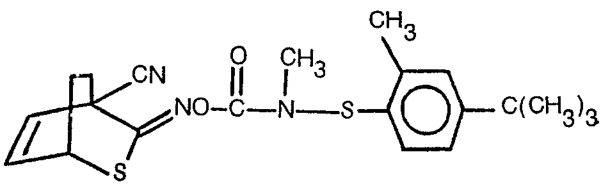 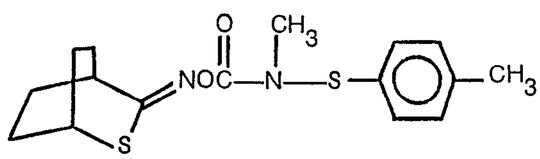 ,

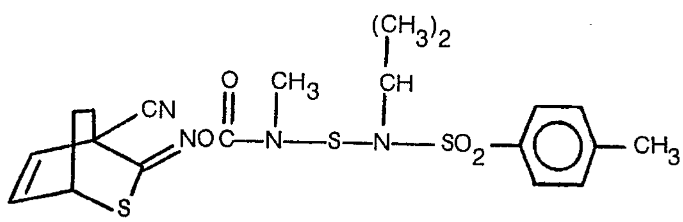 ,

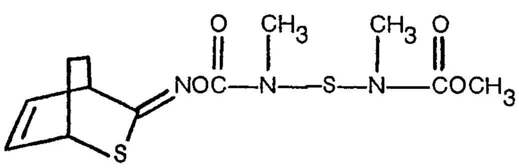 , 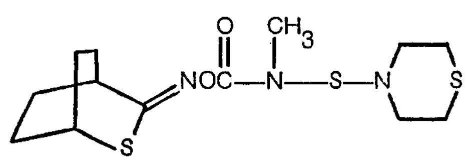 ,

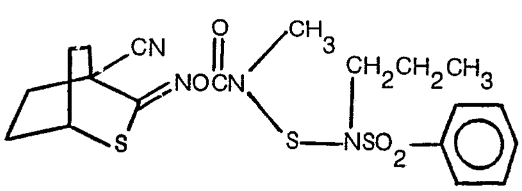 , 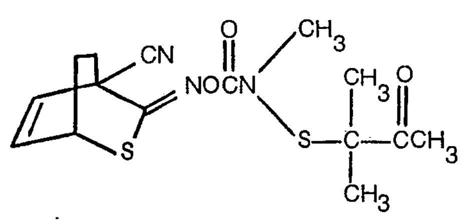 ,

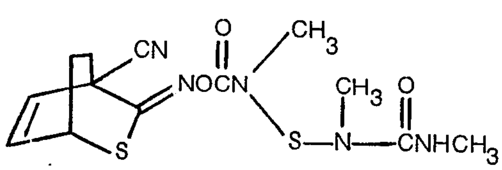 , 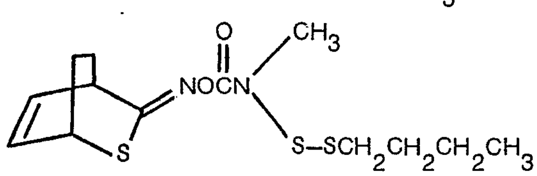 ,

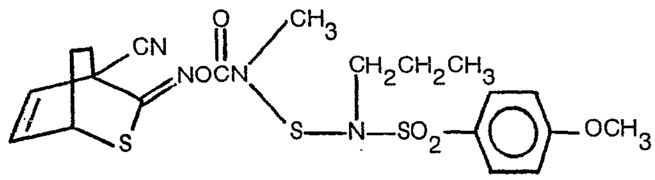 ,

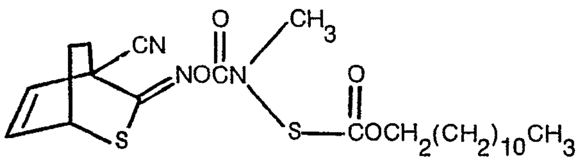 , 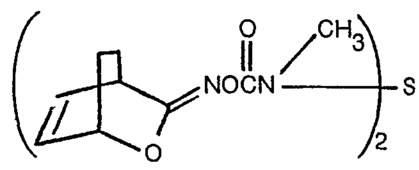 ,

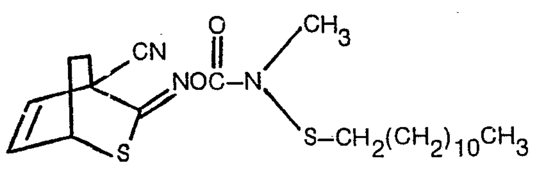 , 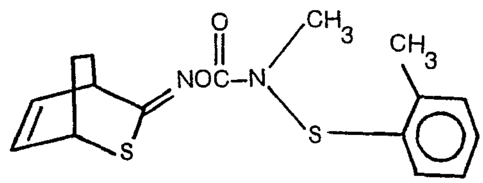 ,

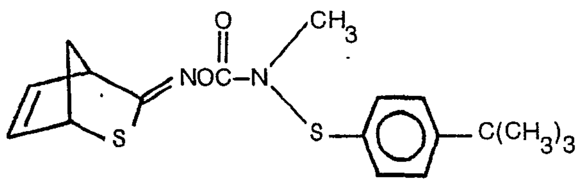 und

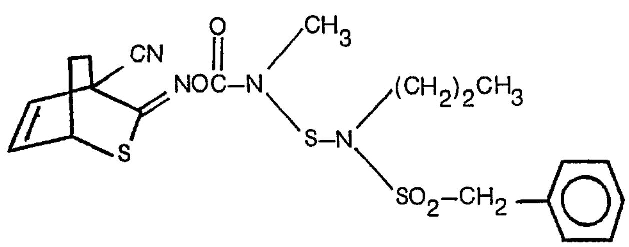

ausgewählt ist.

6. Eine arthropodizide Zusammensetzung, gekennzeichnet durch einen Carrier und eine heterobicyclische Verbindung nach einem der Ansprüche 1 bis 5.

7. Eine nematozide Zusammensetzung, gekennzeichnet durch einen Carrier und eine heterobicyclische Verbindung nach Anspruch 2, wobei in der Formel g = 0 ist.

8. Ein Verfahren zur Bekämpfung von Arthropoden oder Nematoden, dadurch gekennzeichnet, daß eine wirksame Menge von wenigstens einer Verbindung nach Anspruch 2, bei der in der Formel g = 0 ist oder wenigstens eine Verbindung nach Anspruch 7 auf die Arthropoden oder Nematoden bzw. auf einen ihrer Wirte Angewendet wird.

9. Ein Verfahren nach Anspruch 9, dadurch gekennzeichnet, daß die Verbindung oder die Zusammensetzung direkt auf die Arthropoden oder die Nematoden so appliziert wird, daß die Verbindung in einer Menge von 0,112 bis 22,4 kg/ha (0,1 bis 20 lb/A) bereitgestellt wird.

10. Ein Verfahren nach Anspruch 9, dadurch gekennzeichnet, daß die Menge im Bereich von 0,28 bis 4,48 kg/ha (0,25 bis 4 lb/A) liegt.

11. Ein Verfahren zur Herstellung von Verbindungen der Formel

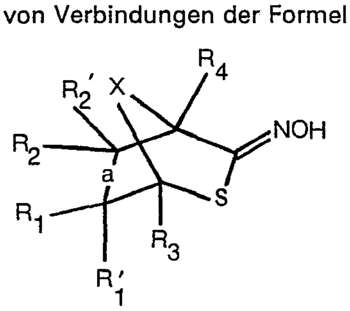

bei der

a eine Einfach- oder Doppelbindung ist;

X für O, S, $NR_6$, 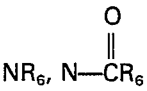 steht,

und wobei $R_6$ ein Alkylrest ist oder ein Brückenglied darstellt, welches aus der Gruppe

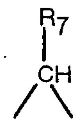 , 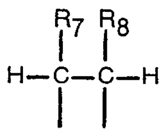 , 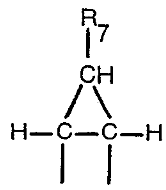 , 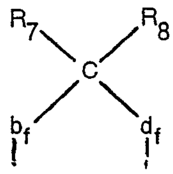 und 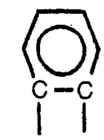 ,

ausgewählt ist,
wobei $R_7$ und $R_8$ unabhängig für Wasserstoff, Halogen, Cyano, Alkyl, Alkoxy, Alkoxycarbonyl oder Alkylthio stehen und b und d unabhängig Kohlenstoff oder Sauerstoff darstellen und f = 0 oder 1 ist;
$R_1$ bis $R_4$ incl., unabhängig für Wasserstoff, Halogen, Hydroxy, Cyano, Alkyl, Alkoxy, Haloalkyl, Alkylcarboxy, Arylcarboxy, Alkylaminocarboxy, Carbamoyl, Alkylcarbamoyl, Dialkylcarbamoyl, Alkylthio, Alkylsulfinyl, Alkylsulfonyl, Alkylamino, Dialkylamino, Alkoxycarbonyl, Trifluormethyl, Pyrrolidyl, Phenyl, Nitro, Thiocyano, Thiocarbamyl, Alkylthiocarbamyl, Dialkylthiocarbamyl, Arylthiocarbamyl, oder die Gruppe

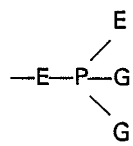 steht, wobei E = O oder S ist und

G Alkyl, Alkoxy, Alkylthio, Amino Alkylamino oder Dialkylamino darstellt; oder falls a eine Einfachbindung ist und $R_1'$ und $R_2'$ für Wasserstoff stehen, stellen $R_1$ und $R_2$ zusammen

—O—, 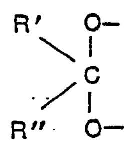 oder 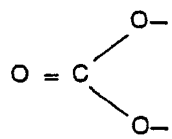 ,

dar, wobei R' und R" Wasserstoff oder Alkyl darstellen; oder $R_1'$ und $R_1$ oder $R_2$ und $R_2'$ für = O stehen; oder wenn a eine Doppelbindung ist, sind $R_1'$ und $R_2'$ nicht vorhanden;
bei welchem ein cyclisches Dien der Formel

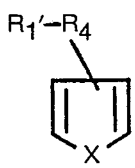 ,

wobei $R_1'$ bis $R_4$ und X die oben definierten Bedeutungen aufweisen
mit einem Dienophil, aus der Gruppe

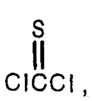 , 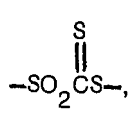 , 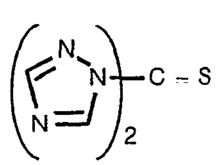 und 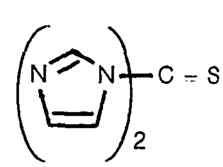

unter cyclisierenden Bedingungen umgesetzt wird, dadurch gekennzeichnet, daß das resultierende Addukt mit Hydroxylamin zu einem Thiolhydroximidat umgesetzt wird.

12. Ein Verfahren zur Herstellung einer Verbindung nach Anspruch 1, wobei in der Formel A für —O— und Y für H steht, dadurch gekennzeichnet, daß ein cyclisches Dien der Formel

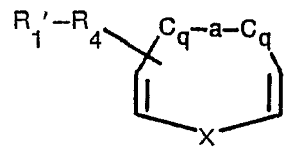,

wobei $R_1'$ bis $R_4$, X q und a die im Anspruch 1 definierten Bedeutungen haben mit Chlorosulfonylisocyanat unter cyclisierenden Bedingungen umgesetzt wird, um so eine Imino-oxabicyclische Verbindung der Formel

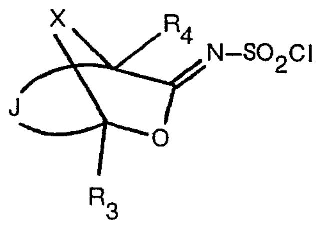

zu bilden, wobei J, X, $R_3$ und $R_4$ die im Anspruch 1 definierten Bedeutungen haben und die iminooxabicyclische Verbindung mit Hydroxylamin umgesetzt wird, um ein Oxahydroximidat zu bilden.

**Revendications**

1. Un composé hétérobicyclique de formule:

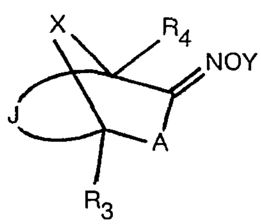

(I)

caractérisé en ce que soit:

A représente S, O, $S(O)_m$, où m est 1 ou 2, ou $NR_5$, où $R_5$ est un hydrogène, un alkyle, un aryle ou cyano;
J représente le groupe

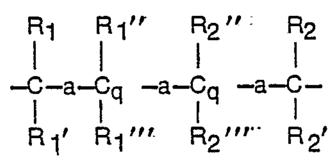

où q indépendamment est 0 ou 1, a indépendamment est une simple liaison ou une double liaison et $R_1$, $R_1'$, $R_1''$, $R_1'''$, $R_2$, $R_2'$, $R_2''$ et $R_2'''$ sont comme défini ci-dessous;
X représente O, S, $NR_6$,

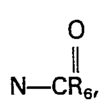

où $R_6$ est un hydrogène ou un alkyle, ou un élément de pontage choisi parmi

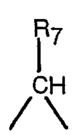, 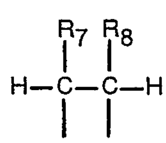, 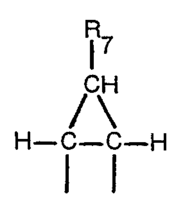, 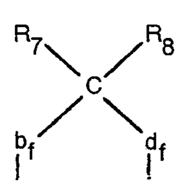 et 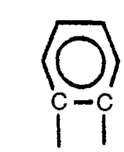,

où $R_7$ et $R_8$ indépendamment représentent un hydrogène, un halogène, un cyano, un alkyle, un alcoxy, un alcoxycarbonyle ou un alkylthio et b et d indépendamment représentent un carbone ou un oxygène et f est 0 ou 1;

$R_1$—$R_1'''$ inclusivement, $R_2$—$R_2'''$ inclusivement, $R_3$ et $R_4$ indépendamment représentent un hydrogène, un halogène, un hydroxy, un cyano, un alkyle, un alcoxy, un halogénoalkyle, un alkylcarboxy, un arylcarboxy, un alkylaminocarboxy, un carbamoyle, un alkylcarbamoyle, un dialkylcarbamoyle, un alkylthio, un alkylsulfinyle, un alkylsulfonyle, un alkylamino, un dialkylamino, un alcoxycarbonyle, un trifluorométhyle, un pyrrolidyle, un phényle, un nitro, une thiocyano, un thiocarbamyle, un alkylthiocarbamyle, un dialkylthiocarbamyle, un arylthiocarbamyle ou le groupe

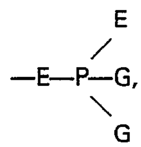

où E est O ou S, et G représente un alkyle, un alcoxy, un alkylthio, un amino, un alkylamino ou un dialkylamino: ou lorsque a est une simple liaison et au moins deux de $R_1'$, $R_1'''$, $R_2'$ ou $R_2'''$ sont un hydrogène sur des atomes de carbones adjacents, $R_1$, $R_1''$, $R_2$ ou $R_2''$ sur les mêmes atomes de carbone adjacents représentent ensemble

—O—, 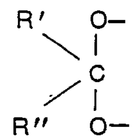 ou 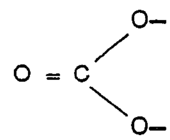 ,

où R' et R'' représentent un hydrogène ou un alkyle; ou l'un quelconque de $R_1$ et $R_1'$, $R_1''$ et $R_1'''$, $R_2''$ et $R_2'''$ ou $R_2$ et $R_2'$ représente =O: ou lorsque q indépendamment est O ou 1 et a indépendamment est une bouble liaison, $R_1'$, $R_1'''$, $R_2'''$ ou $R_2'$ sont absents; et

Y représente un hydrogène ou

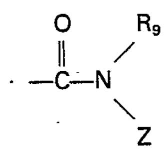

où $R_9$ représente un hydrogène, un alkyle, un hydroxyalkyle, un alcényle, un alcynyle, un aralkyle, un alcoxyalkyle ou un polyoxyalkylène; et Z représente

a) 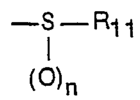 ,

où n est 0, 1 ou 2 et $R_{11}$ est un pyridyle, un pyrimidyle, un phényle ou un phényle substitué avec au moins un composant choisi parmi un hydroxy, un alkyle, un alcoxy, un halogène, un nitro, un trifluorométhyle et un cyano;

b) 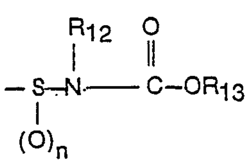 ,

où n est 0, 1 ou 2 et $R_{12}$ est un alkyle, un alcoxyalkyle ou

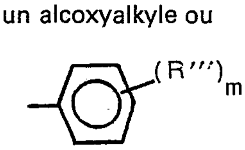 ,

où m est 0, 1, 2 ou 3 et R''' est un hydrogène, un halogène, un cyano, un nitro, un akyle, un alcoxy, un alkylthio, un alkylsulfonyle ou un phényloxy et $R_{13}$ est un alkyle, un alcoxyalkyle, un naphtyle, un alkylthioalkyle,

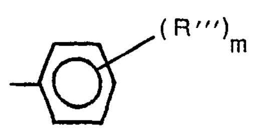 , 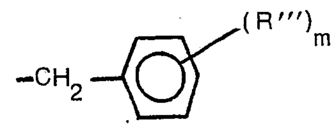 ,

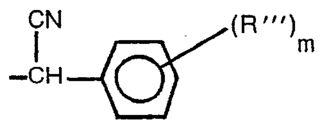 ou 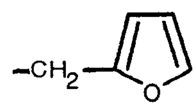

où $(R''')_m$ a la signification définie ci-dessus,

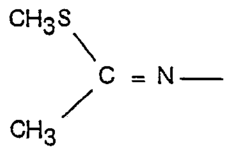 , 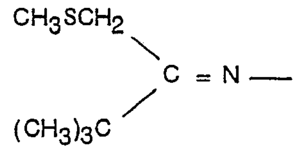

ou Q, où Q—OY représente la formule (I) comme défini ci-dessus;

c) 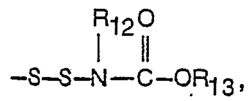

où $R_{12}$ et $R_{13}$ ont les significations définies ci-dessus;

d) 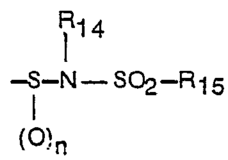 ,

où n est 0, 1 ou 2, $R_{14}$ est un phényle, un alkyle, un alcoxyalkyle, un alcoxycarbonylalkyle, un alkylthioalkyle ou un carboxyalkyle et $R_{15}$ est un alkyle,

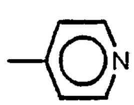 , 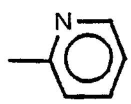 , 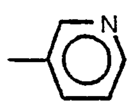 ou 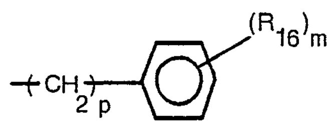

où m est 0—5, p est 0—5 et $R_{16}$ est un halogène, un alkyle, un trifluorométhyle, un nitro ou un alcoxy;

e) $-S-NR_{17}R_{18}$,

où $R_{17}$ et $R_{18}$ sont un alkyle ou un aryle ou ensemble avec l'atome d'azotes représentent

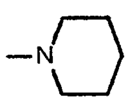 , 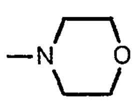 , 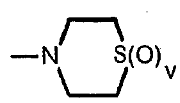 ,

où v est 0, 1 ou 2 ou

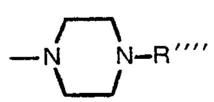 ,

où R'''' est un alkyle;

f) 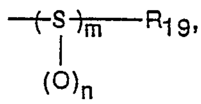

où n est 0, 1 ou 2, m est 1 ou 2 et $R_{19}$ est un alkyle, un cycloalkyle, un halogénoalkyle, un cyanoalkyle, un alcoxycarbonyle, un (alkylthio)carbonyle, un alcoxy(thiocarbonyle), un alkylthio(thiocarbonyle), un aryle ou un aryle substitué avec au moins un substituant choisi parmi un halogène, un cyano, un nitro, un alkyle, un alcoxy, un alkylthio, un alkylsulfonyle et un phényloxy, sous réserve que lorsque $R_{19}$ est un aryle ou un aryle substitué, m est 2:

g) 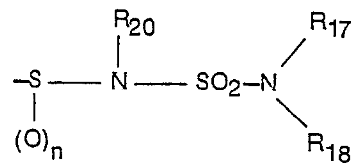

où n est 0, 1 ou 2, $R_{20}$ est un alkyle et $R_{17}$ et $R_{18}$ ont les significations définies ci-dessus:

h) 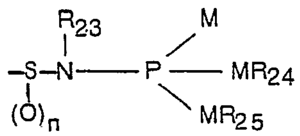 ,

où n est 0, 1 ou 2, chaque M indépendamment est S ou O et $R_{23}$, $R_{24}$ et $R_{25}$ indépendamment représentent un alkyle ou $R_{24}$ et $R_{25}$ représentent ensemble

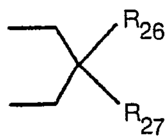 ou 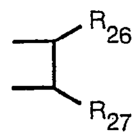 ,

où $R_{26}$ et $R_{27}$ représentent indépendamment un hydrogène ou un alkyle:

i) 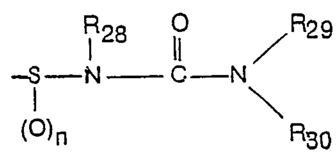 ,

où n est 0, 1 ou 2, $R_{28}$ est un alkyle ou un aryle et $R_{29}$ et $R_{30}$ indépendamment représentent un hydrogène, un alkyle, un aryle ou un alcoxy;

j) 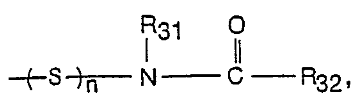

où n est 1 ou 2, $R_{31}$ est un alkyle et $R_{32}$ est un fluoro, un alkyle, un aryle ou un aralkyle:

k) 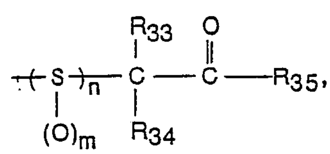

où m est 0, 1 ou 2, n est 1 ou 2 et $R_{33}$, $R_{34}$ et $R_{35}$ représentent indépendamment un hydrogène, un alkyle ou un aryle; ou

l) 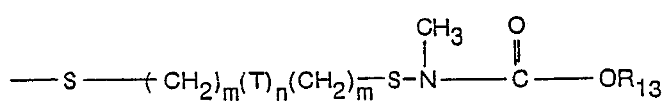 ,

où T est O, S ou $—CH_2—$, m est 1 ou 2, n est 0 ou 1 et $R_{13}$ a la signification définie ci-dessus: où dans les définitions ci-dessus, sauf indication contraire, "alkyle" et "alcoxy" désignent des groupes qui contiennent 1 à 22 atomes de carbone et comprennent toutes les structures droites et ramifiées, "alcényle" et "alcynyle" désignent des groupes qui contiennent jusqu'à 22 atomes de carbone et comprennent toutes les structures droites et ramifiées, "cycloalkyle" désigne des groupes qui contiennent 3 à 8 atomes de carbone, "aryle", désigne des groupes qui contiennent 6 à 12 atomes de carbone et "aralkyle" désigne des groupes qui contiennent un groupe aryle comme défini ci-dessus, fixé à un groupe alkyle comme défini ci-dessus; ou A représente S, J représente —CH=CH—, X représente $—CH_2CH_2$, $R_3$ représente H, $R_4$ représente CN et Y représente

$C(=O)N(CH_3)SN(C_{12}H_{25}\text{-}n)SO_2NC_4H_9$.

2. Un composé hétérobicyclique de formule

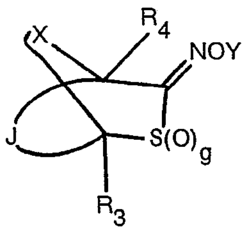

dans laquelle g est 0, 1 ou 2, J, X et Y ont la signification définie dans la revendication 1 et $R_1$—$R_1'''$, inclusivement $R_2$—$R_2'''$, inclusivement, $R_3$ et $R_4$, indépendamment, représentent un hydrogène, un halogène, un hydroxy, un cyano, un alkyle, un alcoxy, un halogénoalkyle, un alkylcarboxy, un arylcarboxy, un alkylaminocarboxy, un carbamoyle, un alkylcarbamoyle, un dialkylcarbamoyle, un alkylthio, un alkylsulfinyle, un alkylsulfonyle, un alkylamino, un dialkylamino, un alcoxycarbonyle, un trifluorométhyle, un pyrrolidyle, un phényle, un nitro, un thiocyano, un thiocarbamyle, un alkylthiocarbamyle, un dialkylthiocarbamyle, un arylthiocarbamyle ou le groupe

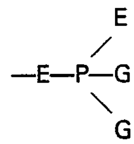

où E est O ou S et G représentent un alkyle, un alcoxy, un alkylthio, un amino, un alkylamino ou un dialkylamino.

3. Un composé selon la revendication 1 où A et J ont les significations définies dans la revendication 1, X est un élément de pontage choisi parmi

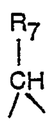 , 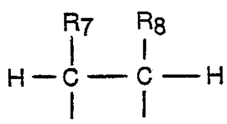 , 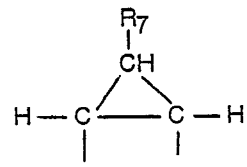 ,

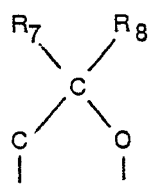 ou 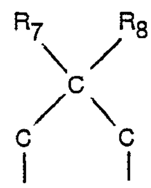 ,

où $R_7$ et $R_8$ représentent indépendamment un hydrogène ou un cyano, $R_1$—$R_1'''$, inclusivement, et $R_2$—$R_2'''$, inclusivement, sont un hydrogène, ou, lorsque a est une double liaison, $R_1$ et $R_2$ et, de plus, si q indépendamment dans chaque cas est 1, $R_1''$ ou $R_2''$ également sont indépendamment un hydrogène ou un halogène et $R_1'$, $R_2'$, $R_1'''$ ou $R_2'''$ sont absents;

$R_3$ et $R_4$ indépendamment représentent un hydrogène, un halogène, un cyano ou un alcoxycarbonyle;

Y représente

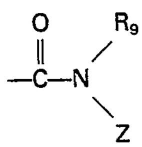

où $R_9$ représente un hydrogène, un alkyle, un hydroxyalkyle, un alcényle ou un alcynyle; et Z représente

a) 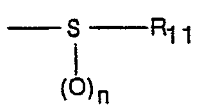 ,

où n est 0, 1 ou 2 et $R_{11}$ est un phényle ou un phényle substitué avec au moins un composant choisi parmi un halogène, un alkyle, un alcoxy, un trifluorométhyle, un nitro et un cyano;

b) 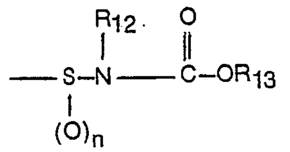 ,

où n est 0, 1 ou 2, $R_{12}$ est un alkyle et $R_{13}$ est un alkyle, un alcoxyalkyle ou Q, où Q—OY représente la formule (I) comme défini dans la revendication 1;

c) 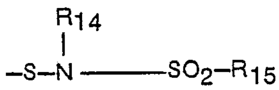 ,

où $R_{14}$ est un alkyle et $R_{15}$ est un alkyle ou

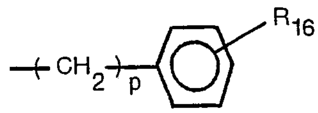

où p est 0—5 et $R_{16}$ est un halogène, un alkyle, un trifluorométhyle, un nitro, un méthoxy, un cyano ou un dialkylamino;

d) $-S-NR_{17}R_{18}$,

où $R_{17}$ et $R_{18}$ sont un alkyle ou un aryle ou ensemble avec l'atome d'azote représentent

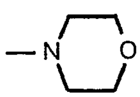 ou 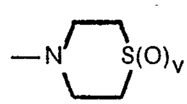 ,

où v est 0, 1 ou 2,

e) $-(S)_n-R_{19}$ ,

où n est 1 ou 2 et $R_{19}$ est un alkyle, un cyanoalkyle ou un alcoxycarbonyle;

f) 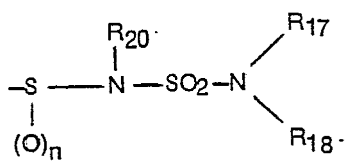 ,

où n est 0, 1 ou 2, $R_{20}$ est un alkyle et $R_{17}$ et $R_{18}$ ont les significations définies ci-dessus;

g) 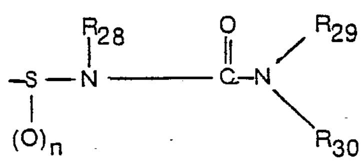 ,

où n est 0, 1 ou 2, $R_{28}$ est un alkyle ou un aryle et $R_{29}$ et $R_{30}$ représentent indépendamment un hydrogène, un alkyle, un aryle ou un alcoxy; ou

h) 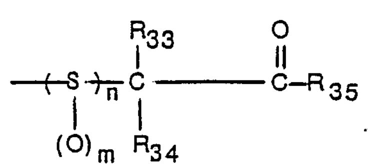 ,

où m est 0, 1 ou 2 et n est 1 ou 2 et $R_{33}$, $R_{34}$ et $R_{35}$ indépendamment représentent un hydrogène, un alkyle ou un aryle.

4. Composé selon la revendication 1 où A représente S ou O;

J a la signification définie dans la revendication 1;

X est un élément de pontage choisi parmi

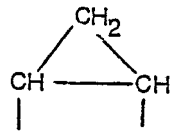 ;

$R_1$—$R_1'''$, inclusivement, $R_2$—$R_2'''$, inclusivement, $R_3$ et $R_4$ ont les significations définies dans la revendication 3; et

Y représente

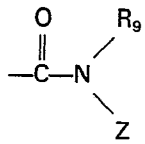

où $R_9$ représente un hydrogène, un alkyle, un alcényle ou un alcynyle; et Z représente

a) 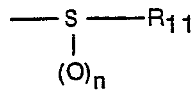 ,

où n est 0, 1 ou 2, $R_{11}$ est un alkyle, un phényle ou un phényle substitué par au moins un composant choisi parmi un halogène, un alkyle, un alcoxy, un trifluorométhyle, un nitro et un cyano;

b) 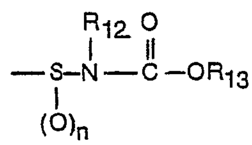 ,

où n est 0, 1 ou 2, $R_{12}$ est un alkyle et $R_{13}$ est un alkyle, un alcoxyalkyle ou Q où Q—OY représente la formule (I) comme défini dans la revendication 1;

c) 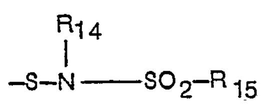 ,

où $R_{14}$ et $R_{15}$ sont un alkyle, un aryle ou un aryle substitué avec au moins un composant choisi parmi un halogène, un alkyle, un trifluorométhyle, un nitro, un méthoxy, un cyano et un dialkylamino;

d) $-S-NR_{17}R_{18}$,

où $R_{17}$ et $R_{18}$ sont un alkyle ou un aryle ou avec l'atome d'azote représentent

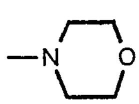 , 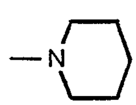 ou 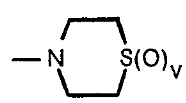 ,

où v est 0, 1 ou 2;

e) 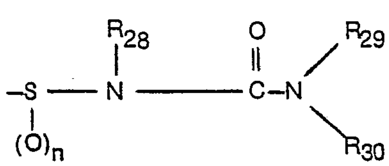 ,

où n est 0, 1 ou 2, $R_{28}$ est un alkyle ou un aryle et $R_{29}$ et $R_{30}$ indépendamment représentent un hydrogène, un alkyle, un aryle ou un alcoxy; et

f) 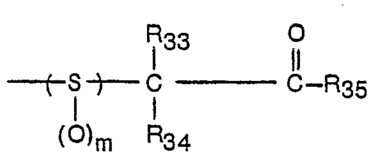 ,

où m est 0, 1 ou 2, n est 1 ou 2 et $R_{33}$, $R_{34}$ et $R_{35}$ indépendamment représentent un hydrogène, un alkyle ou un aryle.

5. Un composé selon la revendication 1 contenant la structure

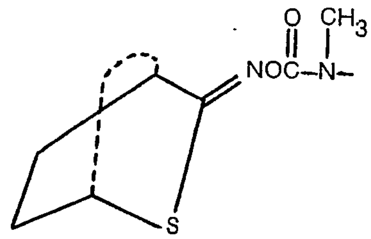

et choisi dans le groupe constitué par

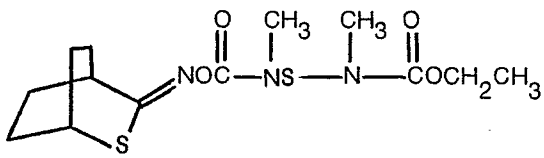 , 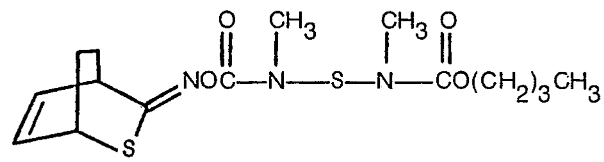 ,

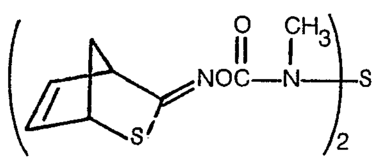 , 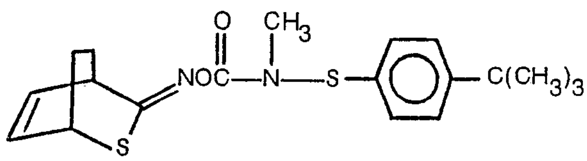 ,

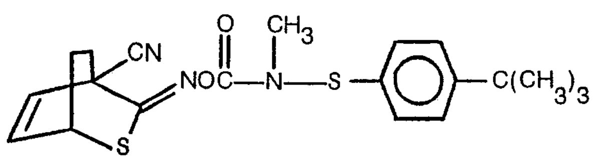 , 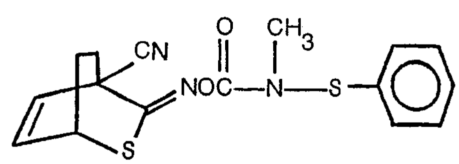 ,

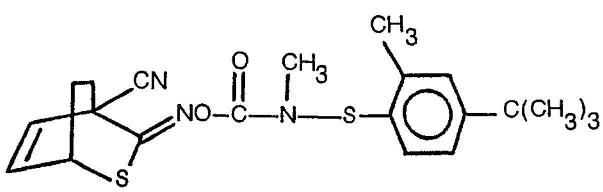 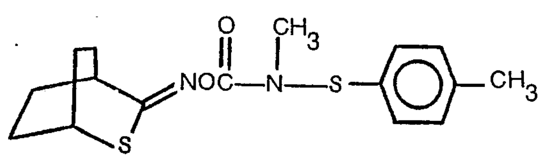

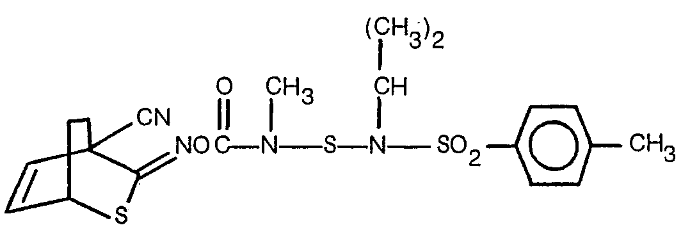 ,

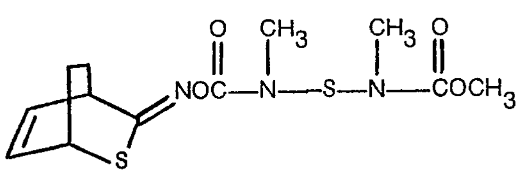 , 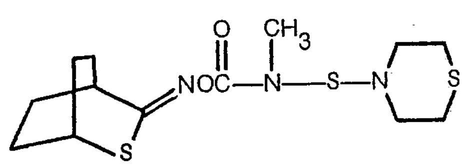 ,

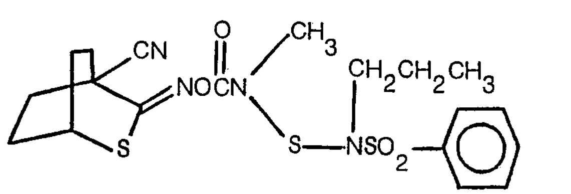 , 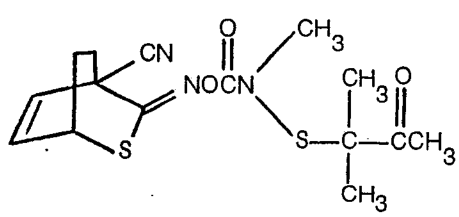 ,

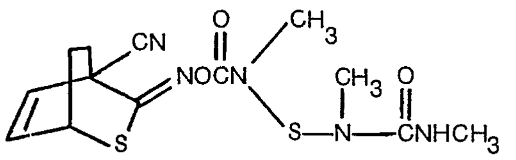 ,

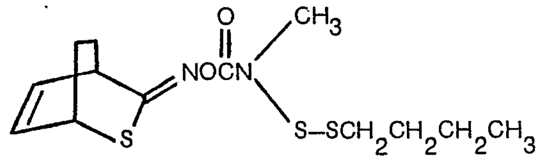 ,

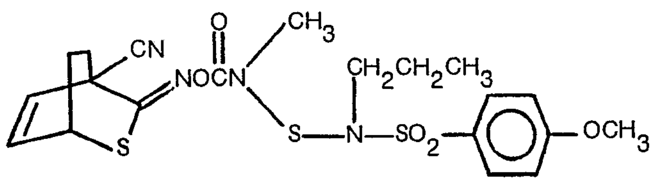 ,

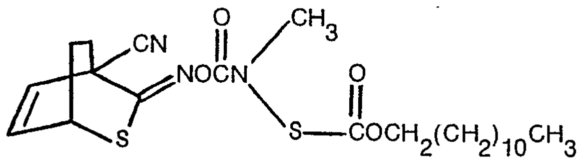 ,

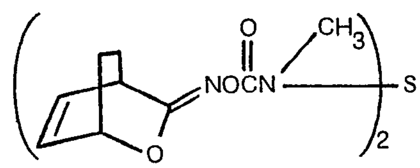 ,

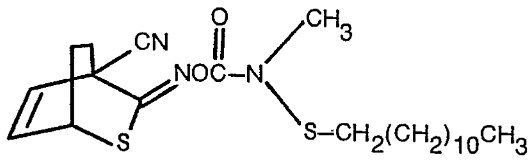 ,

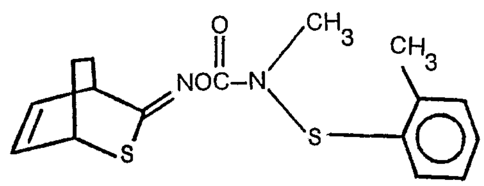 ,

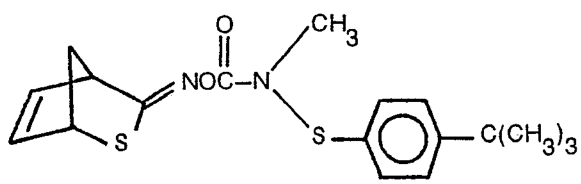

et

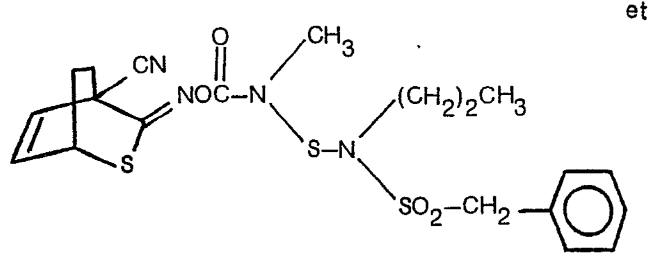

6. Une composition arthropodicide comprenant un support et un composé hétérobicyclique selon l'une quelconque des revendications 1 à 5.

7. Une composition nématocide comprenant un support et un composé hétérobicyclique selon la revendication 2, où dans la formule, g est 0.

8. Un procédé de lutte contre les arthropodes ou les nématodes qui comprend l'application, aux arthropodes ou aux nématodes ou à un habitat de ceux-ci, d'une quantité efficace d'au moins un composé selon la revendication 2, où, dans la formule, g est 0 ou d'au moins une composition selon la revendication 7.

9. Un procédé selon la revendication 8 dans lequel le composé ou la composition est appliqué directement aux arthropodes ou aux nématodes de façon à ce que la dose du composé soit dans la gamme de 0,112 à 22,4 kg/ha (0,1 à 20 lb/A).

10. Un procédé selon la revendication 9 dans lequel la dose est dans la gamme de 0,28 à 4,48 kg/ha (0,25 à 4 lb/A).

11. Un procédé de préparation d'un composé de formule:

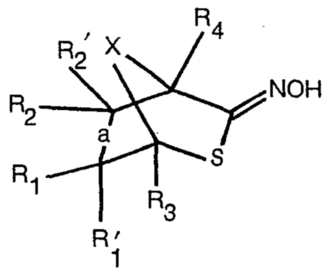

dans laquelle

a est une simple liaison ou une double liaison;

x représente O, S, $NR_6$,

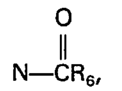

où $R_6$ est un alkyle, ou un élément de pontage choisi parmi

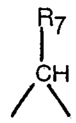 , 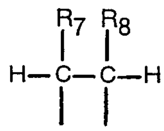 , 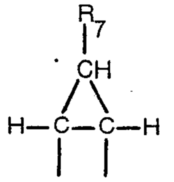 , 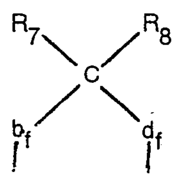 et 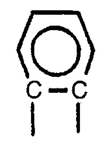 ,

où $R_7$ et $R_8$ indépendamment représentent un hydrogène, un halogène, un cyano, un alkyle, un alcoxy, un alcoxycarbonyle ou un alkylthio et b et d indépendamment représentent un carbone ou un oxygène et f est 0 ou 1;

$R_1'$—$R_4$ inclusivement représentent indépendamment un hydrogène, un halogène, un hydroxy, un cyano, un alkyle, un alcoxy, un halogénoalkyle, un alkylcarboxy, un arylcarboxy, un alkylaminocarboxy, un carbamoyle, un alkylcarbamoyle, un dialkylcarbamoyle, un alkylthio, un alkylsulfinyle, un alkylsulfonyle, un alkylamino, un dialkylamino, un alcoxycarbonyle, un trifluorométhyle, un pyrrolidyle, un phényle, un nitro, un thiocyano, un thiocarbamyle, un alkylthiocarbamyle, un dialkylthiocarbamyle, un arylthiocarbamyle ou le groupe

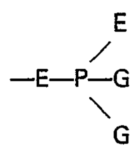

où E est O ou S et G représente un alkyle, un alcoxy, un alkylthio, un amino, un alkylamino ou un dialkylamino; ou, lorsque a est une simple liaison et $R_1'$ et $R_2'$ sont un hydrogène, $R_1$ et $R_2$ représentent ensemble

—O—, 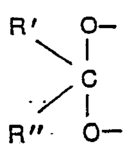 or 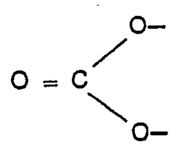 ,

où R' et R'' représentent un hydrogène ou un alkyle; ou $R_1'$ et $R_1$ ou $R_2$ et $R_2'$ représentent =O; ou, lorsque a est une double liaison, $R_1'$ et $R_2'$ sont absents; dans lequel on fait réagir un diène cyclique de formule

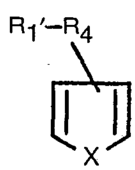 ,

dans laquelle $R_1'$—$R_4$ et X ont les significations définies ci-dessus, avec un diénophile choisi parmi

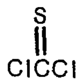 , 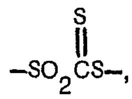 , 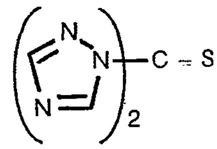 et 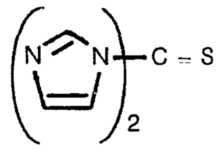

dans des conditions de cyclisation, caractérisé en ce que on fait réagir le produit d'addition obtenu avec l'hydroxylamine pour former un thiolhydroximidate.

12. Un procédé de préparation d'un composé selon la revendication 1, où, dans la formule, A représente —O— et Y représente H, caractérisé en ce que on fait réagir un diène cyclique de formule

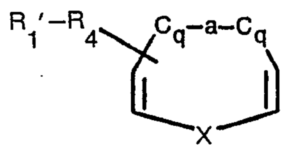 ,

dans laquelle $R_1'$—$R_4$, X, q et a ont les significations définies dans la revendication 1, avec l'isocyanate de chlorosulfonyle dans des conditions de cyclisation pour former un composé imino-oxabicyclique de formule

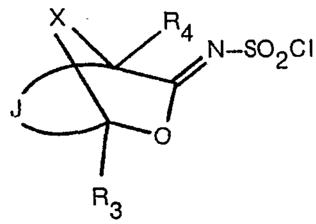

dans laquelle J, X, $R_3$ et $R_4$ ont les significations définies dans la revendication 1 et on fait réagir le composé iminooxabicyclique avec l'hydroxylamine pour former un oxahydroximidate.